# EUROPEAN PATENT APPLICATION

(11) **EP 4 450 498 A1**
(43) Date of publication of application: **23.10.2024**
(21) Application number: 22906271.6
(22) Date of filing: 01.12.2022
(51) Int. Cl.: C07D 471/04, C07D 487/04, C07D 401/14, C07D 403/14, A61K 31/4375, A61P 35/00

(54) **PARP INHIBITOR, PHARMACEUTICAL COMPOSITION COMPRISING SAME, AND USE THEREOF**

(30) Priority: 17.12.2021 CN 202111559484
(71) Applicant: Keythera (Suzhou) Bio-Pharmaceuticals Co., Limited, Suzhou, Jiangsu 215000 (CN)
(72) Inventor: DENG, Yongqi, Suzhou, Jiangsu 215000 (CN); TIAN, Yuan, Suzhou, Jiangsu 215000 (CN); JIA, Yanlin, Suzhou, Jiangsu 215000 (CN); ZHU, Shijun, Suzhou, Jiangsu 215000 (CN)
(74) Representative: Maikowski & Ninnemann Patentanwälte Partnerschaft mbB
(86) International application number: PCT/CN2022/135894
(87) International publication number: WO 2023/109521

(57) **Abstract**

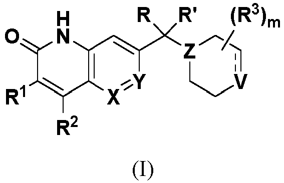

A compound of formula (I), a pharmaceutical composition comprising same, and the use thereof as a PARP inhibitor, preferably as a PARP1 selective inhibitor.

## Description

### FIELD OF THE INVENTION

The present invention relates to a PARP inhibitor, a pharmaceutical composition comprising same, and use thereof in the prophylaxis or treatment of a disease that can be ameliorated by inhibiting PARP.

### BACKGROUND OF THE INVENTION

Poly(ADP-ribose) polymerase (PARP) can catalyze the transfer of ADP-ribose residues from NAD+ to target substrates, modifying proteins through ribosylation. This poly(ADP-ribosylation) is a posttranslational modification of proteins that is widely involved in various physiological and pathological processes. PARP can poly(ADP-ribosylate) various nuclear proteins, including histones, RNA polymerase, DNA polymerase, and DNA ligase. PARP is activated by recognizing damaged DNA fragments, leading to the glycosylation modification of nuclear proteins, thereby completing the function of DNA repair. In normal cells, other DNA repair mechanisms, such as the BRCA pathway, can also complete DNA repair, allowing cell survival. Therefore, inhibiting PARP does not significantly affect DNA repair in normal cells. In some cancer patients, when BRCA genes are mutated, the gene repair of tumor cells mainly relies on the PARP mechanism. In these patients, PARP inhibitors can block DNA repair functions, causing tumor cell apoptosis. In recent years, PARP inhibitors have been used to treat ovarian and breast cancer patients carrying BRCA mutations, achieving significant therapeutic effects.

Currently approved first-generation PARP inhibitors have certain inhibitory effects on bone marrow hematopoietic function, limiting their combined use with chemotherapy drugs that also inhibit bone marrow hematopoietic function.

Additionally, since PARP is involved in regulating various physiological and pathological processes (oxidative stress response, promoting inflammatory response, exacerbating viral infections, and controlling glucose stability), PARP inhibitors can also be used to treat diseases related to these functions, including diseases characterized by oxidative stress (e.g., ischemia-reperfusion injury, inflammatory diseases, burns, Parkinson's disease, Huntington's disease, Alzheimer's disease, and toxic injuries); inflammatory diseases (asthma, arthritis, colitis, chronic obstructive pulmonary disease, acute respiratory distress syndrome, atherosclerosis, post-myocardial infarction cardiac remodeling, sepsis, endotoxin shock, hemorrhagic shock, graft-versus-host disease, encephalomyelitis, and autoimmune nephritis); viral infections (anti-human immunodeficiency virus-1, Venezuelan equine encephalitis virus, herpes simplex virus, human hepatitis B virus, and human cytomegalovirus infections); and metabolic disorders (metabolic syndrome and type II diabetes and its subsequent complications, such as diabetic neurological, renal and eye complications).

### SUMMARY OF THE INVENTION

The present invention provides PARP inhibitors that can be used for the prophylaxis or treatment of diseases that can be ameliorated by inhibiting PARP. Preferably, the compounds of the present invention have selective inhibitory activity against PARP-1. Additionally, the compounds of the present invention possess better physicochemical properties (e.g., solubility, physical and/or chemical stability), improved pharmacokinetic properties (e.g., improved bioavailability, suitable half-life, and duration of action), improved safety (lower toxicity, e.g., reduced cardiotoxicity, and/or fewer side effects), and excellent properties such as being less prone to develop resistance.

One aspect of the present invention provides a compound or a pharmaceutically acceptable salt, ester, stereoisomer, tautomer, polymorph, solvate, metabolite, isotopically labeled compound or prodrug thereof, wherein the compound has the structure of Formula (I): 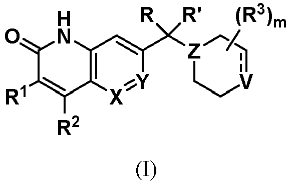 wherein:
--- is a single bond or a double bond;
X is N or CR⁵;
Y is Nor CR⁵';
Z is CR⁶ or N;
when --- is a single bond, V is CR⁷R^{A} or NR^{A}; when --- is a double bond, V is CR^{A};
R^{A} is selected from the group consisting of H, halogen, -OH, -NH₂, -CN, -NO₂, C₁₋₆ alkyl, C₁₋₆ haloalkyl, C₃₋₆ cyclic hydrocarbyl, 3- to 10-membered heterocyclyl, C₆₋₁₀ aryl, 5- to 14-membered heteroaryl, C₆₋₁₂ aralkyl, -C(=O)R^{a}, -OC(=O)R^{a}, -C(=O)OR^{a}, -OR^{a}, -SR^{a}, -S(=O)R^{a}, -S(=O)₂R^{a}, -S(=O)₂NR^{a}R^{b}, -NR^{a}R^{b}, - C(=O)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -C₁₋₆ alkylene-CN, - C₁₋₆ alkylene-OR^{a}, -C₁₋₆ alkylene-NR^{a}R^{b}, -O-C₁₋₆ alkylene-NR^{a}R^{b} and 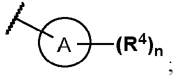
ring A is a C₃₋₆ hydrocarbon ring, 3- to 10-membered heterocycle, C₆₋₁₀ aromatic ring or 5- to 14-membered heteroaromatic ring;
R and R', at each occurrence, are each independently selected from the group consisting of H, halogen, -OH, -NH₂, -CN, -NO₂, C₁₋₆ alkyl, C₁₋₆ haloalkyl, C₃₋₆ cyclic hydrocarbyl, 3- to 10-membered heterocyclyl, C₆₋₁₀ aryl, 5- to 14-membered heteroaryl, C₆₋₁₂ aralkyl, -C(=O)R^{a}, -OC(=O)R^{a}, -C(=O)OR^{a}, -OR^{a}, -SR^{a}, - S(=O)R^{a}, -S(=O)₂R^{a}, -S(=O)₂NR^{a}R^{b}, -NR^{a}R^{b}, -C(=O)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -C₁₋₆ alkylene-CN, -C₁₋₆ alkylene-OR^{a}, -C₁₋₆ alkylene-NR^{a}R^{b} and -O-C₁₋₆ alkylene-NR^{a}R^{b}; preferably, R and R' are each independently selected from the group consisting of H, -CN and C₁₋₆ alkyl;
R¹ and R² are each independently selected from the group consisting of halogen, -OH, -NH₂, -CN, -NO₂, C₁₋₆ alkyl, C₁₋₆ haloalkyl, C₃₋₆ cyclic hydrocarbyl, 3- to 10-membered heterocyclyl, C₆₋₁₀ aryl, 5- to 14-membered heteroaryl, C₆₋₁₂ aralkyl, -C(=O)R^{a}, -OC(=O)R^{a}, -C(=O)OR^{a}, -OR^{a}, -SR^{a}, -S(=O)R^{a}, -S(=O)₂R^{a}, - S(=O)₂NR^{a}R^{b}, -NR^{a}R^{b}, -C(=O)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -C₁₋₆ alkylene-CN, -C₁₋₆ alkylene-OR^{a}, -C₁₋₆ alkylene-NR^{a}R^{b} and -O-C₁₋₆ alkylene-NR^{a}R^{b};
R³, at each occurrence, is each independently selected from the group consisting of halogen, -OH, =O, -NH₂, -CN, -NOz, C₁₋₆ alkyl, C₁₋₆ haloalkyl, C₃₋₆ cyclic hydrocarbyl, 3- to 10-membered heterocyclyl, C₆₋₁₀ aryl, 5- to 14-membered heteroaryl, C₆₋₁₂ aralkyl, -C(=O)R^{a}, -OC(=O)R^{a}, -C(=O)OR^{a}, -OR^{a}, -SR^{a}, -S(=O)R^{a}, -S(=O)₂R^{a}, -S(=O)₂NR^{a}R^{b}, -NR^{a}R^{b}, -C(=O)NR^{a}R^{b}, -NR^{a}-C(=O)R^{b}, -NR^{a}-C(=O)OR^{b}, -NR^{a}-S(=O)₂-R^{b}, - NR^{a}-C(=O)-NR^{a}R^{b}, -C₁₋₆ alkylene-CN, -C₁₋₆ alkylene-OR^{a}, -C₁₋₆ alkylene-NR^{a}R^{b} and -O-C₁₋₆ alkylene-NR^{a}R^{b};
when m>1, two R³ groups optionally together form -C₁₋₆ alkylene- or -C₂₋₆ alkenylene-, the alkylene chain and alkenylene chain are optionally interrupted by one or more groups independently selected from the group consisting of O, C(=O), C(=O)O, NR, S, S=O and S(=O)z;
or, R³ and R^{A} together with the groups to which they are attached optionally form a C₃₋₆ hydrocarbon ring, 3- to 10-membered heterocycle, C₆₋₁₀ aromatic ring or 5- to 14-membered heteroaromatic ring;
R4, at each occurrence, is each independently selected from the group consisting of halogen, -OH, -NH₂, -CN, -NOz, C₁₋₆ alkyl, C₁₋₆ haloalkyl, C₃₋₆ cyclic hydrocarbyl, 3- to 10-membered heterocyclyl, C₆₋₁₀ aryl, 5-to 14-membered heteroaryl, C₆₋₁₂ aralkyl, -C(=O)R^{a}, -OC(=O)R^{a}, -C(=O)OR^{a}, -OR^{a}, -SR^{a}, -S(=O)R^{a}, - S(=O)₂R^{a}, -S(=O)₂NR^{a}R^{b}, -NR^{a}R^{b}, -C(=O)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -C₁₋₆ alkylene-CN, -C₁₋₆ alkylene-OR^{a}, -C₁₋₆ alkylene-NR^{a}R^{b} and -O-C₁₋₆ alkylene-NR^{a}R^{b}; when two R⁴ groups are ortho to each other on ring A, the two R⁴ groups together with the groups to which they are attached optionally form 3- to 10-membered heterocycle or 5- to 14-membered heteroaromatic ring;
or, R³ and R⁴ together with the groups to which they are attached optionally form a C₃₋₆ hydrocarbon ring, 3- to 10-membered heterocycle, C₆₋₁₀ aromatic ring or 5- to 14-membered heteroaromatic ring;
R⁵, R⁵', R⁶ and R⁷, at each occurrence, are each independently selected from the group consisting of H, halogen, -OH, -NH₂, -CN, -NOz, C₁₋₆ alkyl, C₁₋₆ haloalkyl, C₃₋₆ cyclic hydrocarbyl, 3- to 10-membered heterocyclyl, C₆₋₁₀ aryl, 5- to 14-membered heteroaryl, C₆₋₁₂ aralkyl, -C(=O)R^{a}, -OC(=O)R^{a}, -C(=O)OR^{a}, - OR^{a}, -SR^{a}, -S(=O)R^{a}, -S(=O)₂R^{a}, -S(=O)₂NR^{a}R^{b}, -NR^{a}R^{b}, -C(=O)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -C₁₋₆ alkylene-CN, -C₁₋₆ alkylene-OR^{a}, -C₁₋₆ alkylene-NR^{a}R^{b} and -O-C₁₋₆ alkylene-NR^{a}R^{b};
R^{a} and R^{b}, at each occurrence, are each independently selected from the group consisting of H, C₁₋₆ alkyl, C₂₋₆ alkenyl, C₁₋₆ haloalkyl, C₃₋₁₀ cyclic hydrocarbyl, 3- to 10-membered heterocyclyl, C₆₋₁₀ aryl, 5- to 14-membered heteroaryl and C₆₋₁₂ aralkyl;
the above alkyl, alkylene, haloalkyl, alkenyl, alkenylene, hydrocarbon ring, cyclic hydrocarbyl, heterocycle, heterocyclyl, aryl, aromatic ring, heteroaryl, heteroaromatic ring and aralkyl, at each occurrence, are each optionally substituted with one or more substituents independently selected from the group consisting of: halogen, -OH, =O, -NH₂, -CN, -NOz, C₁₋₆ alkyl, C₁₋₆ haloalkyl, C₃₋₆ cyclic hydrocarbyl, 3- to 10-membered heterocyclyl, C₆₋₁₀ aryl, 5- to 14-membered heteroaryl, C₆₋₁₂ aralkyl, -C(=O)R^{c}, -OC(=O)R^{c}, - C(=O)OR^{c}, -OR^{c}, -SR^{c}, -S(=O)R^{c}, -S(=O)₂R^{c}, -S(=O)₂NR^{c}R^{d}, -NR^{c}R^{d}, -C(=O)NR^{c}R^{d}, -NR^{c}-C(=O)R^{d}, -NR^{c}-C(=O)OR^{d}, -NR^{c}-S(=O)₂-R^{d}, -NR^{c}-C(=O)-NR^{c}R^{d}, -C₁₋₆ alkylene-OR^{a}, -C₁₋₆ alkylene-NR^{c}R^{d} and -O-C₁₋₆ alkylene-NR^{c}R^{d}, the alkyl, alkylene, haloalkyl, cyclic hydrocarbyl, heterocyclyl, aryl, heteroaryl and aralkyl are further optionally substituted with one or more substituents independently selected from the group consisting of: halogen, -OH, =O, -NH₂, -CN, -NOz, C₁₋₆ alkyl, C₁₋₆ haloalkyl, C₃₋₆ cyclic hydrocarbyl, 3- to 10-membered heterocyclyl, C₆₋₁₀ aryl, 5- to 14-membered heteroaryl and C₆₋₁₂ aralkyl;
R^{c} and R^{d}, at each occurrence, are each independently selected from the group consisting of H, C₁₋₆ alkyl, C₁₋₆ haloalkyl, C₃₋₁₀ cyclic hydrocarbyl, 3- to 10-membered heterocyclyl, C₆₋₁₀ aryl, 5- to 14-membered heteroaryl and C₆₋₁₂ aralkyl; and
m and n are each independently an integer of 0, 1, 2, 3, or 4.

Another aspect of the present invention provides a pharmaceutical composition comprising a prophylactically or therapeutically effective amount of the compound of the present invention or a pharmaceutically acceptable salt, ester, stereoisomer, tautomer, polymorph, solvate, metabolite, isotopically labeled compound or prodrug thereof, and one or more pharmaceutically acceptable carriers, and the pharmaceutical composition is preferably in the form of a solid, liquid or transdermal formulation.

Another aspect of the present invention provides use of the compound of the present invention or a pharmaceutically acceptable salt, ester, stereoisomer, tautomer, polymorph, solvate, metabolite, isotopically labeled compound or prodrug thereof or the pharmaceutical composition of the present invention in the manufacture of a medicament for use as a PARP inhibitor (preferably as a PARP1 selective inhibitor).

Another aspect of the present invention provides the compound of the present invention or a pharmaceutically acceptable salt, ester, stereoisomer, tautomer, polymorph, solvate, metabolite, isotopically labeled compound or prodrug thereof or the pharmaceutical composition of the present invention, for use as a PARP inhibitor (preferably as a PARP1 selective inhibitor).

Another aspect of the present invention provides a method for the prophylaxis or the treatment of a disease that can be ameliorated by inhibiting PARP (preferably PARP-1), the method comprising administering to a subject in need thereof an effective amount of the compound of the present invention or a pharmaceutically acceptable salt, ester, stereoisomer, tautomer, polymorph, solvate, metabolite, isotopically labeled compound or prodrug thereof or the pharmaceutical composition of the present invention.

### BRIEF DESCRIPTION OF THE DRAWINGS

**Figure 1** shows the effect of Compound 1 in inhibiting tumor growth in the MDA-MB-436 breast cancer mouse tumor model.
**Figure 2** shows the impact of Compound 1 on mouse body weight in the MDA-MB-436 breast cancer mouse tumor model.
**Figure 3** shows the effect of Compound 1 in inhibiting tumor growth in the breast cancer PDX model.
**Figure 4** shows the impact of Compound 1 on mouse body weight in the breast cancer PDX model.

### DETAILED DESCRIPTION OF THE INVENTION

### Definition

Unless otherwise defined in the context, all technical and scientific terms used herein are intended to have the same meaning as commonly understood by a person skilled in the art. References to techniques employed herein are intended to refer to the techniques as commonly understood in the art, including variations on those techniques or substitutions of equivalent techniques which would be apparent to a person skilled in the art. While it is believed that the following terms will be readily understood by a person skilled in the art, the following definitions are nevertheless put forth to better illustrate the present invention.

The terms "contain", "include", "comprise", "have", or "relate to", as well as other variations used herein are inclusive or open-ended, and do not exclude additional, unrecited elements or method steps.

As used herein, the term "alkylene" refers to a saturated divalent hydrocarbyl, preferably refers to a saturated divalent hydrocarbyl having 1, 2, 3, 4, 5 or 6 carbon atoms, e.g., methylene, ethylene, propylene or butylene.

As used herein, the term "alkyl" is defined as a straight or branched saturated aliphatic hydrocarbon. In some embodiments, alkyl has 1-12, e.g., 1-6, carbon atoms. For example, as used herein, the term "C₁₋₆ alkyl" refers to a linear or branched group having 1-6 carbon atoms (such as methyl, ethyl, n-propyl, isopropyl, n-butyl, isobutyl, sec-butyl, *tert*-butyl, n-pentyl, or n-hexyl), which is optionally substituted with one or more (e.g., 1 to 3) suitable substituents such as halogen (in which case the group may be referred to as "haloalkyl") (e.g., CF₃, C₂F₅, CHF₂, CH₂F, CH₂CF₃, CH₂Cl or -CH₂CH₂CF₃, *etc*.). The term "C₁₋₄ alkyl" refers to a linear or branched aliphatic hydrocarbon chain having 1-4 carbon atoms (i.e., methyl, ethyl, n-propyl, isopropyl, n-butyl, isobutyl, sec-butyl or *tert*-butyl).

As used herein, the term "alkenyl" refers to a hydrocarbyl group containing one or more double bonds, which preferably has 2, 3, 4, 5 or 6 carbon atoms, e.g., ethenyl, propenyl, or allyl. When the compound of the present invention contains an alkenylene group, the compound may exist as the pure E (entgegen) form, the pure Z (zusammen) form, or any mixture thereof. The term "alkenylene" refers to a divalent hydrocarbyl group containing one or more double bonds.

As used herein, the term "heteroalkyl" refers to an optionally substituted alkyl group having one or more backbone atoms selected from atoms other than carbon, such as oxygen, nitrogen, sulfur, phosphorus, or a combination thereof. A numerical range (e.g., C₁₋₆ heteroalkyl) can be given to indicate the number of carbon in the chain (in this example, 1-6 carbon atoms are comprised). For example, the -CH₂OCH₂CH₃ group is referred to as a C₃ heteroalkyl group. The linkage with the rest of the molecule can be through the heteroatom or carbon atom in the heteroalkyl chain. The term "heteroalkylene" refers to a corresponding divalent group, including, e.g., "C₁₋₆ heteroalkylene", "C₁₋₄ heteroalkylene", *etc,* preferably -CH₂OCH₂-.

As used herein, the terms "cyclic hydrocarbylene", "cyclic hydrocarbyl" and "hydrocarbon ring" refer to a saturated (i.e., "cycloalkylene" and "cycloalkyl") or unsaturated (i.e., having one or more double and/or triple bonds in the ring) monocyclic or polycyclic hydrocarbon ring having e.g., 3-10 (suitably having 3-8, and more suitably having 3-6) ring carbon atoms, including but not limited to cyclopropyl(ene) (ring), cyclobutyl(ene) (ring), cyclopentyl(ene) (ring), cyclohexyl(ene) (ring), cycloheptyl(ene) (ring), cyclooctyl(ene) (ring), cyclononyl(ene) (ring), cyclohexenyl(ene) (ring), and the like.

As used herein, the term "cycloalkyl" refers to a saturated, non-aromatic, monocyclic or polycyclic (e.g., bicyclic) hydrocarbon ring (e.g., monocyclic, such as cyclopropyl, cyclobutyl, cyclopentyl, cyclohexyl, cycloheptyl, cyclooctyl, or cyclononyl, or bicyclic, including spiro, fused or bridged cyclic system (such as bicyclo[1.1.1]pentyl, bicyclo[2.2.1]heptyl, bicyclo[3.2.1]octyl or bicyclo[5.2.0]nonyl, or decahydronaphthalene *etc*.)), which is optionally substituted with one or more (e.g., 1 to 3) suitable substituents. The cycloalkyl has 3 to 15 carbon atoms. For example, the term "C₃₋₆ cycloalkyl" refers to a saturated or unsaturated, non-aromatic, monocyclic or polycyclic (e.g., bicyclic) hydrocarbon ring having 3 to 6 ring forming carbon atoms (e.g., cyclopropyl, cyclobutyl, cyclopentyl, or cyclohexyl), which is optionally substituted with one or more (e.g., 1 to 3) suitable substituents, e.g., methyl substituted cyclopropyl.

As used herein, the term "heterocyclyl" or "heterocycle" refers to a saturated or unsaturated, monovalent, monocyclic or bicyclic residue having 2, 3, 4, 5, 6, 7, 8 or 9 carbon atoms and one or more (e.g., 1, 2, 3 or 4) heteroatom-containing groups selected from the group consisting of C(=O), O, S, S(=O), S(=O)₂, and NR^{'} wherein R^{'} represents a hydrogen atom, C₁₋₆ alkyl, or C₁₋₆ haloalkyl group, in the ring. The heterocyclyl may be linked to the rest of a molecule through any one of the carbon atoms or a nitrogen atom (if present). In particular, 3- to 10-membered heterocyclyl refers to a group having 3 to 10 carbon atoms and heteroatom(s) in the ring, such as, but are not limited to, oxiranyl, aziridinyl, azetidinyl, oxetanyl, tetrahydrofuranyl, dioxolinyl, pyrrolidinyl, pyrrolidinonyl, imidazolidinyl, pyrazolidinyl, pyrrolinyl, tetrahydropyranyl, piperidinyl, morpholinyl, dithianyl, thiomorpholinyl, piperazinyl or trithianyl.

As used herein, the term "heterocyclyl" or "heterocycle" encompasses fused ring structures, the attachment point between the fused ring structure and another group can be from any ring in the fused ring structure. As such, the heterocyclyl of the present invention also includes but is not limited to heterocyclyl-fused heterocyclyl, heterocyclyl-fused cycloalkyl, monoheterocyclyl-fused monoheterocyclyl, monoheterocyclyl-fused monocycloalkyl, such as 3- to 7-membered (mono)heterocyclyl-fused 3- to 7-membered (mono)heterocyclyl, 3- to 7-membered (mono)heterocyclyl-fused (mono)cycloalkyl, 3- to 7-membered (mono)heterocyclyl-fused C₄₋₆ (mono)cycloalkyl, and the examples include, but are not limited to, pyrrolidinyl-fused cyclopropyl, cyclopentyl-fused azacyclopropyl, pyrrolidinyl-fused cyclobutyl, pyrrolidinyl-fused pyrrolidinyl, pyrrolidinyl-fused piperidinyl, pyrrolidinyl-fused piperazinyl, piperidinylfused morpholinyl,

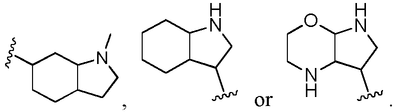

As used herein, the term "heterocyclyl" or "heterocycle" encompasses bridged heterocyclyl and spiro heterocyclyl.

As used herein, the term "bridged heterocycle" refers to a ring structure comprising one or more (e.g., 1, 2, 3 or 4) heteroatoms (e.g., oxygen, nitrogen, and/or sulfur atoms), formed by two saturated rings sharing two ring atoms which are not directly linked, including, but not limited to, 7- to 10-membered bridged heterocycle, 8- to 10-membered bridged heterocycle, 7- to 10-membered nitrogen-containing bridged heterocycle, 7- to 10-membered oxygen-containing bridged heterocycle, 7- to 10-membered sulfur-containing bridged heterocycle, and the like, such as 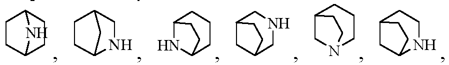 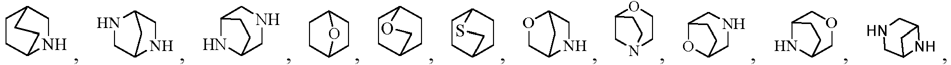 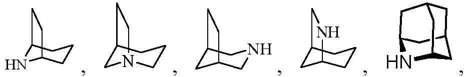 *etc.* The "nitrogen-containing bridged heterocycle", "oxygen-containing bridged heterocycle" and "sulfur-containing bridged heterocycle" optionally further comprise one or more additional heteroatoms selected from oxygen, nitrogen and sulfur.

As used herein, the term "spiro heterocycle" refers to a ring structure comprising one or more (e.g., 1, 2, 3 or 4) heteroatoms (e.g., oxygen atoms, nitrogen atoms, sulfur atoms) formed by two or more saturated rings sharing one ring atom, including, but not limited to, 5- to 10-membered spiro heterocycle, 6- to 10-membered spiro heterocycle, 6- to 10-membered nitrogen-containing spiro heterocycle, 6- to 10-membered oxygen-containing spiro heterocycle, and 6- to 10-membered sulfur-containing heterocycle, *etc*., such as

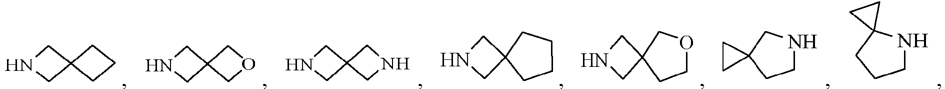

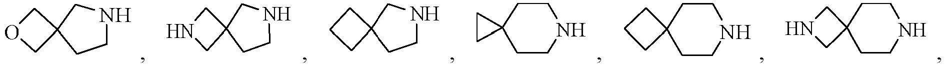

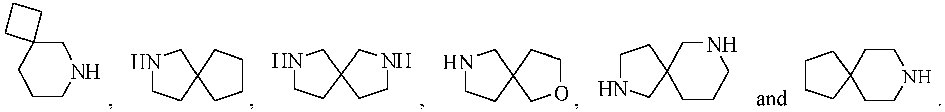

The "nitrogen-containing spiro heterocycle", "oxygen-containing spiro heterocycle" and "sulfur-containing spiro heterocycle" optionally further comprise one or more additional heteroatoms selected from oxygen, nitrogen and sulfur. The term "6- to 10-membered nitrogen-containing spiro heterocyclyl" refers to a spiro heterocyclyl group comprising a total of 6-10 ring atoms, and at least one of the ring atoms is a nitrogen atom.

As used herein, the term "aryl" or "aromatic ring" refers to an all-carbon monocyclic or fused-ring polycyclic aromatic group having a conjugated π electron system. For example, as used herein, the term "C₆₋₁₄ aryl" refers to an aromatic group containing 6 to 14 carbon atoms, such as phenyl or naphthyl. Aryl is optionally substituted with one or more (such as 1 to 3) suitable substituents (e.g., halogen, -OH, -CN, -NO₂, C₁₋₆ alkyl, *etc*.).

The term "aralkyl" preferably means aryl substituted alkyl, wherein the aryl and the alkyl are as defined herein. Normally, the aryl group may have 6-14 carbon atoms, and the alkyl group may have 1-6 carbon atoms. Exemplary aralkyl group includes, but is not limited to, benzyl, phenylethyl, phenylpropyl, phenylbutyl.

As used herein, the term "heteroaryl" or "heteroaromatic ring" refers to a monovalent monocyclic, bicyclic or tricyclic aromatic ring system having 5, 6, 8, 9, 10, 11, 12, 13 or 14 ring atoms, particularly 1 or 2 or 3 or 4 or 5 or 6 or 9 or 10 carbon atoms, and containing at least one heteroatom (such as O, N, or S), which can be same to different. Moreover, in each instance, it can be benzo-fused. In particular, heteroaryl is selected from the group consisting of thienyl, furyl, pyrrolyl, oxazolyl, thiazolyl, imidazolyl, pyrazolyl, isoxazolyl, isothiazolyl, oxadiazolyl, triazolyl, thiadiazolyl *etc.,* and benzo derivatives thereof; or pyridinyl, pyridazinyl, pyrimidinyl, pyrazinyl, triazinyl, *etc.,* and benzo derivatives thereof.

As used herein, the term "heteroaryl" or "heteroaromatic ring" encompasses fused ring structures, the attachment point between the fused ring structure and another group can be from any ring in the fused ring structure. As such, the heteroaryl group of the present invention further includes but is not limited to (mono)heteroaryl-fused (mono)heteroaryl, (mono)heteroaryl-fused (monocyclo)aryl, (mono)heteroaryl-fused (mono)heterocyclyl and (mono)heteroaryl-fused (mono)cycloalkyl, e.g., 5- to 6-membered (mono)heteroaryl-fused 5- to 6-membered (mono)heteroaryl, 5- to 6-membered (mono)heteroaryl-fused phenyl, 5- to 6-membered (mono)heteroaryl-fused 5- to 6-membered (mono)heterocyclyl or 5- to 6-membered (mono)heteroaryl-fused C₄₋₆ (mono)cycloalkyl (e.g., 5- to 6-membered heteroaryl-fused cyclobutyl, 5- to 6-membered heteroaryl-fused cyclopentyl or 5- to 6-membered heteroaryl-fused cyclohexyl), and the examples include but are not limited to indolyl, isoindolyl, indazolyl, benzoimidazole, quinolinyl, isoquinolinyl, 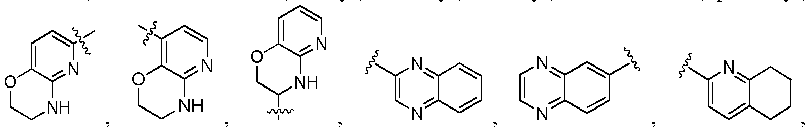 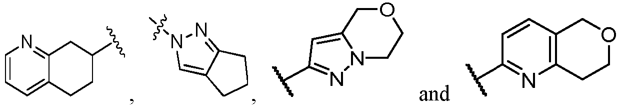 and the like.

As used herein, the term "halo" or "halogen" are defined to include F, Cl, Br, or I.

As used herein, the term "alkylthio" means an alkyl group as defined above attached to a parent molecular moiety through a sulfur atom. Representative examples of C₁₋₆ alkylthio include, but are not limited to, methylthio, ethylthio, *tert*-butylthio and hexylthio.

As used herein, the term "nitrogen containing heterocycle" refers to a saturated or unsaturated monocyclic or bicyclic group having 2, 3, 4, 5, 6, 7, 8, 9, 10, 11, 12 or 13 carbon atoms and at least one nitrogen atom in the ring, which may further optionally comprise one or more (e.g., one, two, three or four) ring members selected from the group consisting of N, O, C=O, S, S=O and S(=O)₂. The nitrogen-containing heterocycle is connected with the rest of the molecule through a nitrogen atom. The nitrogen-containing heterocycle is preferably a saturated nitrogen-containing monocyclic ring. In particular, a 3- to 14-membered nitrogen-containing heterocycle is a group having 3-14 carbon atoms and heteroatoms (at least one of which is a nitrogen atom) in the ring, including but not limited to a 3-membered nitrogen-containing heterocycle (such as aziridinyl), a 4-membered nitrogen-containing heterocycle (such as azetidinyl), a 5-membered nitrogen-containing heterocycle (such as pyrrolyl, pyrrolidinyl (pyrrolidinyl ring), pyrrolinyl, pyrrolidonyl, imidazolyl, imidazolidinyl, imidazolinyl, pyrazolyl, pyrazolinyl), 6-membered nitrogen-containing heterocycle (such as piperidinyl (piperidinyl ring), morpholinyl, thiomorpholinyl, piperazinyl), 7-membered nitrogen-containing heterocycle, *etc.*

The term "substituted" means that one or more (e.g., one, two, three, or four) hydrogens on the designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds.

If a substituent is described as being "optionally substituted," the substituent may be either (1) not substituted, or (2) substituted. If a carbon of a substituent is described as being optionally substituted with one or more of a list of substituents, one or more of the hydrogens on the carbon (to the extent there are any) may separately and/or together be replaced with an independently selected optional substituent. If a nitrogen of a substituent is described as being optionally substituted with one or more of a list of substituents, one or more of the hydrogens on the nitrogen (to the extent there are any) may each be replaced with an independently selected optional substituent.

If substituents are described as being "independently selected" from a group, each substituent is selected independent of the other(s). Each substituent therefore may be identical to or different from the other substituent(s).

As used herein, the term "one or more" means one or more than one (e.g., 2, 3, 4, 5 or 10) as reasonable.

As used herein, unless specified, the point of attachment of a substituent can be from any suitable position of the substituent.

When a bond to a substituent is shown to cross a bond connecting two atoms in a ring, then such substituent may be bonded to any of the ring-forming atoms in that ring that are substitutable.

The present invention also includes all pharmaceutically acceptable isotopically labeled compounds, which are identical to those of the present invention except that one or more atoms are replaced by an atom having the same atomic number, but an atomic mass or mass number different from the atomic mass or mass number which predominates in nature. Examples of isotopes suitable for inclusion in the compound of the present invention include, but are not limited to, isotopes of hydrogen (such as deuterium (D, ²H), tritium (T, ³H)); carbon, such as ¹¹C, ¹³C, and ¹⁴C; chlorine, such as ³⁶Cl; fluorine, such as ¹⁸F; iodine, such as ¹²³I and ¹²⁵I; nitrogen, such as ¹³N and ¹⁵N; oxygen, such as ¹⁵O, ¹⁷O, and ¹⁸O; phosphorus, such as ³²P; and sulfur, such as ³⁵S. Certain isotopically labeled compounds of the present invention, for example those incorporating a radioactive isotope, are useful in drug and/or substrate tissue distribution studies (e.g., assays). The radioactive isotopes tritium, i.e., ³H, and carbon-14, i.e., ¹⁴C, are particularly useful for this purpose in view of their ease of incorporation and ready means of detection. Substitution with positron-emitting isotopes, such as ¹¹C, ¹⁸F, ¹⁵O and ¹³N, can be useful in positron emission tomography (PET) studies for examining substrate receptor occupancy. Isotopically labeled compounds of the present invention can generally be prepared by processes analogous to those described in the accompanying Schemes and/or in the Examples and Preparations, by using an appropriate isotopically labeled reagent in place of the non-labeled reagent previously employed. Pharmaceutically acceptable solvates in accordance with the invention include those wherein the solvent of crystallization may be isotopically substituted, e.g., D₂O, acetone-*d₆*, or DMSO-*d₆*.

The term "stereoisomer" refers to isomers with at least one asymmetric center. A compound having one or more (e.g., one, two, three or four) asymmetric centers can give rise to a racemic mixture, single enantiomer, diastereomer mixture and individual diastereomer. Certain individual molecules may exist as geometric isomers (cis/trans). Similarly, the compound of the present invention may exist as a mixture of two or more structurally different forms in rapid equilibrium (generally referred to as tautomer). Typical examples of a tautomer include a keto-enol tautomer, phenol-keto tautomer, nitroso-oxime tautomer, imine-enamine tautomer and the like. It is to be understood that all such isomers and mixtures thereof in any proportion (such as 60%, 65%, 70%, 75%, 80%, 85%, 90%, 95%, 96%, 97%, 98%, and 99%) are encompassed within the scope of the present invention.

The carbon-carbon bonds of the compound of the present invention may be depicted herein using a solid line ( 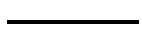 ), a solid wedge ( 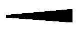 ), or a dotted wedge ( 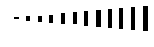 ). The use of a solid line to depict bonds to asymmetric carbon atoms is meant to indicate that all possible stereoisomers (e.g., specific enantiomers, racemic mixtures, *etc*.) at that carbon atom are included. The use of either a solid or dotted wedge to depict bonds to asymmetric carbon atoms is meant to indicate that the stereoisomer shown is present. When present in racemic compounds, solid and dotted wedges are used to define relative stereochemistry, rather than absolute stereochemistry. Unless stated otherwise, it is intended that the compound of the present invention can exist as stereoisomers, which include cis and trans isomers, optical isomers such as R and S enantiomers, diastereomers, geometric isomers, rotational isomers, conformational isomers, atropisomers, and mixtures thereof. The compound of the present invention may exhibit more than one type of isomerism, and consist of mixtures thereof (such as racemates and diastereomeric pairs).

The present invention includes all possible crystalline forms or polymorphs of the compound of the present invention, either as a single polymorph, or as a mixture of more than one polymorphs, in any ratio.

It also should be understood that, certain compounds of the present invention can be used for the treatment in a free form, or where appropriate, in a form of a pharmaceutically acceptable derivative. In the present invention, the pharmaceutically acceptable derivative includes, but is not limited to a pharmaceutically acceptable salt, ester, solvate, metabolite or prodrug, which can directly or indirectly provide the compound of the present invention or a metabolite or residue thereof after being administered to a patient in need thereof. Therefore, "the compound of the present invention" mentioned herein also means to encompass various derivative forms of the compound as mentioned above.

A pharmaceutically acceptable salt of the compound of the present invention includes an acid addition salt and a base addition salt thereof.

A suitable acid addition salt is formed from an acid which forms a pharmaceutically acceptable salt. Specific examples include aspartate, benzoate, bicarbonate/carbonate, bisulfate/sulfate, fumarate, gluceptate, gluconate, glucuronate, hexafluorophosphate, hydrobromide/bromide, hydroiodide/iodide, maleate, malonate, methylsulfate, naphthylate, nicotinate, nitrate, orotate, oxalate, palmitate and the like.

A suitable base addition salt is formed from a base which forms a pharmaceutically acceptable salt. Specific examples include aluminum, arginine, choline, diethylamine, lysine, magnesium, meglumine, potassium salts, and the like.

For a review on suitable salts, see "Hand book of Pharmaceutical Salts: Properties, Selection, and Use" by Stahl and Wermuth (Wiley-VCH, 2002). The method for preparing a pharmaceutically acceptable salt of the compound of the present invention is known to a person skilled in the art.

As used herein, the term "ester" refers to those derived from the compounds of the various formulae in the present application, which include physiologically-hydrolyzable esters (which may be hydrolyzed under physiological conditions to release the compounds of the present invention in the form of free acids or alcohols). The compound of the present invention itself may be an ester as well.

The compound of the present invention can exist as a solvate (preferably a hydrate), wherein the compound of the present invention contains a polar solvent, in particular water, methanol or ethanol for example, as a structural element of the crystal lattice of the compound. The amount of the polar solvent, in particular water, may exist in a stoichiometric or non-stoichiometric ratio.

The metabolite of the compound of the present invention, namely a substance formed *in vivo* upon administration of the compound of the present invention, is also included within the scope of the present invention. Such a product may result e.g., from the oxidation, reduction, hydrolysis, amidation, de-amidation, esterification, de-esterification, enzymolysis, and the like, of the administered compound. Accordingly, the present invention encompasses the metabolite of the compound of the present invention, including a compound produced by a method comprising contacting the compound of the present invention with a mammal for a period of time sufficient to result in a metabolic product thereof.

Also within the scope of the present invention is a prodrug of the compound of the invention, which is certain derivative of the compound of the invention that may have little or no pharmacological activity itself, but can, when administered into or onto the body, be converted into the compound of the invention having the desired activity, for example, by hydrolytic cleavage. In general, such prodrug will be a functional derivative of the compound which is readily converted *in vivo* into the compound with desired therapeutic activity. Further information on the use of the prodrug may be found in "Pro-drugs as Novel Delivery Systems", Vol. 14, ACS Symposium Series (T. Higuchi and V. Stella) and "Bioreversible Carriers in Drug Design," Pergamon Press, 1987 (edited by E. B. Roche, American Pharmaceutical Association). The prodrug in accordance with the invention can, for example, be produced by replacing appropriate functionalities present in the compound of the present invention with certain moieties known to those skilled in the art as "pro-moieties" as described, for example, in "Design of Prodrugs" by H. Bundgaard (Elsevier, 1985).

The present invention further encompasses the compound of the present invention having a protecting group. During any of the processes for preparation of the compound of the present invention, it may be necessary and/or desirable to protect sensitive or reactive groups on any of the molecules concerned, thereby resulting in the chemically protected form of the compound of the present invention. This may be achieved by means of conventional protecting groups, e.g., those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991, which is incorporated herein by reference. The protecting groups may be removed at a convenient subsequent stage using methods known from the art.

As used herein, the term "about" refers to a range within ±10%, preferably within ±5%, and more preferably within ±2% of the specified value.

### Compound

In some embodiments, the present application provides a compound or a pharmaceutically acceptable salt, ester, stereoisomer, tautomer, polymorph, solvate, metabolite, isotopically labeled compound or prodrug thereof, wherein the compound has the structure of Formula (I): 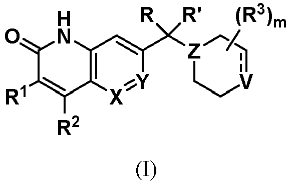 wherein:
--- is a single bond or a double bond;
X is N or CR⁵;
Y is Nor CR⁵';
Z is CR⁶ or N;
when --- is a single bond, V is CR⁷R^{A} or NR^{A}; when --- is a double bond, V is CR^{A};
R^{A} is selected from the group consisting of H, halogen, -OH, -NH₂, -CN, -NO₂, C₁₋₆ alkyl, C₁₋₆ haloalkyl, C₃₋₆ cyclic hydrocarbyl, 3- to 10-membered heterocyclyl, C₆₋₁₀ aryl, 5- to 14-membered heteroaryl, C₆₋₁₂ aralkyl, -C(=O)R^{a}, -OC(=O)R^{a}, -C(=O)OR^{a}, -OR^{a}, -SR^{a}, -S(=O)R^{a}, -S(=O)₂R^{a}, -S(=O)₂NR^{a}R^{b}, -NR^{a}R^{b}, - C(=O)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -C₁₋₆ alkylene-CN, - C₁₋₆ alkylene-OR^{a}, -C₁₋₆ alkylene-NR^{a}R^{b}, -O-C₁₋₆ alkylene-NR^{a}R^{b} and 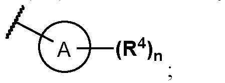
ring A is a C₃₋₆ hydrocarbon ring, 3- to 10-membered heterocycle, C₆₋₁₀ aromatic ring or 5- to 14-membered heteroaromatic ring;
R and R', at each occurrence, are each independently selected from the group consisting of H, halogen, -OH, -NH₂, -CN, -NO₂, C₁₋₆ alkyl, C₁₋₆ haloalkyl, C₃₋₆ cyclic hydrocarbyl, 3- to 10-membered heterocyclyl, C₆₋₁₀ aryl, 5- to 14-membered heteroaryl, C₆₋₁₂ aralkyl, -C(=O)R^{a}, -OC(=O)R^{a}, -C(=O)OR^{a}, -OR^{a}, -SR^{a}, - S(=O)R^{a}, -S(=O)₂R^{a}, -S(=O)₂NR^{a}R^{b}, -NR^{a}R^{b}, -C(=O)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -C₁₋₆ alkylene-CN, -C₁₋₆ alkylene-OR^{a}, -C₁₋₆ alkylene-NR^{a}R^{b} and -O-C₁₋₆ alkylene-NR^{a}R^{b}; preferably, R and R' are each independently selected from the group consisting of H, -CN and C₁₋₆ alkyl;
R¹ and R² are each independently selected from the group consisting of halogen, -OH, -NH₂, -CN, -NO₂, C₁₋₆ alkyl, C₁₋₆ haloalkyl, C₃₋₆ cyclic hydrocarbyl, 3- to 10-membered heterocyclyl, C₆₋₁₀ aryl, 5- to 14-membered heteroaryl, C₆₋₁₂ aralkyl, -C(=O)R^{a}, -OC(=O)R^{a}, -C(=O)OR^{a}, -OR^{a}, -SR^{a}, -S(=O)R^{a}, -S(=O)₂R^{a}, - S(=O)₂NR^{a}R^{b}, -NR^{a}R^{b}, -C(=O)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -C₁₋₆ alkylene-CN, -C₁₋₆ alkylene-OR^{a}, -C₁₋₆ alkylene-NR^{a}R^{b} and -O-C₁₋₆ alkylene-NR^{a}R^{b};
R³, at each occurrence, is each independently selected from the group consisting of halogen, -OH, =O, -NH₂, -CN, -NO₂, C₁₋₆ alkyl, C₁₋₆ haloalkyl, C₃₋₆ cyclic hydrocarbyl, 3- to 10-membered heterocyclyl, C₆₋₁₀ aryl, 5- to 14-membered heteroaryl, C₆₋₁₂ aralkyl, -C(=O)R^{a}, -OC(=O)R^{a}, -C(=O)OR^{a}, -OR^{a}, -SR^{a}, -S(=O)R^{a}, -S(=O)₂R^{a}, -S(=O)₂NR^{a}R^{b}, -NR^{a}R^{b}, -C(=O)NR^{a}R^{b}, -NR^{a}-C(=O)R^{b}, -NR^{a}-C(=O)OR^{b}, -NR^{a}-S(=O)₂-R^{b}, - NR^{a}-C(=O)-NR^{a}R^{b}, -C₁₋₆ alkylene-CN, -C₁₋₆ alkylene-OR^{a}, -C₁₋₆ alkylene-NR^{a}R^{b} and -O-C₁₋₆ alkylene-NR^{a}R^{b};
when m>1, two R³ groups optionally together form -C₁₋₆ alkylene- or -C₂₋₆ alkenylene-, the alkylene chain and alkenylene chain are optionally interrupted by one or more groups independently selected from the group consisting of O, C(=O), C(=O)O, NR, S, S=O and S(=O)z;
or, R³ and R^{A} together with the groups to which they are attached optionally form a C₃₋₆ hydrocarbon ring, 3- to 10-membered heterocycle, C₆₋₁₀ aromatic ring or 5- to 14-membered heteroaromatic ring;
R4, at each occurrence, is each independently selected from the group consisting of halogen, -OH, -NH₂, -CN, -NO₂, C₁₋₆ alkyl, C₁₋₆ haloalkyl, C₃₋₆ cyclic hydrocarbyl, 3- to 10-membered heterocyclyl, C₆₋₁₀ aryl, 5-to 14-membered heteroaryl, C₆₋₁₂ aralkyl, -C(=O)R^{a}, -OC(=O)R^{a}, -C(=O)OR^{a}, -OR^{c}, -SR^{a}, -S(=O)R^{a}, - S(=O)₂R^{a}, -S(=O)₂NR^{a}R^{b}, -NR^{a}R^{b}, -C(=O)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -C₁₋₆ alkylene-CN, -C₁₋₆ alkylene-OR^{a}, -C₁₋₆ alkylene-NR^{a}R^{b} and -O-C₁₋₆ alkylene-NR^{a}R^{b}; when two R⁴ groups are ortho to each other on ring A, the two R⁴ groups together with the groups to which they are attached optionally form 3- to 10-membered heterocycle or 5- to 14-membered heteroaromatic ring;
or, R³ and R⁴ together with the groups to which they are attached optionally form a C₃₋₆ hydrocarbon ring, 3- to 10-membered heterocycle, C₆₋₁₀ aromatic ring or 5- to 14-membered heteroaromatic ring;
R⁵, R⁵', R⁶ and R⁷, at each occurrence, are each independently selected from the group consisting of H, halogen, -OH, -NH₂, -CN, -NOz, C₁₋₆ alkyl, C₁₋₆ haloalkyl, C₃₋₆ cyclic hydrocarbyl, 3- to 10-membered heterocyclyl, C₆₋₁₀ aryl, 5- to 14-membered heteroaryl, C₆₋₁₂ aralkyl, -C(=O)R^{a}, -OC(=O)R^{a}, -C(=O)OR^{a}, - OR^{a}, -SR^{a}, -S(=O)R^{a}, -S(=O)₂R^{a}, -S(=O)₂NR^{a}R^{b}, -NR^{a}R^{b}, -C(=O)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -C₁₋₆ alkylene-CN, -C₁₋₆ alkylene-OR^{a}, -C₁₋₆ alkylene-NR^{a}R^{b} and -O-C₁₋₆ alkylene-NR^{a}R^{b};
R^{a} and R^{b}, at each occurrence, are each independently selected from the group consisting of H, C₁₋₆ alkyl, C₁₋₆ haloalkyl, C₂₋₆ alkenyl, C₃₋₁₀ cyclic hydrocarbyl, 3- to 10-membered heterocyclyl, C₆₋₁₀ aryl, 5- to 14-membered heteroaryl and C₆₋₁₂ aralkyl;
the above alkyl, alkylene, haloalkyl, alkenyl, alkenylene, hydrocarbon ring, cyclic hydrocarbyl, heterocycle, heterocyclyl, aryl, aromatic ring, heteroaryl, heteroaromatic ring and aralkyl, at each occurrence, are each optionally substituted with one or more substituents independently selected from the group consisting of: halogen, -OH, =O, -NH₂, -CN, -NOz, C₁₋₆ alkyl, C₁₋₆ haloalkyl, C₃₋₆ cyclic hydrocarbyl, 3- to 10-membered heterocyclyl, C₆₋₁₀ aryl, 5- to 14-membered heteroaryl, C₆₋₁₂ aralkyl, -C(=O)R^{c}, -OC(=O)R^{c}, - C(=O)OR^{c}, -OR^{c}, -SR^{c}, -S(=O)R^{c}, -S(=O)₂R^{c}, -S(=O)₂NR^{c}R^{d}, -NR^{c}R^{d}, -C(=O)NR^{c}R^{d}, -NR^{c}-C(=O)R^{d}, -NR^{c}-C(=O)OR^{d}, -NR^{c}-S(=O)₂-R^{d}, -NR^{c}-C(=O)-NR^{c}R^{d}, -C₁₋₆ alkylene-OR^{a}, -C₁₋₆ alkylene-NR^{c}R^{d} and -O-C₁₋₆ alkylene-NR^{c}R^{d}, the alkyl, alkylene, haloalkyl, cyclic hydrocarbyl, heterocyclyl, aryl, heteroaryl and aralkyl are further optionally substituted with one or more substituents independently selected from the group consisting of: halogen, -OH, =O, -NH₂, -CN, -NOz, C₁₋₆ alkyl, C₁₋₆ haloalkyl, C₃₋₆ cyclic hydrocarbyl, 3- to 10-membered heterocyclyl, C₆₋₁₀ aryl, 5- to 14-membered heteroaryl and C₆₋₁₂ aralkyl;
R^{c} and R^{d}, at each occurrence, are each independently selected from the group consisting of H, C₁₋₆ alkyl, C₁₋₆ haloalkyl, C₃₋₁₀ cyclic hydrocarbyl, 3- to 10-membered heterocyclyl, C₆₋₁₀ aryl, 5- to 14-membered heteroaryl and C₆₋₁₂ aralkyl; and
m and n are each independently an integer of 0, 1, 2, 3, or 4.

In some embodiments, the present application provides the above compound or a pharmaceutically acceptable salt, ester, stereoisomer, tautomer, polymorph, solvate, metabolite, isotopically labeled compound or prodrug thereof, wherein R^{A} is selected from the group consisting of C₁₋₆ haloalkyl (preferably 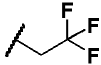 ), -C(=O)R^{a} (preferably 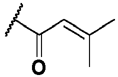 ) and

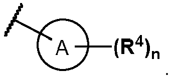

In some embodiments, the present application provides the above compound or a pharmaceutically acceptable salt, ester, stereoisomer, tautomer, polymorph, solvate, metabolite, isotopically labeled compound or prodrug thereof, wherein the compound has the structure of Formula (I)-1 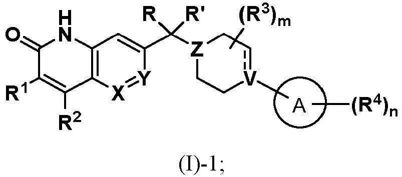 preferably, the compound has the structure of Formula (II), (III), (IV) or (V):

| | |
|---|---|
| 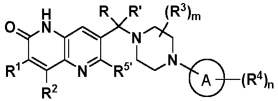 | 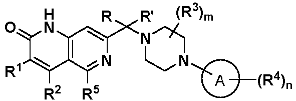 |
| (II) | (III) |
| 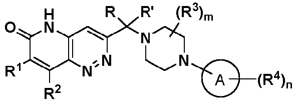 | 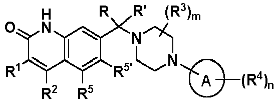 |
| (IV) | (V). |

In some embodiments, the present application provides the above compound or a pharmaceutically acceptable salt, ester, stereoisomer, tautomer, polymorph, solvate, metabolite, isotopically labeled compound or prodrug thereof, wherein R¹ and R² are each independently halogen, -CN, C₁₋₆ alkyl or C₁₋₆ haloalkyl; for example, R¹ and R² are each independently C₁₋₆ alkyl or C₁₋₆ haloalkyl;
preferably, R¹ and R² are each independently F, -CN, methyl, difluoromethyl, trifluoromethyl, ethyl, n-propyl or isopropyl; for example, R¹ and R² are each independently methyl, trifluoromethyl, ethyl, n-propyl or isopropyl;
most preferably, R¹ is trifluoromethyl, and R² is methyl; or R¹ and R² are both methyl.

In some embodiments, the present application provides the above compound or a pharmaceutically acceptable salt, ester, stereoisomer, tautomer, polymorph, solvate, metabolite, isotopically labeled compound or prodrug thereof, wherein R³, at each occurrence, is each independently halogen, -OH, =O, - NH₂, -CN, C₁₋₆ alkyl, C₁₋₆ haloalkyl, -C₁₋₆ alkylene-CN, -C₁₋₆ alkylene-OR^{a} or -C₁₋₆ alkylene-NR^{a}R^{b}; when m>1, two R³ groups optionally together form -C₁₋₄ alkylene-;
preferably, R³, at each occurrence, is each independently =O, -CN, C₁₋₆ alkyl, C₁₋₆ haloalkyl, -C₁₋₆ alkylene-CN, -C₁₋₆ alkylene-OR^{a} or -C₁₋₆ alkylene-NR^{a}R^{b}; when m>1, two R³ groups optionally together form -C₁₋₄ alkylene-;
more preferably, R³, at each occurrence, is each independently =O, -CN, -CH₃, -CF₃, -CH₂CN, -CH₂NH₂, -CH₂CH₂NH₂, -CH₂OH, -CH₂CH₂OH or -CH₂OCH₃; when m>1, two R³ groups optionally together form - CH₂CH₂-.

In some embodiments, R³ and R^{A} together with the groups to which they are attached optionally form 5- to 6-membered heteroaromatic ring (preferably a triazole ring), which is optionally substituted with C₁₋₆ haloalkyl (preferably trifluoromethyl).

In some embodiments, the present application provides the above compound or a pharmaceutically acceptable salt, ester, stereoisomer, tautomer, polymorph, solvate, metabolite, isotopically labeled compound or prodrug thereof, wherein R⁶ and R⁷, at each occurrence, are each independently selected from the group consisting of H, halogen, -OH, -CN, -C₁₋₆ alkylene-OR^{a} and -C₁₋₆ alkylene-NR^{a}R^{b};
R⁶ and R⁷, at each occurrence, are each independently selected from the group consisting of H, -F, -OH, -CN, -CH₂OH and -CH₂NH₂.

In some embodiments, the present application provides the above compound or a pharmaceutically acceptable salt, ester, stereoisomer, tautomer, polymorph, solvate, metabolite, isotopically labeled compound or prodrug thereof, wherein 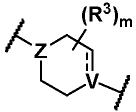 is selected from the group consisting of 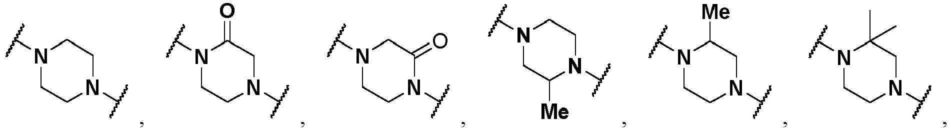 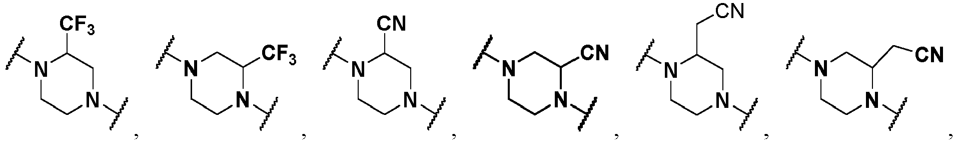 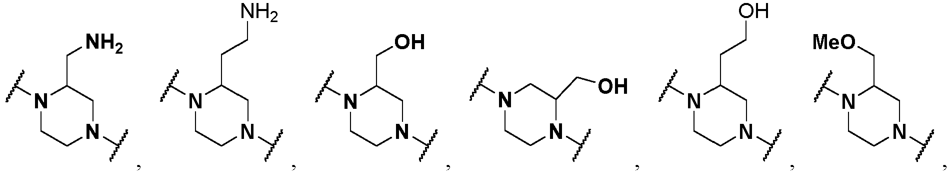 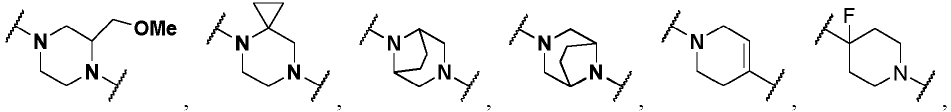 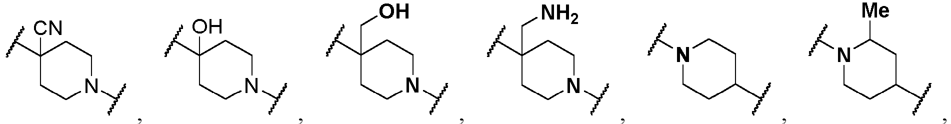 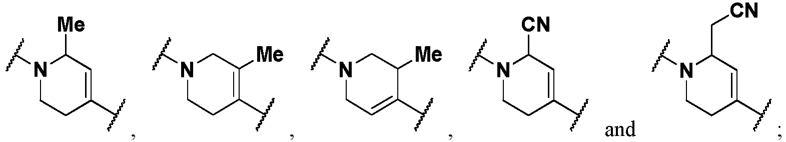 preferably, 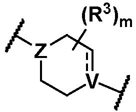 is selected from the group consisting of

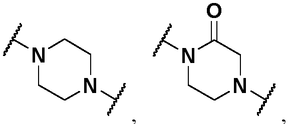

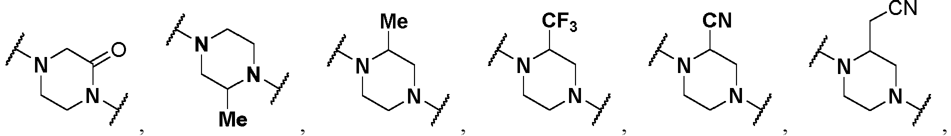

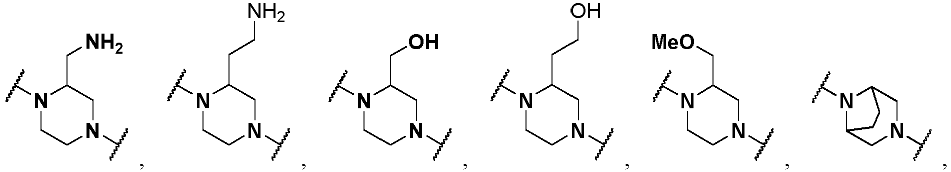

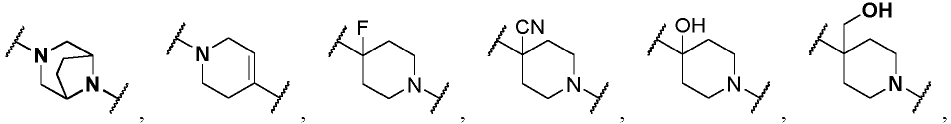

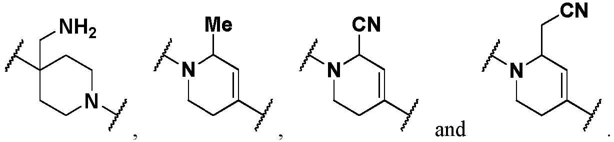

In some embodiments, the present application provides the above compound or a pharmaceutically acceptable salt, ester, stereoisomer, tautomer, polymorph, solvate, metabolite, isotopically labeled compound or prodrug thereof, wherein R⁴, at each occurrence, is each independently halogen, -CN, C₁₋₆ alkyl, C₁₋₆ haloalkyl, C₃₋₆ cyclic hydrocarbyl, 5- to 14-membered heteroaryl, -S(=O)₂NR^{a}R^{b}, -C(=O)NR^{a}R^{b}, -NR^{a}-C(=O)R^{b}-NR^{a}-C(=O)-NR^{a}R^{b} or -C₁₋₆ alkylene-OR^{a}; when two R⁴ groups are ortho to each other on ring A, the two R⁴ groups together with the groups to which they are attached optionally form 5- to 6-membered heterocycle or 5- to 6-membered heteroaromatic ring;
preferably, R⁴, at each occurrence, is each independently F, -CN, -CH₃, -CF₃, 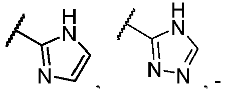 S(=O)₂NHCH₃, -C(=O)NH₂, -C(=O)NHCH₃, -C(=O)NHCD₃, -C(=O)NHCH₂CH₃, -C(=O)NHCH₂CF₃, - C(=O)NHCH₂CH₂OH, -C(=O)NH(cyclopropyl), -NHC(=O)CH₃, -NHC(=O)(cyclopropyl), - NHC(=O)NHCH₃, -CH₂OH, 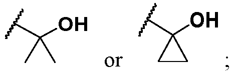 when two R⁴ groups are ortho to each other on ring A, the two R⁴ groups together with the groups to which they are attached optionally form 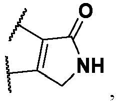 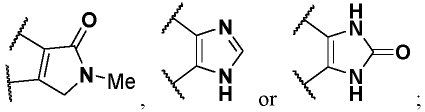
more preferably, R⁴, at each occurrence, is each independently F, -CN, -CH₃, -CF₃, 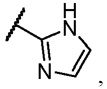 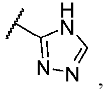 -S(=O)₂NHCH₃, -C(=O)NH₂, -C(=O)NHCH₃, -NHC(=O)CH₃, -NHC(=O)(cyclopropyl), - NHC(=O)NHCH₃, -CH₂OH, 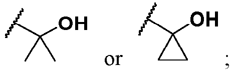 when two R⁴ groups are ortho to each other on ring A, the two R⁴ groups together with the groups to which they are attached optionally form 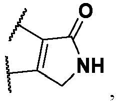 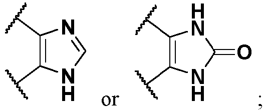
or, R³ and R⁴ together with the groups to which they are attached optionally form 5- to 6-membered heteroaromatic ring, preferably an imidazole ring.

In some embodiments, the present application provides the above compound or a pharmaceutically acceptable salt, ester, stereoisomer, tautomer, polymorph, solvate, metabolite, isotopically labeled compound or prodrug thereof, wherein ring A is a C₄₋₆ hydrocarbon ring, 5- to 6-membered heterocycle, C₆ aromatic ring or 5- to 6-membered heteroaromatic ring, more preferably is a bicyclo[1.1.1]pentane ring, piperidine ring, benzene ring, imidazole ring, thiazole ring, pyridine ring, pyrazine ring, pyridazine ring, or pyrimidine ring, and most preferably is a bicyclo[1.1.1]pentane ring, piperidine ring, benzene ring, imidazole ring, thiazole ring, pyridine ring, pyridazine ring, or pyrimidine ring.

In some embodiments, the present application provides the above compound or a pharmaceutically acceptable salt, ester, stereoisomer, tautomer, polymorph, solvate, metabolite, isotopically labeled compound or prodrug thereof, wherein 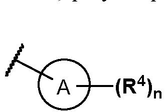 is 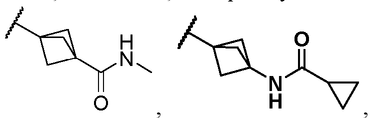 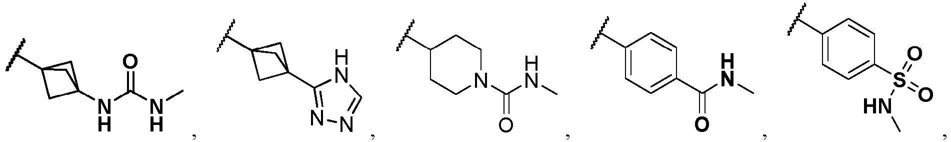 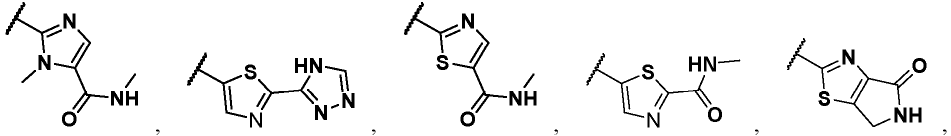 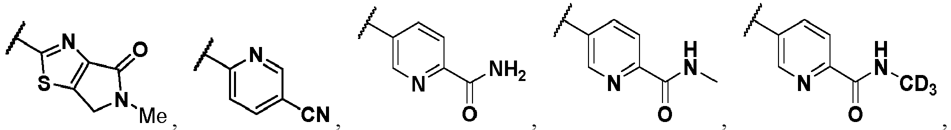 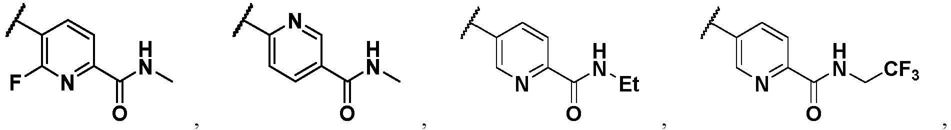 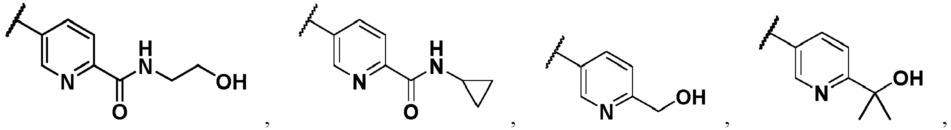 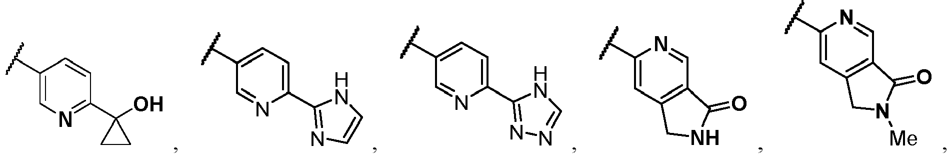 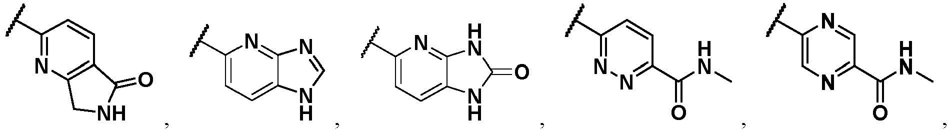 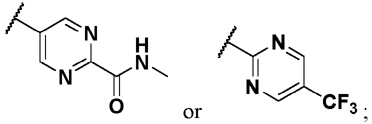 preferably, 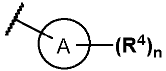 is

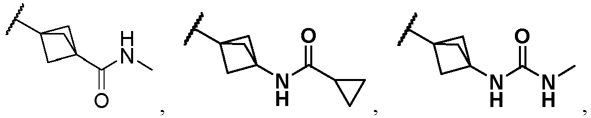

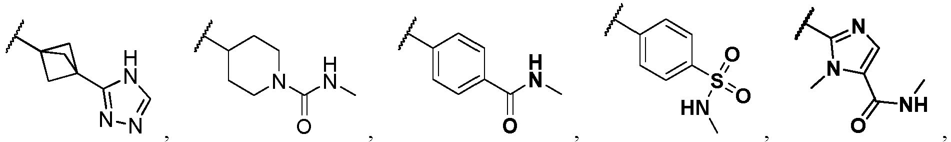

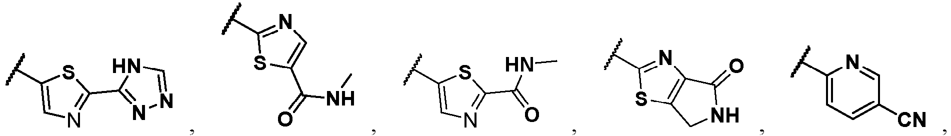

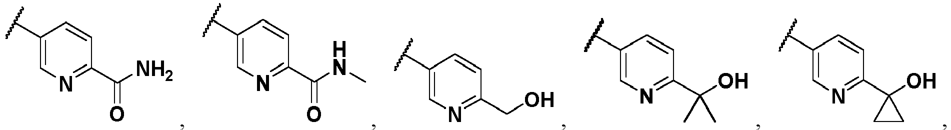

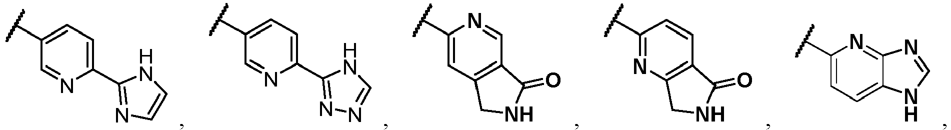

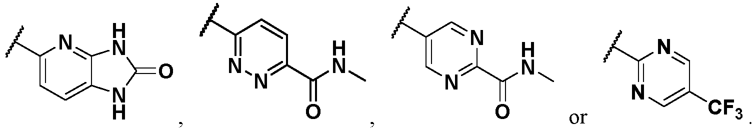

The present invention encompasses compounds resulting from any combination of the various embodiments.

In preferred embodiments, the present invention provides the above compound or a pharmaceutically acceptable salt, ester, stereoisomer, tautomer, polymorph, solvate, metabolite, isotopically labeled compound or prodrug thereof, wherein the compound is selected from the group consisting of:

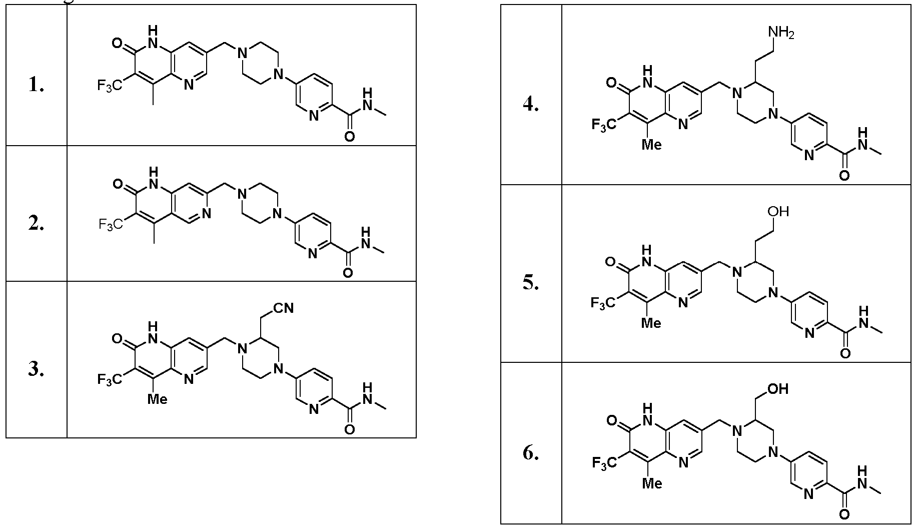

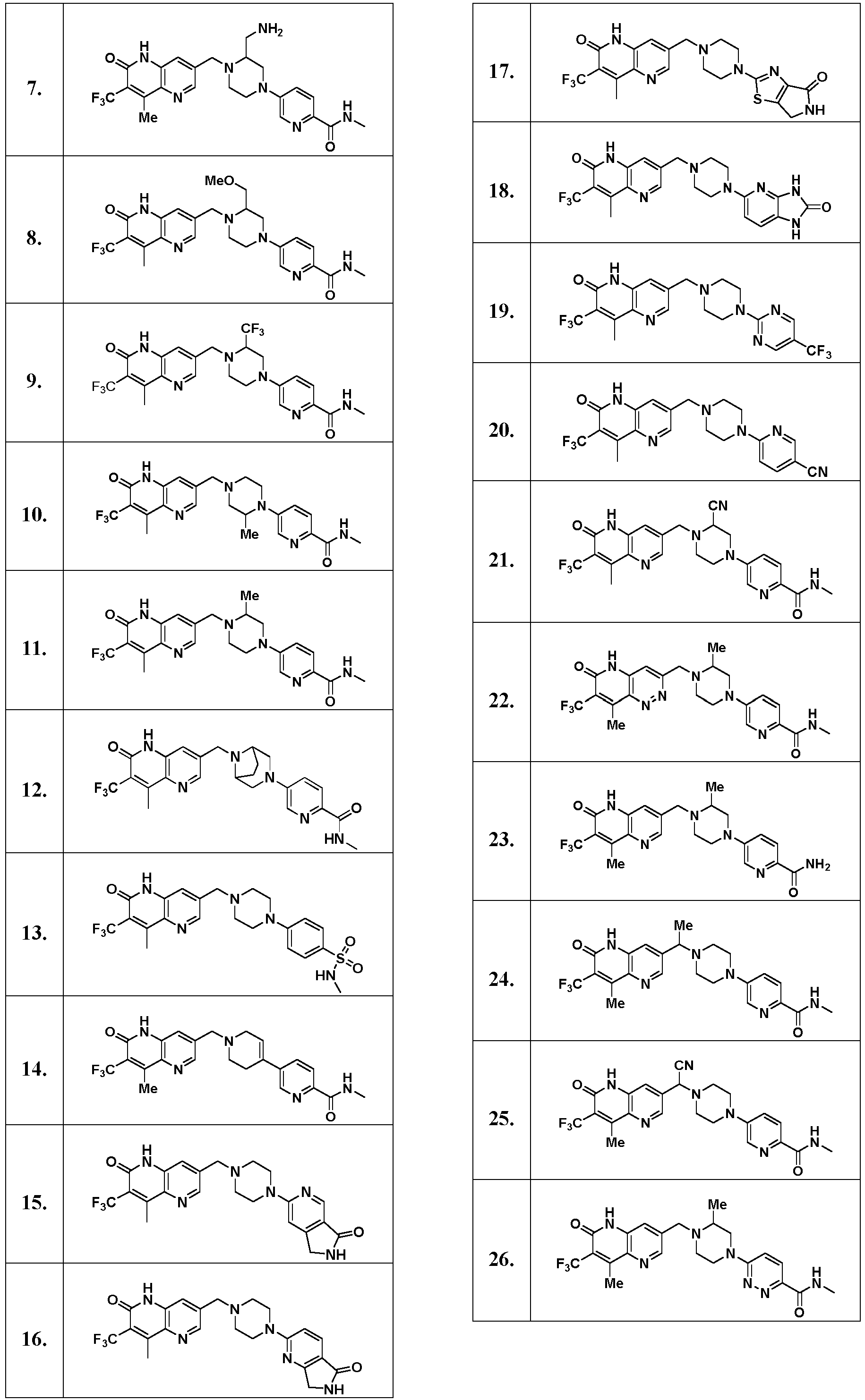

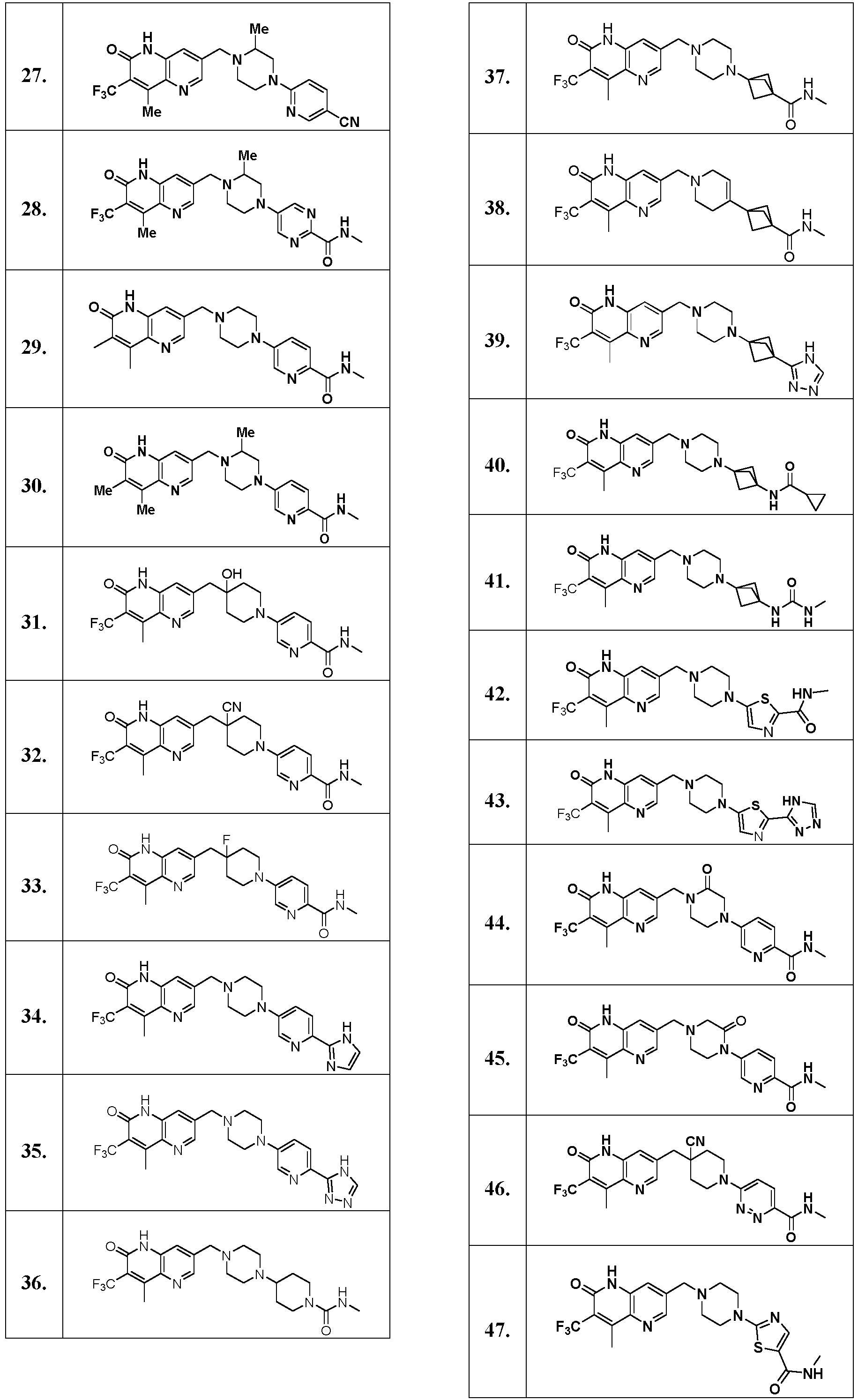

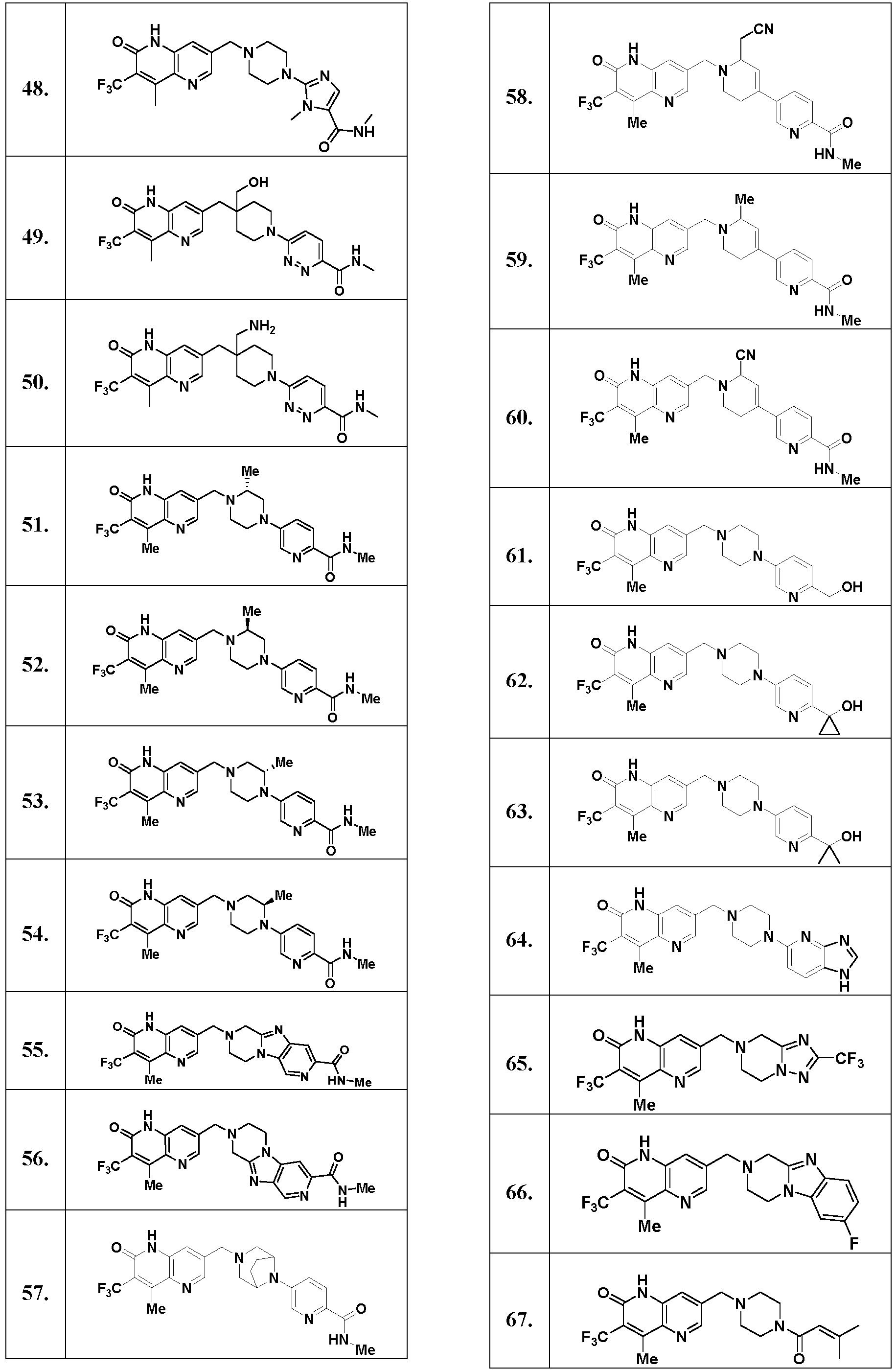

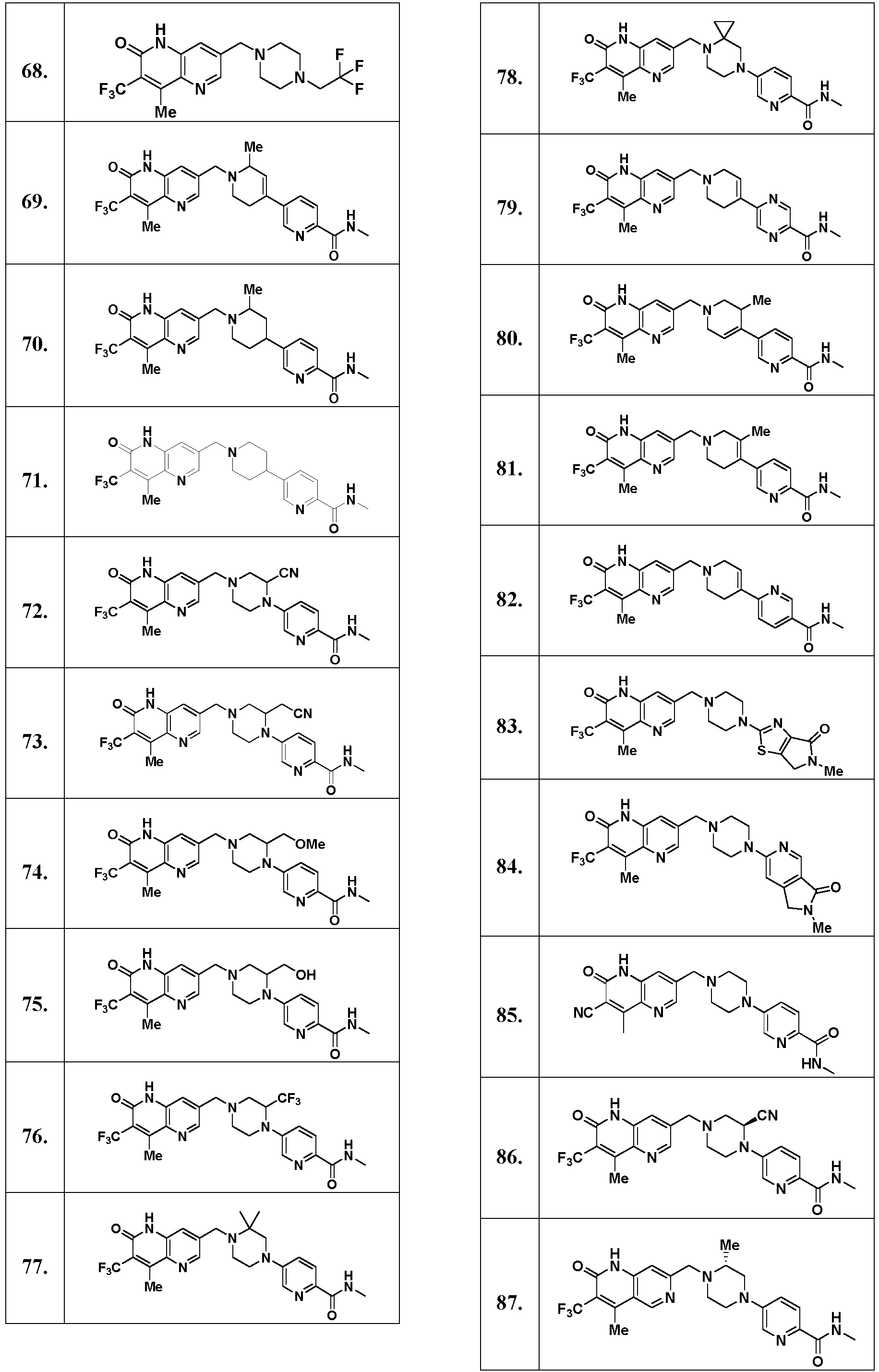

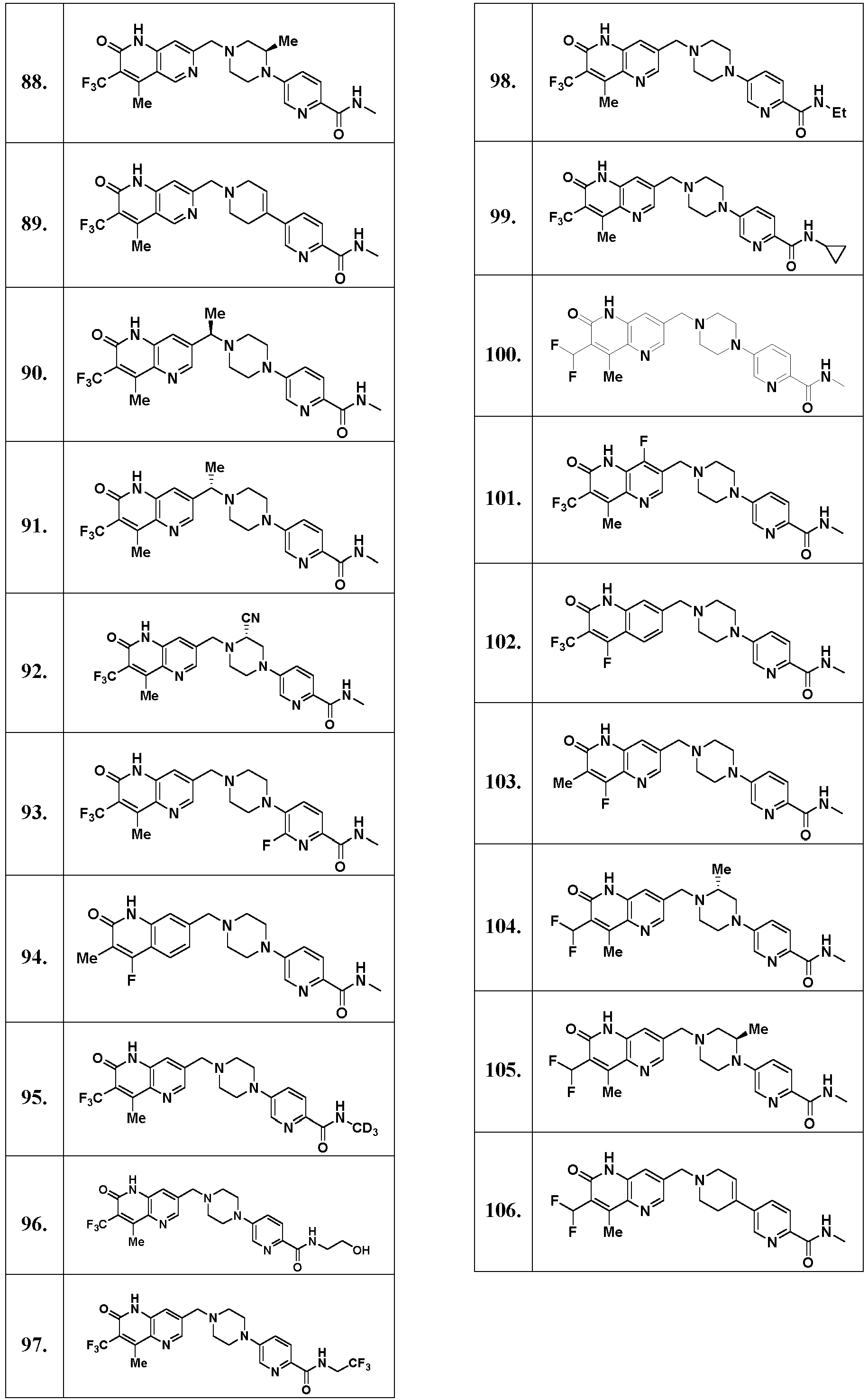

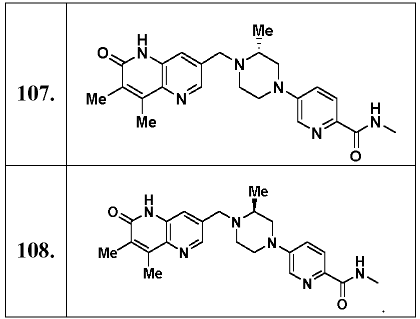

### Pharmaceutical composition and therapeutic method

In some embodiments, the present invention provides a pharmaceutical composition comprising a prophylactically or therapeutically effective amount of the compound of the present invention or a pharmaceutically acceptable salt, ester, stereoisomer, tautomer, polymorph, solvate, metabolite, isotopically labeled compound or prodrug thereof, and one or more pharmaceutically acceptable carriers, and the pharmaceutical composition is preferably in the form of a solid, liquid or transdermal formulation.

In some embodiments, the present invention provides the use of the compound of the present invention or a pharmaceutically acceptable salt, ester, stereoisomer, tautomer, polymorph, solvate, metabolite, isotopically labeled compound or prodrug thereof or the pharmaceutical composition of the present invention in the manufacture of a medicament for use as a PARP inhibitor (preferably as a PARP1 selective inhibitor).

In some embodiments, the present invention provides the compound of the present invention or a pharmaceutically acceptable salt, ester, stereoisomer, tautomer, polymorph, solvate, metabolite, isotopically labeled compound or prodrug thereof or the pharmaceutical composition of the present invention, for use as a PARP inhibitor (preferably as a PARP1 selective inhibitor).

In some embodiments, the present invention provides a method for the prophylaxis or the treatment of a disease that can be ameliorated by inhibiting PARP (preferably PARP-1), the method comprising administering to a subject in need thereof an effective amount of the compound of the present invention or a pharmaceutically acceptable salt, ester, stereoisomer, tautomer, polymorph, solvate, metabolite, isotopically labeled compound or prodrug thereof or the pharmaceutical composition of the present invention.

The disease that can be ameliorated by inhibiting PARP (preferably PARP-1) is, for example, cancer, including but not limited to ovarian cancer, breast cancer, prostate cancer, kidney cancer, liver cancer, pancreatic cancer, gastric cancer, lung cancer, head and neck cancer, thyroid cancer, malignant glioma, leukemia, lymphoma, and multiple myeloma.

The term "pharmaceutically acceptable carrier" in the present invention refers to a diluent, auxiliary material, excipient, or vehicle with which a therapeutic is administered, and it is, within the scope of sound medical judgment, suitable for contact with the tissues of human beings and animals without excessive toxicity, irritation, allergic response, or other problem or complication, commensurate with a reasonable benefit/risk ratio.

The pharmaceutically acceptable carrier which can be employed in the pharmaceutical composition of the present invention includes, but is not limited to sterile liquids, such as water and oils, including those of petroleum, animal, vegetable or synthetic origin, such as peanut oil, soybean oil, mineral oil, sesame oil and the like. Water is an exemplary carrier when the pharmaceutical composition is administered intravenously. Physiological salines as well as aqueous dextrose and glycerol solutions can also be employed as liquid carriers, particularly for injectable solutions. Suitable pharmaceutical excipients include starch, glucose, lactose, sucrose, gelatin, maltose, chalk, silica gel, sodium stearate, glycerol monostearate, talc, sodium chloride, dried skim milk, glycerol, propylene glycol, water, ethanol and the like. The pharmaceutical composition, if desired, can also contain minor amounts of wetting or emulsifying agents, or pH buffering agents. Oral formulations can include standard carriers such as pharmaceutical grades of mannitol, lactose, starch, magnesium stearate, sodium saccharine, cellulose, magnesium carbonate, *etc.* Examples of suitable pharmaceutical carriers are described in e.g., Remington's Pharmaceutical Sciences (1990).

The pharmaceutical composition of the present invention can act systemically and/or topically. To this end, it can be administered through a suitable route, such as through injection, (intravenous, intraarterial, subcutaneous, intraperitoneal, intramuscular injection, including dripping), or transdermal administration, or administered via oral, buccal, nasal, transmucosal, topical, as an ophthalmic formulation, or via inhalation.

For these routes of administration, the pharmaceutical composition of the present invention can be administered in a suitable dosage form.

Such dosage forms include, but are not limited to tablets, capsules, lozenges, hard candies, powders, sprays, creams, salves, suppositories, gels, pastes, lotions, ointments, aqueous suspensions, injectable solutions, elixirs, and syrups.

As used herein, the term "effective amount" refers to the amount of a compound being administered which will relieve to some extent one or more of the symptoms of the disorder being treated.

Dosage regimens may be adjusted to provide the optimum desired response. For example, a single bolus may be administered, several divided doses may be administered over time, or the dose may be proportionally reduced or increased as indicated by the exigencies of the therapeutic situation. It is to be noted that dosage values may vary with the type and severity of the condition to be alleviated, and may include single or multiple doses. It is to be further understood that for any particular subject, specific dosage regimens should be adjusted over time according to the individual need and the professional judgment of the person administering or supervising the administration of the composition.

The amount of the compound of the present invention administered will be dependent on the subject being treated, the severity of the disorder or condition, the rate of administration, the disposition of the compound and the discretion of the prescribing physician. Generally, an effective dosage is in the range of about 0.0001 to about 50 mg per kg body weight per day, for example about 0.01 to about 10 mg/kg/day, in single or divided doses. For a 70 kg human, this would amount to about 0.007 mg to about 3500 mg/day, for example about 0.7 mg to about 700 mg/day. In some instances, dosage levels below the lower limit of the aforesaid range may be more than adequate, while in other cases, still larger doses may be employed without causing any harmful side effect, provided that such larger doses are first divided into several small doses for administration throughout the day.

The content or dosage of the compound of the present invention in the pharmaceutical composition is about 0.01 mg to about 1000 mg, suitably 0.1-500 mg, preferably 0.5-300 mg, more preferably 1-150 mg, particularly preferably 1-50 mg, e.g., 1.5 mg, 2 mg, 4 mg, 10 mg, 25 mg, *etc.*

Unless otherwise indicated, the term "treating" or "treatment", as used herein, means reversing, alleviating, inhibiting the progress of, or preventing the disorder or condition to which such term applies, or one or more symptoms of such disorder or condition.

As used herein, the term "subject" includes a human or non-human animal. An exemplary human subject includes a human subject having a disease (such as one described herein) (referred to as a patient), or a normal subject. The term "non-human animal" as used herein includes all vertebrates, such as non-mammals (e.g., birds, amphibians, reptiles) and mammals, such as non-human primates, livestock and/or domesticated animals (such as sheep, dog, cat, cow, pig and the like).

In some embodiments, the pharmaceutical composition of the present invention can further comprise one or more additional therapeutic agents or prophylactic agents.

### Example

The present invention is further described in conjunction with the following examples, but these examples are not intended to limit the scope of the present invention.

Experimental methods in the examples of the present invention, unless otherwise specified, were generally carried out under conventional conditions or according to the conditions recommended by the manufacturer of the raw materials or commercial products. Reagents without specific sources mentioned were conventional reagents available on the market. All evaporations were carried out using a rotary evaporator under vacuum. Analytical samples were dried under vacuum (1-5 mm Hg) at room temperature. Thin-layer chromatography (prep-TLC) or high-performance liquid chromatography (HPLC) separations were performed on preparative silica gel plates. Flash column chromatography purification was carried out using SEPAFLASH pre-packed silica gel columns, and mixed solvent systems are reported by volume ratio.

The structures of the compounds were confirmed by nuclear magnetic resonance spectroscopy (NMR) or mass spectrometry (MS).

NMR spectra were recorded using a Varian NMR System 400MHz high-resolution NMR instrument. Chemical shifts (δ) are given in parts per million (ppm). The solvents for the analysis were deuterated chloroform (CDCl₃), hexadeuterated dimethyl sulfoxide (DMSO-*d₆*), or deuterated methanol (CD₃OD). Tetramethylsilane (TMS) was used as the internal standard. The multiplicity of ¹H NMR spectral peaks is abbreviated as follows: s for singlet, bs for broad singlet, d for doublet, t for triplet, q for quartet, m for multiplet, dd for double doublet, *etc.*

The liquid chromatography-mass spectrometry (LC-MS) instrument used was an Agilent 1260 series 6135 mass spectrometer with electrospray ionization, and the analysis method was as follows:
Agilent LC-MS 1260-6135, column: Agilent ZORBAX SB-C18 (50 mm × 2.1 mm × 5 µm); column temperature: 25°C; flow rate: 1.5 mL/min; mobile phase: a gradient from 95% [water + 0.1% trifluoroacetic acid] and 5% [acetonitrile + 0.1% trifluoroacetic acid] to 5% [water + 0.05% trifluoroacetic acid] and 95% [acetonitrile + 0.05% trifluoroacetic acid] over 2.5 minutes.

The high-performance liquid chromatography instrument used was a Hanbang 50 ml binary semipreparative liquid chromatography system, column type: Hedera ODS-2 250* 10 mm* 10 µm; mobile phase: phase A [water + 0.1% trifluoroacetic acid], phase B [acetonitrile + 0.1% trifluoroacetic acid];

Thin-layer chromatography silica gel plates used were Huanghai GF254 silica gel plates.

Unless otherwise specified, reactions in the examples were carried out under a nitrogen atmosphere.

The progress of reactions in the examples was monitored by thin-layer chromatography (TLC) or liquid chromatography-mass spectrometry (LC-MS).

The abbreviations used in the present invention have the following meanings:

| **Abbreviation** | **Meaning** |
|---|---|
| ACN/MeCN | Acetonitrile |
| Ac₂O | Acetic anhydride |
| AcOH/CH₃COOH | Acetic acid |
| aq. | Aqueous solution |
| Bn | Benzyl |
| Boc | *tert-*Butyloxycarbonyl |
| (Boc)₂O | Di-*tert*-butyl dicarbonate |
| n-Bu | n-Butyl |
| n-BuOH | n-Butanol |
| Cbz | Benzyloxycarbonyl |
| CDI | N,N'-Carbonyldiimidazole |
| Cs₂CO₃ | Cesium carbonate |
| DAST | Diethylaminosulfur trifluoride |
| DCM | Dichloromethane |
| DEAD | Diethyl azodicarboxylate |
| DIEA/DIPEA | N,N-Diisopropylethylamine |
| DMA | N,N-Dimethylacetamide |
| DMAc | N,N-Dimethylacetamide |
| DMAP | 4-Dimethylaminopyridine |
| DMF | N,N-Dimethylformamide |
| DMSO | Dimethyl sulfoxide |
| DOX / 1,4-dioxane | 1,4-Dioxane |
| EA/EtOAc | Ethyl acetate |
| EDCI | N-(3-Dimethylaminopropyl)-N'-ethylcarbodiimide hydrochloride |
| Et₃N | Triethylamine |
| EtNH₂ | Ethylamine |
| EtOH | Ethanol |
| h | Hour |
| H₂ | Hydrogen |
| HATU | O-(7-Aza-1H-benzotriazol-1-yl)-N,N,N',N'-tetramethyluronium hexafluorophosphate |
| HCl | Hydrochloric acid |
| H₂O | Water |
| HOAc | |
| HOBT | 1-Hydroxybenzotriazole |
| HPLC | High-Performance Liquid Chromatography |
| I₂ | Iodine |
| K₂CO₃ | Potassium carbonate |
| KF | Potassium fluoride |
| KI | Potassium iodide |
| KOH | Potassium hydroxide |
| K₃PO₄ | Potassium phosphate |
| LDA | Lithium diisopropylamide |
| LAH/LiAlH₄ | Lithium aluminum hydride |
| LiBH₄ | Lithium borohydride |
| LiOH | Lithium hydroxide |
| m-CPBA | m-Chloroperoxybenzoic acid |
| MeMgBr | Methylmagnesium bromide |
| MeOH | Methanol |
| MeNH₂ | Methylamine |
| min | Minute |
| MnO₂ | Manganese dioxide |
| Mn(OAc)₃·2H₂O | Manganese(III) acetate dihydrate |
| MsOH | Methanesulfonic acid |
| MW | Microwave |
| NaH | Sodium hydride |
| NaHCO₃ | Sodium bicarbonate |
| NaOH | Sodium hydroxide |
| NBS | N-Bromosuccinimide |
| NH₃ | Ammonia |
| N₂H₄-H₂O | Hydrazine hydrate |
| NH₄OAc | Ammonium acetate |
| NH₄OH | Ammonium hydroxide |
| NMP | N-Methylpyrrolidone |
| o/n | Overnight |
| Pd/C | Palladium/carbon |
| Pd₂(dba)₃ | Tris(dibenzylideneacetone)dipalladium |
| Pd(dppf)Cl₂ | [1,1'-Bis(diphenylphosphino)ferrocene]dichloropalladium |
| Pd(OH)₂ | Palladium hydroxide |
| Pd(PPh₃)₂Cl₂ | Bis(triphenylphosphine)palladium(II) chloride |
| PE | Petroleum ether |
| PhMe | Toluene |
| POCl₃ | Phosphorus oxychloride |
| PPh₃ | Triphenylphosphine |
| RuPhos | 2-Dicyclohexylphosphino-2',6'-diisopropoxy-1,1'-biphenyl |
| SEM | 2-(Trimethylsilyl)ethoxymethyl |
| SEMCl | 2-(Trimethylsilyl)ethoxymethyl chloride |
| SFC | Supercritical fluid chromatography |
| SOCl₂ | Thionyl chloride |
| TEA | Triethylamine |
| TFA | Trifluoroacetic acid |
| THF | Tetrahydrofuran |
| TLC | Thin-layer chromatography |
| TMSCN | Trimethylsilyl cyanide |
| r.t./rt | Room temperature |
| Xantphos | 4,5-Bis(diphenylphosphino)-9,9-dimethylxanthene |
| XeF₂ | Xenon difluoride |
| Xphos | 2-Dicyclohexylphosphino-2',4',6'-triisopropylbiphenyl |
| ZnI₂ | Zinc iodide |

### Example 1: preparation of N-methyl-5-(4-((8-methyl-6-oxo-7-(trifluoromethyl)-5,6-dihydro-1,5-naphthyridin-3-yl)methyl)piperazin-1-yl)picolinamide (compound (1))

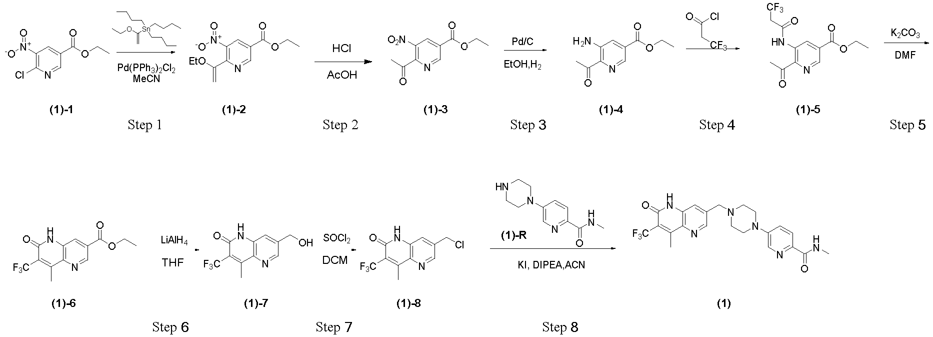

### Step 1. preparation of compound (1)-2

6-Chloro-5-nitropyridine-3-carboxylate **((1)-1)** (3 g), tributyl(1-ethoxyvinyl)tin (5.8 g), and Pd(PPh₃)₂Cl₂ (91.3 mg) were dissolved in acetonitrile (15 mL). The reaction mixture was purged with nitrogen and heated to 65°C for 2 hours. After completion, the reaction was quenched with 10% aqueous KF solution, filtered, and washed with ethyl acetate. The filtrate was concentrated and purified by column chromatography (eluent: petroleum ether-ethyl acetate = 20:1). The eluent was collected, and the solvent was evaporated under reduced pressure to yield a light purple liquid **(1)-2** (3.1 g, yield 89.6%). MS-ESI m/z: 267.2 [M+H]⁺.

### Step 2. preparation of compound (1)-3

Compound **(1)-2** (3.1 g) was dissolved in AcOH (30 mL), and 3M HCl (16 mL) was added. The reaction was carried out at room temperature for 1 hour. After completion, the mixture was concentrated, and the pH was adjusted to 8-9 using aqueous KOH and sodium bicarbonate solutions. The mixture was extracted with ethyl acetate, and the organic phase was washed with saturated brine, dried over anhydrous sodium sulfate, filtered, and the solvent was evaporated under reduced pressure to yield a brown liquid **(1)-3** (3.05 g, yield 100%). MS-ESI m/z: 239.2 [M+H]⁺.

### Step 3. preparation of compound (1)-4

Compound **(1)-3** (750 mg) was dissolved in ethanol (10 mL), and 10% Pd/C (75 mg) was added. The reaction mixture was stirred overnight at room temperature under a hydrogen atmosphere. After completion, the mixture was filtered through diatomaceous earth, washed with ethanol, and concentrated to yield a yellow solid **(1)-4** (580 mg, yield 89%). MS-ESI m/z: 209.2 [M+H]⁺.

### Step 4. preparation of compound (1)-5

Compound **(1)-4** (580 mg) and 3,3,3-trifluoropropionyl chloride (1.1 g) were dissolved in THF (15 mL) and reacted at 0°C for 5 minutes. DIPEA (260 mg) was then added, and the reaction was allowed to proceed at room temperature for 2 hours. After completion, the reaction was quenched with water and extracted with ethyl acetate. The organic phase was washed with saturated brine, dried over anhydrous sodium sulfate, filtered, and concentrated. The product was purified by column chromatography (eluent: petroleum ether-ethyl acetate = 9:1). The eluent was collected, and the solvent was evaporated under reduced pressure to yield a white solid **(1)-5** (950 mg, yield 100%). MS-ESI m/z: 319.2 [M+H]⁺.

### Step 5. preparation of compound (1)-6

Compound **(1)-5** (4.7 g) was dissolved in DMF (305 mL), and potassium carbonate (10.68 g) was added. The reaction mixture was heated to 60°C for 1 hour. After completion, the mixture was diluted with water and extracted with ethyl acetate. The organic phase was washed with saturated brine, dried over anhydrous sodium sulfate, filtered, and concentrated. The product was purified by column chromatography (eluent: dichloromethane-methanol = 20:1). The eluent was collected, and the solvent was evaporated under reduced pressure to yield a light yellow solid **(1)-6** (2.3 g, yield 52%). MS-ESI m/z: 301.2 [M+H]⁺.

### Step 6. preparation of compound (1)-7

Compound (1)-6 (100 mg) was dissolved in THF (3 mL), and LiAlH4 (25.3 mg) was added in portions at 0°C. The reaction was carried out at 0°C for 1 hour. After completion, the reaction was quenched with decahydrate sodium sulfate, filtered through diatomaceous earth, and the filter cake was washed with dichloromethane-methanol (10:1). The filtrate was concentrated and purified by preparative silica gel plate chromatography (eluent: dichloromethane-methanol = 20:1) to yield a light yellow solid **(1)-7** (50 mg, yield 58%). MS-ESI m/z: 259.2 [M+H]⁺.

### Step 7. preparation of compound (1)-8

Compound **(1)-7** (50 mg) and DMF (1.5 mg) were dissolved in DCM (2 mL). Thionyl chloride (138 mg) was added at 0°C, and the reaction was carried out at room temperature for 0.5 hour. After completion, the mixture was concentrated to yield a crude gray solid **(1)-8** (70 mg), which was used directly in the next step. MS-ESI m/z: 277.2 [M+H]⁺.

### Step 8. preparation of compound (1)

The crude product **(1)-8, (1)-R** (48 mg), KI (6 mg), and DIPEA (0.21 mL) were dissolved in acetonitrile (3 mL) and reacted at 75°C for 20 minutes. After completion, the mixture was concentrated and purified by preparative HPLC to yield a white solid **(1)** (2.4 mg, yield 2.8%). MS-ESI m/z: 461.2 [M+H]⁺. ¹H NMR (400 MHz, DMSO-*d₆*) δ 8.58 (s, 1H), 8.39 (d, *J* = 4.8 Hz, 1H), 8.27 (d, *J* = 2.9 Hz, 1H), 7.83 (d, *J* = 8.8 Hz, 1H), 7.69 (s, 1H), 7.42 - 7.37 (m, 1H), 3.72 (s, 2H), 2.86 - 2.70 (m, 5H), 2.62 - 2.54 (m, 4H), 2.53 - 2.50 (m, 6H).

### Example 2: preparation of N-methyl-5-(4-((4-methyl-2-oxo-3-(trifluoromethyl)-1,2-dihydro-1,6-naphthyridin-7-yl)methyl)piperazin-1-yl)picolinamide (compound (2))

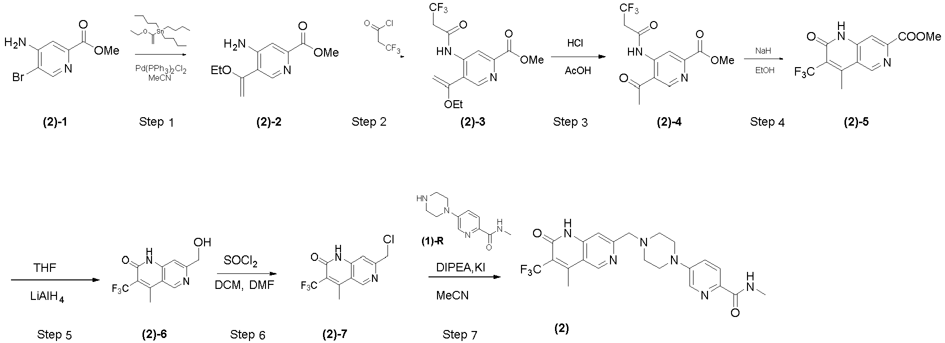

### Steps 1-7. preparation of compound (2)

Using 4-amino-5-bromopyridine-2-carboxylate **((2)-1)** as the starting material, **compound (2)** was prepared according to a method similar to the synthetic route of **compound (1).** MS-ESI m/z: 461.2 [M+H]⁺. ¹H NMR (400 MHz, Chloroform-*d*) δ 11.00 (s, 1H), 9.12 (s, 1H), 8.18 (d, *J* = 2.9 Hz, 1H), 8.08 (d, *J* = 8.7 Hz, 1H), 7.82 (d, *J =* 5.5 Hz, 1H), 7.41 (s, 1H), 7.23 (dd, *J* = 8.8, 2.9 Hz, 1H), 3.84 (s, 2H), 3.40 (t, *J* = 5.0 Hz, 4H), 3.04 (d, *J* = 5.1 Hz, 3H), 2.83 - 2.79 (m, 3H), 2.79 - 2.73 (m, 4H).

### Example 3: preparation of 5-(3-(cyanomethyl)-4-((8-methyl-6-oxo-7-(trifluoromethyl)-5,6-dihydro-1,5-naphthyridin-3-yl)methyl)piperazin-1-yl)-N-methylpicolinamide (compound (3))

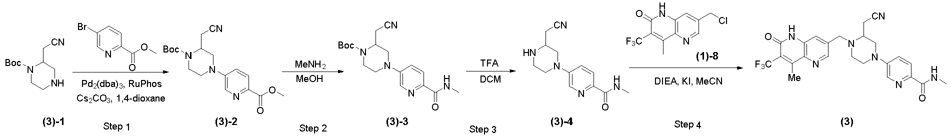

### Step 1. preparation of compound (3)-2

2-(Cyanomethyl)piperazin-1-yl *tert*-butyl carbonate **((3)-1)** (760 mg) was dissolved in 1,4-dioxane (10 mL). Then, 5-bromopyridine-2-carboxylate (728.7 mg), Pd₂(dba)₃ (617 mg), RuPhos (629 mg), and cesium carbonate (3.3 g) were added. The reaction mixture was heated to 100°C and reacted for 3 hours. The reaction mixture was then diluted with water and extracted with ethyl acetate. The organic phase was washed with saturated brine, dried over anhydrous sodium sulfate, filtered, and concentrated. The crude product was purified by silica gel column chromatography (eluent: dichloromethane-methanol = 20:1). The eluent was collected, and the solvent was evaporated under reduced pressure to obtain a yellow oil **(3)-2** (1.1 g, yield 90%). MS-ESI m/z: 361.2 [M+H]⁺.

### Step 2. preparation of compound (3)-3

Compound **(3)-2** (1.1 g) was dissolved in methanol (10 mL), and aqueous methylamine (5 mL, 40% wt) was added. The reaction mixture was sealed and reacted at 90°C for 1.5 hours. After completion, the mixture was concentrated to yield a yellow oil **(3)-3** (920 mg, yield: 83%). MS-ESI m/z: 360.2 [M+H]⁺.

### Step 3. preparation of compound (3)-4

Compound **(3)-3** (580 mg) was dissolved in dichloromethane (4 mL), and trifluoroacetic acid (4 mL) was added at 0°C. The reaction was carried out at room temperature. After completion, the reaction mixture was concentrated, and the residue was diluted with water and dichloromethane. The aqueous phase was adjusted to about pH 10 with ammonia. The mixture was then extracted with dichloromethane, and the organic phase was dried over anhydrous sodium sulfate, filtered, and concentrated to obtain a pale yellow oil **(3)-4** (280 mg, yield 67%). MS-ESI m/z: 260.1 [M+H]⁺.

### Step 4. preparation of compound (3)

Compound **(1)-8** (20 mg) was dissolved in acetonitrile (2 mL). Compound **(3)-4** (28.1 mg), DIPEA (28 mg), and potassium iodide (18 mg) were added. The reaction mixture was reacted at 80°C for 1 hour. After completion, the mixture was diluted with water and extracted with dichloromethane. The organic phase was dried over anhydrous sodium sulfate, filtered, and concentrated. The crude product was purified by silica gel column chromatography (eluent: dichloromethane-methanol = 9:1). The eluent was collected and concentrated to obtain a pale yellow solid **(3)** (2.7 mg, yield 1 %). MS-ESI m/z: 500.2 [M+H]⁺. ¹H NMR (400 MHz, Chloroform-d) δ 12.31 (s, 1H), 8.65 (d, J = 2.0 Hz, 1H), 8.20 (d, J = 2.8 Hz, 1H), 8.09 (d, J = 8.8 Hz, 1H), 7.81 (s, 2H), 7.30 - 7.27 (m, 1H), 4.02 (d, J = 14.8 Hz, 1H), 3.82 (d, J = 14.8 Hz, 1H), 3.57 (dd, J = 12.4, 5.0 Hz, 1H), 3.47 (dd, J = 12.4, 3.3 Hz, 1H), 3.40 - 3.28 (m, 2H), 3.24 (m, 1H), 3.02 (d, J = 5.2 Hz, 3H), 2.99 - 2.94 (m, 1H), 2.90 (q, J = 3.2 Hz, 3H), 2.85 (m, 1H), 2.74 - 2.62 (m, 2H).

### Example 5: preparation of 5-(3-(2-hydroxyethyl)-4-((8-methyl-6-oxo-7-(trifluoromethyl)-5,6-dihydro-1,5-naphthyridin-3-yl)methyl)piperazin-1-yl)-N-methylpicolinamide (compound (5))

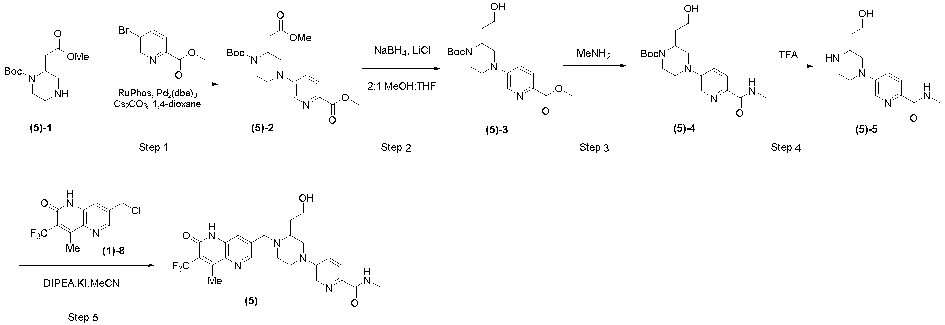

### Step 1. preparation of compound (5)-2

Using methyl 2-(1-BOC-2-piperazinyl) acetate ((5)-1) as the starting material, **compound (5)-2** was prepared according to a method similar to the synthetic route of **compound ((3)-2)**. MS-ESI m/z: 394.2 [M+H]⁺.

### Step 2. preparation of compound (5)-3

Lithium chloride monohydrate (491 mg) and sodium borohydride (615 mg) were dissolved in methanoltetrahydrofuran (4 mL, v/v 2:1). This solution was slowly added to a solution of **(5)-2** (400 mg) in methanoltetrahydrofuran (8 mL, v/v 2:1) in an ice bath. The reaction was carried out for 1 hour in the ice bath. The reaction was quenched with water, and the mixture was extracted with ethyl acetate. The organic phase was washed with saturated brine, dried over anhydrous sodium sulfate, filtered, and concentrated. The crude product was purified by silica gel column chromatography (eluent: petroleum ether-ethyl acetate = 1:1) to obtain a yellow solid **(5)-3** (70 mg, yield 19%). MS-ESI m/z: 366.2 [M+H]⁺.

### Steps 3-5. preparation of compound (5)

Using **(5)-3** as the starting material, **compound (5)** was prepared according to a method similar to the synthetic route of **compound (3).** MS-ESI m/z: 505.2 [M+H]⁺. ¹H NMR (400 MHz, DMSO-*d₆*) δ 12.20 (s, 1H), 8.57 (d, J = 1.9 Hz, 1H), 8.15 (d, J = 2.4 Hz, 1H), 7.93 (d, J = 4.9 Hz, 1H), 7.70 (d, J = 1.9 Hz, 1H), 7.29 (d, J = 2.9 Hz, 2H), 5.20 (t, J = 5.8 Hz, 1H), 4.45 (d, J = 5.8 Hz, 2H), 3.81 (dd, J = 142.3, 14.7 Hz, 4H), 3.30 (s, 3H), 3.15 (d, J = 22.0 Hz, 3H), 2.88 - 2.64 (m, 4H), 2.59 (d, J = 4.5 Hz, 2H).

### Example 6: preparation of 5-(3-(hydroxymethyl)-4-((8-methyl-6-oxo-7-(trifluoromethyl)-5,6-dihydro-1,5-naphthyridin-3-yl)methyl)piperazin-1-yl)-N-methylpicolinamide (compound (6))

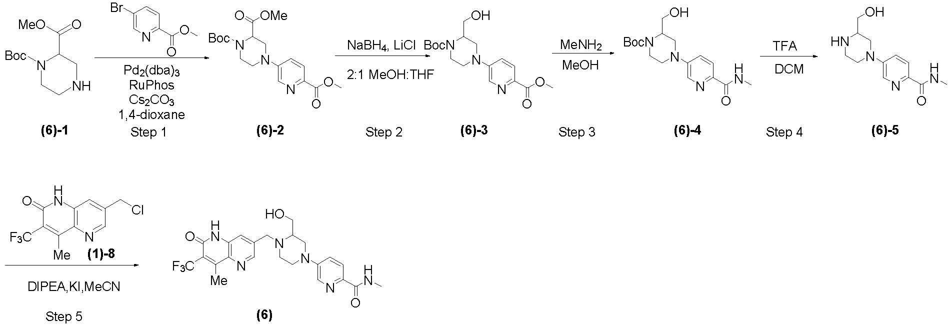

### Steps 1-5. preparation of compound (6)

Using methyl N-1-Boc-2-piperazine-carboxylate **((6)-1)** as the starting material, **compound (6)** was prepared according to a method similar to the synthetic route of **compound (5).** MS-ESI m/z: 491.2 [M+H]⁺. ¹H NMR (400 MHz, DMSO-*d*₆) δ 12.31 (s, 1H), 8.66 (s, 1H), 8.17 (d, *J* = 2.8 Hz, 1H), 8.12 - 8.05 (m, 1H), 7.65 (s, 1H), 7.34 (dd, *J* = 8.7, 2.9 Hz, 1H), 7.31 - 7.25 (m, 1H), 5.22 - 5.17 (m, 1H), 3.90 (d, *J* = 26.0 Hz, 1H), 3.62 - 3.55 (m, 1H), 3.52 - 3.44 (m, 2H), 3.16 - 3.11 (m, 1H), 3.09 - 3.03 (m, 1H), 2.90 - 2.81 (m, 2H), 2.80 - 2.75 (m, 2H), 2.68 - 2.61 (m, 3H), 2.56 - 2.51 (m, 3H), 2.37 -2.30 (m, 1H).

### Example 8: preparation of 5-(3-(methoxymethyl)-4-((8-methyl-6-oxo-7-(trifluoromethyl)-5,6-dihydro-1,5-naphthyridin-3-yl)methyl)piperazin-1-yl)-N-methylpicolinamide (compound (8))

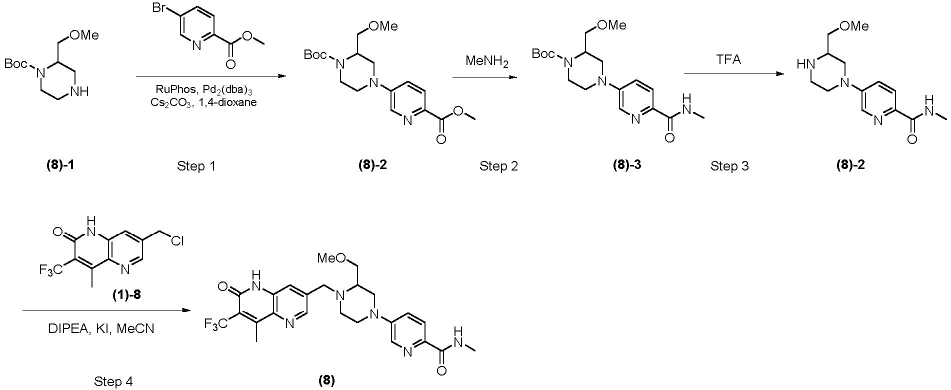

### Steps 1-4. preparation of compound (8)

Using *tert*-butyl 2-(methoxymethyl)piperazine-1-carboxylate **((8)-1**) as the starting material, **compound (8)** was prepared according to a method similar to the synthetic route of **compound (3)**. MS-ESI m/z: 505.2 [M+H]⁺. ¹H NMR (400 MHz, DMSO-*d₆*) δ 12.10 (s, 1H),8.59 (d, J = 1.8 Hz, 1H), 8.39 (t, J = 4.9 Hz, 1H), 8.25 (d, J = 2.8 Hz, 1H), 7.84 (d, J = 8.7 Hz, 1H), 7.73 (d, J = 1.9 Hz, 1H), 7.38 (dd, J = 8.8, 2.9 Hz, 1H), 4.17 (d, J = 14.7 Hz, 1H), 3.69 - 3.58 (m, 2H), 3.50 (dd, J = 9.9, 5.4 Hz, 2H), 3.35 (s, 2H), 3.25 (s, 3H), 3.12 (dt, J = 12.8, 7.0 Hz, 2H), 2.78 (dd, J = 4.2, 2.8 Hz, 5H), 2.51 (s, 3H).

### Example 9: preparation of N-methyl-5-(4-((8-methyl-6-oxo-7-(trifluoromethyl)-5,6-dihydro-1,5-naphthyridin-3-yl)methyl)-3-(trifluoromethyl)piperazin-1-yl)picolinamide (compound (9))

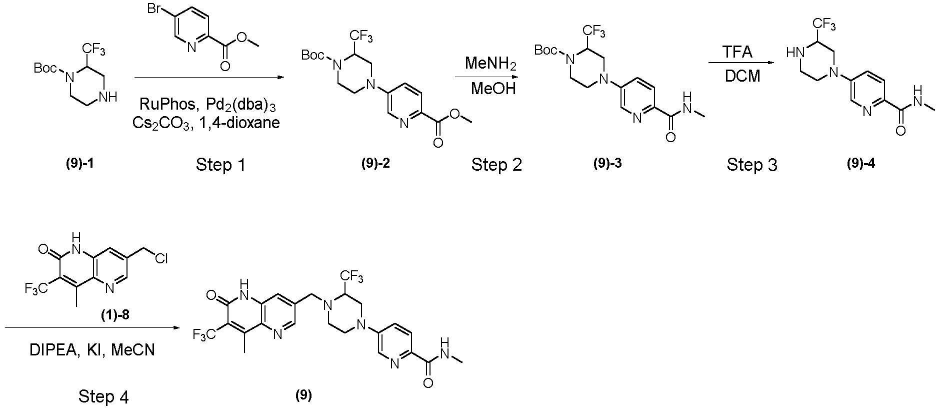

### Steps 1-4. preparation of compound (9)

Using *tert*-butyl 2-trifluoromethylpiperazine-1-carboxylate as the starting material, **compound (9)** was prepared according to a method similar to the synthetic route of **compound (3)**. MS-ESI m/z: 529.1 [M+H]⁺. ¹H NMR (400 MHz, DMSO-*d*₆) δ 12.22 (s, 1H), 8.60 (d, *J* = 2.0 Hz, 1H), 8.39 (q, *J* = 4.8 Hz, 1H), 8.22 (d, *J* = 2.8 Hz, 1H), 7.85 (d, *J* = 8.8 Hz, 1H), 7.74 (d, *J* = 2.0 Hz, 1H), 7.39 - 7.34 (m, 1H), 4.18 (d, *J* = 15.2 Hz, 1H), 4.08 (d, *J =* 15.2 Hz, 1H), 3.98 - 3.92 (m, 1H), 3.90 - 3.83 (m, 1H), 3.63 - 3.52 (m, 2H), 3.26 - 3.12 (m, 2H), 3.03 (d, *J* = 10.0 Hz, 1H), 2.78 (t, *J* = 4.0 Hz, 6H).

### Example 10: preparation of N-methyl-5-(2-methyl-4-((8-methyl-6-oxo-7-(trifluoromethyl)-5,6-dihydro-1,5-naphthyridin-3-yl)methyl)piperazin-1-yl)picolinamide (compound (10))

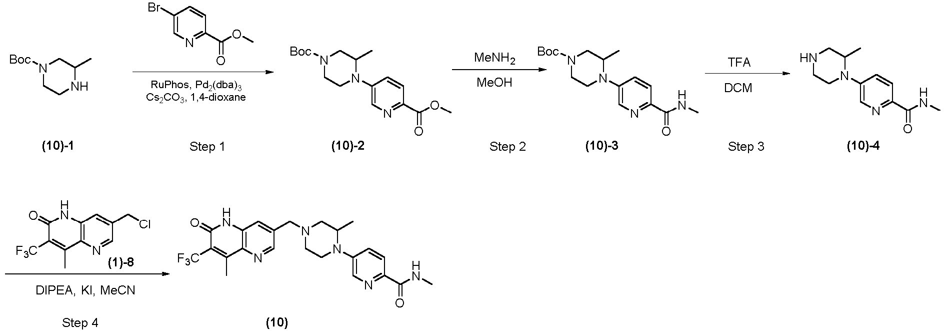

### Steps 1-4. preparation of compound (10)

Using tert-butyl 3-methylpiperazine-1-carboxylate **((10)-1)** as the starting material, **compound (10)** was prepared according to a method similar to the synthetic route of **compound (3).** MS-ESI m/z: 475.2 [M+H]⁺. ¹H NMR (400 MHz, Chloroform-*d*) δ 12.79 (s, 1H), 8.65 (d, *J* = 2.0 Hz, 1H), 8.12 (d, *J* = 2.8 Hz, 1H), 8.05 (d, *J* = 8.8 Hz, 1H), 7.83 (d, *J* = 2.0 Hz, 1H), 7.77 (dd, *J* = 5.2 Hz, 1H), 7.17 (dd, *J* = 8.8, 2.8 Hz, 1H), 4.10 (dd, *J* = 6.8, 3.2 Hz, 1H), 3.80 - 3.59 (m, 2H), 3.50 - 3.40 (m, 1H), 3.27 (td, *J* = 11.6, 3.2 Hz, 1H), 3.01 (d, *J* = 5.2 Hz, 3H), 2.98 - 2.93 (m, 1H), 2.91 (q, *J* = 3.2 Hz, 3H), 2.77 (dt, *J* = 10.8, 2.4 Hz, 1H), 2.55 (dd, *J* = 11.2, 3.6 Hz, 1H), 2.38 (td, *J* = 11.2, 3.6 Hz, 1H), 1.25 (d, *J* = 6.8 Hz, 3H).

### Example 11: preparation of N-methyl-5-(3-methyl-4-((8-methyl-6-oxo-7-(trifluoromethyl)-5,6-dihydro-1,5-naphthyridin-3-yl)methyl)piperazin-1-yl)picolinamide (compound (11))

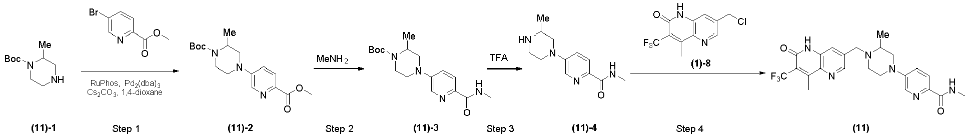

### Steps 1-4. preparation of compound (11)

Using N-1-Boc-2-methylpiperazine **((11)-1**) as the starting material, **compound (11)** was prepared according to a method similar to the synthetic route of **compound (3)**. MS-ESI m/z: 475.2 [M+H]⁺. ¹H NMR (400 MHz, DMSO-*d*₆) δ 12.21 (s, 1H), 8.59 (s, 1H), 8.42 - 8.35 (m, 1H), 8.27 (d, *J* = 2.9 Hz, 1H), 7.82 (d, *J* = 8.8 Hz, 1H), 7.71 (d, *J* = 1.8 Hz, 1H), 7.50-7.30 (m, 1H), 4.15 (d, *J* = 14.6 Hz, 1H), 3.72 (d, *J* = 11.7 Hz, 1H), 3.61 (d, *J* = 12.2 Hz, 1H), 3.45 (d, *J* = 14.6 Hz, 1H), 2.99 (t, *J* = 10.0 Hz, 1H), 2.80 - 2.75 (m, 7H), 2.62 *(d, J=* 6.2 Hz, 1H), 2.36 - 2.28 (m, 2H), 1.18 *(d, J=* 6.1 Hz, 3H).

### Example 12: preparation of N-methyl-5-(8-((8-methyl-6-oxo-7-(trifluoromethyl)-5,6-dihydro-1,5-naphthyridin-3-yl)methyl)-3,8-diazabicyclo[3.2.1]octan-3-yl)picolinamide (compound (12))

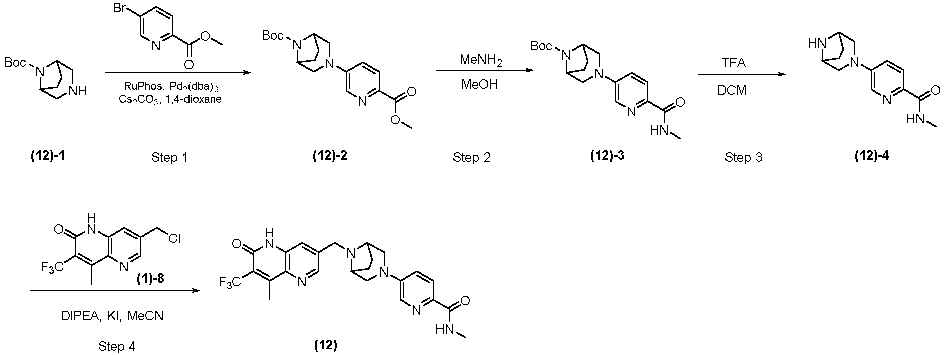

### Steps 1-4. preparation of compound (12)

Using *tert*-butyl 3,8-diazabicyclo[3.2.1]octane-8-carboxylate **((12)-1**) as the starting material, **compound (12)** was prepared according to a method similar to the synthetic route of **compound (3)**. MS-ESI m/z: 487.2 [M+H]⁺. ¹H NMR (400 MHz, Chloroform-*d*) δ 12.37 (s, 1H), 8.65 (s, 1H), 8.10 - 8.00 (m, 2H), 7.90 (s, 1H), 7.81 - 7.74 (m, 1H), 7.10 (dd, *J* = 8.8, 2.8 Hz, 1H), 3.78 (s, 2H), 3.50 - 3.43 (m, 2H), 3.42 - 3.33 (m, 2H), 3.20 (d, *J* = 10.8 Hz, 2H), 3.02 (d, *J* = 5.2 Hz, 3H), 2.93 - 2.84 (m, 3H), 2.18 - 2.03 (m, 2H), 1.92 - 1.81 (m, 2H).

### Example 13: preparation of N-methyl-4-(4-((8-methyl-6-oxo-7-(trifluoromethyl)-5,6-dihydro-1,5-naphthyridin-3-yl)methyl)piperazin-1-yl)benzenesulfonamide (compound (35))

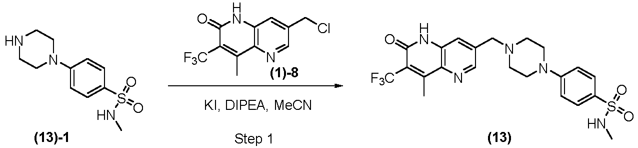

### Step 1. preparation of compound (13)

Compound **(1)-8** (30 mg) and N-methyl-4-(piperazin-1-yl)benzenesulfonamide **((13)-1**) (27 mg) were dissolved in anhydrous acetonitrile (3 mL). KI (18 mg) and DIPEA (42 mg) were then sequentially added, and the mixture was reacted at 90°C for 30 minutes. The reaction was stopped, diluted with water, and extracted with ethyl acetate. The organic layer was dried over anhydrous sodium sulfate, filtered, and concentrated. The mixture was purified on a preparative silica gel plate, to afford a white solid **(13)** (15.5 mg, yield 28%). MS-ESI m/z: 496.5 [M+H]⁺. ¹H NMR (400 MHz, DMSO-*d*₆) δ 12.18 (s, 1H), 8.58 (d, *J* = 1.9 Hz, 1H), 7.69 (d, *J* = 1.9 Hz, 1H), 7.59 - 7.53 (m, 2H), 7.11 (q, *J* = 5.3 Hz, 1H), 7.08 - 7.02 (m, 2H), 3.71 (s, 2H), 3.31-3.25(m, 4H), 2.87-2.67 (m, 3H), 2.55 (t, *J* = 5.1 Hz, 4H), 2.35 (d, *J* = 5.0 Hz, 3H).

### Example 14: preparation of N-methyl-1'-((8-methyl-6-oxo-7-(trifluoromethyl)-5,6-dihydro-1,5-naphthyridin-3-yl)methyl)-1',2',3',6'-tetrahydro-[3,4'-bipyridine]-6-carboxamide (compound (14))

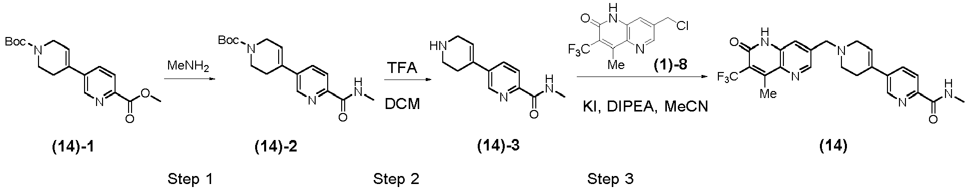

### Steps 1-3. preparation of compound (14)

Using 1'-(*tert*-butyl) 6-methyl 3',6'-dihydro-[3,4'-bipyridine]-1',6(2'H)-dicarboxylate **((14)-1**) as the starting material, **compound (14)** was prepared according to a method similar to steps 2-4 in the synthetic route of compound (3). MS-ESI m/z: 458.2 [M+H]⁺. ¹H NMR (400 MHz, DMSO-*d*₆) δ 12.21 (s, 1H), 8.74 - 8.64 (m, 2H), 8.57 (d, *J* = 1.9 Hz, 1H), 7.98 (s, 2H), 7.70 (s, 1H), 6.68 - 6.31 (m, 1H), 3.78 (s, 2H), 3.21 - 3.17 (m, 2H), 2.81 (d, *J* = 4.9 Hz, 3H), 2.77 (q, *J* = 3.5 Hz, 3H), 2.72 (t, *J* = 5.6 Hz, 2H), 2.59 - 2.53 (s, 2H).

### Example 15: preparation of 4-methyl-7-((4-(3-oxo-2,3-dihydro-1H-pyrrolo[3,4-c]pyridin-6-yl)piperazin-1-yl)methyl)-3-(trifluoromethyl)-1,5-naphthyridin-2(1H)-one (compound (15))

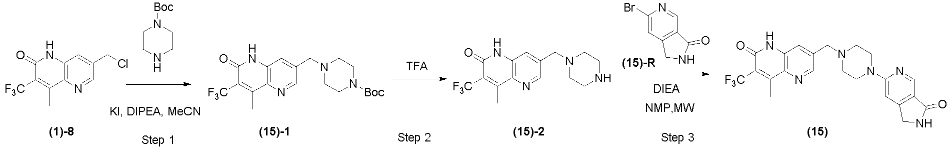

### Step 1. preparation of compound (15)-1

**Compound (1)-8** (450 mg) was dissolved in acetonitrile (6 mL), and 1-*tert*-butoxycarbonylpiperazine (606 mg), DIEA (630 mg), and KI (27 mg) were added. The reaction was carried out under nitrogen protection at 80°C for 1 hour. After the reaction was completed, the mixture was diluted with water and extracted with dichloromethane. The organic phase was washed with saturated brine, dried over anhydrous sodium sulfate, filtered, and concentrated. The product was purified by silica gel column chromatography (eluent: petroleum ether-ethyl acetate = 2:1) to obtain a white solid **(15)-1** (350 mg, yield 50.4%). MS-ESI m/z: 427.2 [M+H]⁺.

### Step 2. preparation of compound (15)-2

Compound **(15)-1** (350 mg) was dissolved in CH₂Cl₂ (1 mL), and trifluoroacetic acid (1 mL) was slowly added dropwise under an ice bath. The mixture was stirred at room temperature for 1 hour and then concentrated. The residue was diluted with water, and the pH was adjusted to about 10 with ammonia. The mixture was extracted with dichloromethane, dried over anhydrous sodium sulfate, filtered, and concentrated to obtain a crude yellow oily product **(15)-2** (230 mg), which was used directly in the next step. MS-ESI m/z: 327.1 [M+H]⁺.

### Step 3. preparation of compound (15)

6-bromo-1,2-dihydro-3H-pyrrolo[3,4-c]pyridin-3-one **((15)-R**) (30 mg) was dissolved in NMP (1 mL), and **(15)-2** (92.58 mg) and DIEA (54.95 mg) were added. The mixture was subjected to microwave irradiation at 150°C for 1 hour, then diluted with water and extracted with ethyl acetate. The organic phase was washed with saturated brine, dried over anhydrous sodium sulfate, filtered, and concentrated. The product was purified by preparative HPLC to obtain a white solid **(15)** (1.0 mg, yield 1.5%). MS-ESI m/z: 459.2 [M+H]⁺. ¹H NMR (400 MHz, DMSO-*d₆*) δ 12.21(s, 1H), 8.58 (d, J = 1.8 Hz, 1H), 8.39 (s, 1H), 8.17 (s, 1H), 7.70 (d, J = 1.9 Hz, 1H), 6.94 (s, 1H), 4.29 (s, 2H), 3.74 - 3.57 (m, 2H), 3.17 (d, J = 5.2 Hz, 6H), 2.76 (d, J = 6.9, 3.3 Hz, 2H), 2.51 (s, 3H).

### Example 17: preparation of 2-(4-((8-methyl-6-oxo-7-(trifluoromethyl)-5,6-dihydro-1,5-naphthyridin-3-yl)methyl)piperazin-1-yl)-5,6-dihydro-4H-pyrrolo[3,4-d]thiazol-4-one (compound (17))

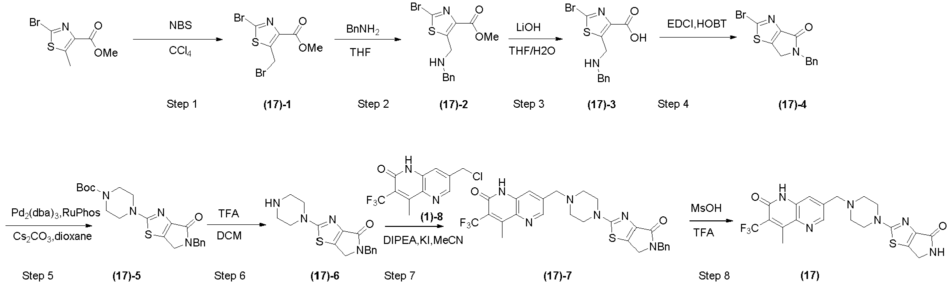

### Step 1. preparation of compound (17)-1

Methyl 2-bromo-5-methylthiazole-4-carboxylate (900 mg) was dissolved in carbon tetrachloride (10 mL), and N-bromosuccinimide (746 mg) and dibenzoyl peroxide (92.3 mg) were added. The reaction was carried out overnight at 80°C. After the reaction was completed, the mixture was concentrated, and the residue was purified by silica gel column chromatography (eluent: PE-EA = 10:1). The eluate was collected and the solvent was evaporated under reduced pressure to obtain a light yellow solid **(17)-1** (900 mg, yield: 74.9%). MS-ESI m/z: 315.9 [M+H]⁺.

### Step 2. preparation of compound (17)-2

Compound **(17)-1** (900 mg) was dissolved in anhydrous tetrahydrofuran (10 mL), and benzylamine (520 mg) and potassium carbonate (671 mg) were added. The reaction was carried out at 80°C for 4 hours. The mixture was then diluted with water and the pH was adjusted to 10 with a saturated sodium bicarbonate solution. The mixture was extracted with dichloromethane, and the organic phase was dried over anhydrous sodium sulfate, filtered, and concentrated. The residue was purified by silica gel column chromatography (eluent: PE-EA = 1:1). The eluate was collected and the solvent was evaporated under reduced pressure to obtain a light yellow solid **(17)-2** (720 mg, yield: 74%). MS-ESI m/z: 340.9 [M+H]⁺.

### Step 3. preparation of compound (17)-3

Compound **(17)-2** (720 mg) was dissolved in tetrahydrofuran (7 mL) and water (3.5 mL), and lithium hydroxide monohydrate (273 mg) was added. The reaction was carried out at room temperature for 4 hours. The mixture was then acidified with dilute hydrochloric acid and concentrated to obtain a crude colorless oily product **(17)-3.** MS-ESI m/z: 326.9 [M+H]⁺.

### Step 4. preparation of compound (17)-4

Compound **(17)-3** was dissolved in N,N-dimethylformamide (8 mL), and EDCI (707 mg), HOBT (293 mg), DIPEA (658 mg), and DMAP (53 mg) were added. The reaction was carried out at 65°C for 4 hours. After the reaction was completed, the mixture was diluted with water and extracted with dichloromethane. The organic phase was dried over anhydrous sodium sulfate, filtered, and concentrated. The residue was purified by silica gel column chromatography (eluent: DCM-MeOH = 25:1). The eluate was collected and the solvent was evaporated under reduced pressure to obtain a light yellow solid **(17)-4** (200 mg, yield: 30%). MS-ESI m/z: 308.9 [M+H]⁺.

### Steps 5-7. preparation of compound (17)-7

Using **(17)-4** as the starting material, **compound (17)-7** was prepared according to a method similar to the synthetic route of **compound (3).** MS-ESI m/z: 555.1 [M+H]⁺.

### Step 8. preparation of compound (17)

Compound **(17)-7** (20 mg) was dissolved in methanesulfonic acid (1 mL), trifluoroacetic acid (1 mL) was added, and the mixture was reacted at 80°C for 16 hours. After the reaction was completed, the mixture was concentrated. The residue was diluted with water and dichloromethane. The pH of the aqueous phase was adjusted to about 10 with ammonia. The mixture was extracted with dichloromethane, and the organic phase was dried over anhydrous sodium sulfate, filtered, and concentrated. The residue was purified by silica gel column chromatography (eluent: DCM-MeOH = 10:1). The eluate was collected and concentrated to obtain a light yellow solid **(17)** (1.8 mg, yield 11%). MS-ESI m/z: 465.1 [M+H]⁺. ¹H NMR (400 MHz, DMSO-*d*₆) δ 12.18 (s, 1H), 8.58 (d, *J* = 2.0 Hz, 1H), 8.25 (s, 1H), 7.69 - 7.66 (m, 1H), 4.30 (s, 2H), 3.72 (s, 2H), 3.53 - 3.42 (m, 4H), 2.86 - 2.73 (m, 2H), 2.55 (d, *J* = 5.2 Hz, 4H).

### Example 18: preparation of 4-methyl-7-((4-(2-oxo-2,3-dihydro-1H-imidazo[4,5-b]pyridin-5-yl)piperazin-1-yl)methyl)-3-(trifluoromethyl)-1,5-naphthyridin-2(1H)-one (compound (18))

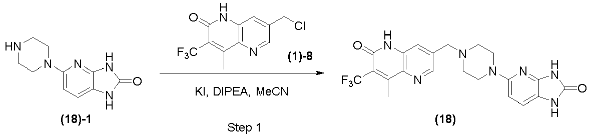

### Step 1. preparation of compound (18)

Using 5-(piperazin-1-yl)-1,3-dihydro-2H-imidazo[4,5-b]pyridin-2(3H)-one **((18)-1)** as the starting material, **compound (18)** was prepared according to a method similar to the synthetic route of compound (3). MS-ESI m/z: 460.2 [M+H]⁺. ¹H NMR (400 MHz, DMSO-*d*₆) δ 12.22 (s, 1H), 10.92 (s, 1H), 10.35 (s, 1H), 8.57 (s, 1H), 7.69 (s, 1H), 7.09 (d, *J* = 8.4 Hz, 1H), 6.35 (d, *J* = 8.4 Hz, 1H), 3.69 (s, 2H), 3.52 - 3.38 (m, 4H), 2.84 - 2.70 (m, 3H), 2.55 - 2.52 (m, 4H).

### Example 19: preparation of 4-methyl-3-(trifluoromethyl)-7-((4-(5-(trifluoromethyl)pyrimidin-2-yl)piperazin-1-yl)methyl)-1,5-naphthyridin-2(1H)-one (compound (19))

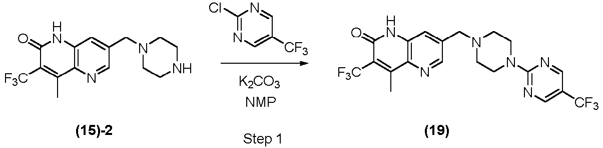

### Step 1. preparation of compound (19)

2-Chloro-5-trifluoromethylpyrimidine (10 mg) was dissolved in NMP (1 mL), and **(15)-2** (19.67 mg) and potassium carbonate (15.14 mg) were added. The mixture was stirred at 80°C for 2 hours. After the reaction was completed, the mixture was diluted with water and extracted with dichloromethane. The organic phase was washed with saturated brine, dried over anhydrous sodium sulfate, filtered, and concentrated. The product was purified by preparative HPLC to obtain a white solid **(19)** (6.3 mg, yield 24.4%). MS-ESI m/z: 473.1 [M+H]⁺. ¹H NMR (400 MHz, DMSO-*d₆*) δ 12.23 (s, 1H), 8.70 (d, J = 1.0 Hz, 2H), 8.58 (d, J = 1.9 Hz, 1H), 7.69 (d, J = 1.9 Hz, 1H), 3.86 (t, J = 5.2 Hz, 4H), 3.70 (s, 2H), 3.40-3.20 (m, 4H), 2.77 (q, J = 3.5 Hz, 3H).

### Example 20: preparation of 6-(4-((8-methyl-6-oxo-7-(trifluoromethyl)-5,6-dihydro-1,5-naphthyridin-3-yl)methyl)piperazin-1-yl)nicotinonitrile (compound (20))

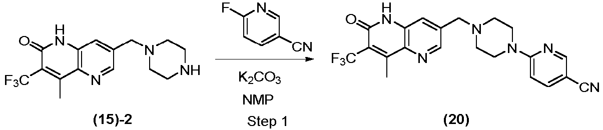

### Step 1. preparation of compound (20)

Using 5-cyano-2-fluoropyridine as the starting material, **compound (20)** was prepared according to a method similar to the synthetic route of **compound (19).** MS-ESI m/z: 429.2 [M+H]⁺. ¹H NMR (400 MHz, DMSO-*d₆*) δ 12.22 (s, 1H), 8.57 (d, J = 1.8 Hz, 1H), 8.48 (d, J = 2.3 Hz, 1H), 7.85 (dd, J = 9.1, 2.4 Hz, 1H), 7.68 (d, J = 1.9 Hz, 1H), 6.93 (d, J = 9.1 Hz, 1H), 3.79 - 3.55 (m, 6H), 2.77 (q, J = 3.5 Hz, 4H), 2.48 (s, 3H).

### Example 21: preparation of 5-(3-cyano-4-((8-methyl-6-oxo-7-(trifluoromethyl)-5,6-dihydro-1,5-naphthyridin-3-yl)methyl)piperazin-1-yl)-N-methylpicolinamide (compound (21))

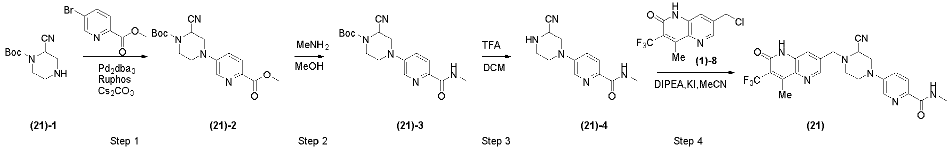

### Step 1. preparation of compound (21)-2

*Tert*-butyl 2-cyanopiperazine-1-carboxylate **((21)-1)** (260 mg) was dissolved in 1,4-dioxane (5 mL), and methyl 5-bromopicolinate (169 mg), tris(dibenzylideneacetone)dipalladium (228 mg), 2-dicyclohexylphosphino-2',6'-diisopropoxy-1,1'-biphenyl (234 mg), and cesium carbonate (1.22 g) were added. The reaction was carried out at 100°C for 3 hours. The reaction mixture was diluted with water and extracted with ethyl acetate. The organic phase was washed with saturated brine, dried over anhydrous sodium sulfate, filtered, and concentrated. The residue was purified by silica gel column chromatography (eluent: PE-EA = 1:1). The eluate was collected and the solvent was evaporated under reduced pressure to obtain a yellow oily product **(21)-2** (300 mg, yield: 69%). MS-ESI m/z: 347.2 [M+H]⁺.

### Step 2. preparation of compound (21)-3

Compound **(21)-2** (300 mg) was dissolved in methanol (4 mL), and an aqueous solution of methylamine (2 mL, 40% wt) was added. The reaction was carried out in a sealed tube at 50°C for 1 hour. After the reaction was completed, the reaction solution was concentrated and purified by silica gel column chromatography (eluent: DCM-MeOH = 20:1). The eluate was collected and the solvent was evaporated under reduced pressure to obtain a yellow solid **(21)-3** (150 mg, yield: 50%). MS-ESI m/z: 346.2 [M+H]⁺.

### Step 3. preparation of compound (21)-4

Compound **(21)-3** (100 mg) was dissolved in dichloromethane (4 mL), and trifluoroacetic acid (1 mL) was added at 0°C. The reaction was carried out at room temperature for 1 hour. After the reaction was completed, the reaction solution was diluted with water and dichloromethane. The pH of the aqueous phase was adjusted to about 10 with ammonia. The mixture was extracted with dichloromethane, and the organic phase was dried over anhydrous sodium sulfate, filtered, and concentrated to obtain a crude light yellow oily product **(21)-4,** which was used directly in the next step. MS-ESI m/z: 246.2 [M+H]⁺.

### Step 4. preparation of compound (21)

Compound **(1)-8** (20 mg) was dissolved in acetonitrile (2 mL), and the crude product **(21)-4,** DIPEA (28 mg), and potassium iodide (2.4 mg) were added. The reaction was carried out at 80°C for 1 hour. After the reaction was completed, the reaction solution was diluted with water and extracted with dichloromethane. The organic phase was dried over anhydrous sodium sulfate, filtered, and concentrated. The residue was purified by silica gel column chromatography (eluent: dichloromethane-methanol = 9:1). The eluate was collected and concentrated to obtain a white solid **(21)** (1.0 mg, yield: 3 %). MS-ESI m/z: 486.2 [M+H]⁺. ¹H NMR (400 MHz, DMSO-*d₆*) δ 12.27 (s, 1H), 8.61 (s, 1H), 8.45 (d, J = 5.2 Hz, 1H), 8.33 (d, J = 2.8 Hz, 1H), 7.86 (d, J = 8.8 Hz, 1H), 7.71 (s, 1H), 7.48 (dd, J = 8.8, 2.8 Hz, 1H), 4.32 (s, 1H), 4.16 (d, J = 12.8 Hz, 2H), 3.90 (d, J = 14.0 Hz, 1H), 3.82 (d, J = 14.0 Hz, 1H), 3.11 (dd, J = 12.8, 3.2 Hz, 2H), 2.89 - 2.83 (m, 2H), 2.78 (q, J = 4.0 Hz, 6H).

### Example 23: preparation of 5-(3-methyl-4-((8-methyl-6-oxo-7-(trifluoromethyl)-5,6-dihydro-1,5-naphthyridin-3-yl)methyl)piperazin-1-yl)picolinamide (compound (23))

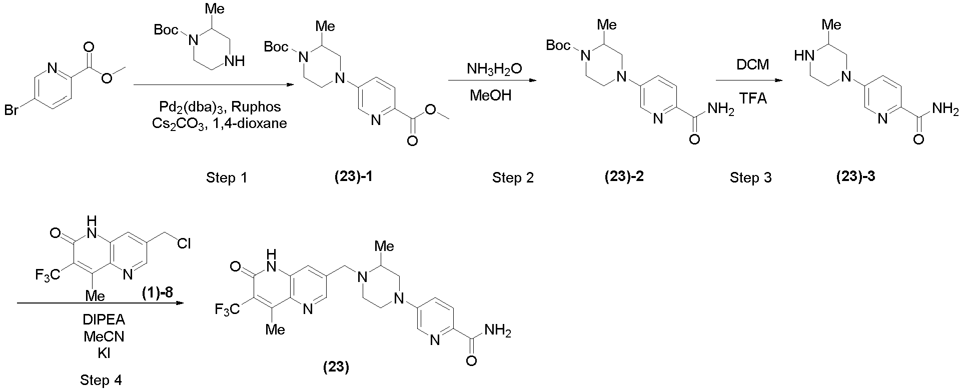

### Steps 1-4. preparation of compound (23)

Using methyl 5-bromopicolinate as the starting material, **compound (23)** was prepared according to a method similar to the synthetic route of **compound (3).** MS-ESI m/z: 461.2 [M+H]⁺. ¹H NMR (400 MHz, DMSO-*d*₆) δ 12.15 (s, 1H), 8.57 (s, 1H), 8.29 - 8.24 (m, 1H), 7.83 (d, *J* = 8.7 Hz, 1H), 7.77 - 7.61 (m, 2H), 7.41 - 7.33 (m, 1H), 7.29 (s, 1H), 3.67 - 3.53 (m, 2H), 3.21 - 3.10 (m, 5H), 2.84 - 2.78 (m, 1H), 2.78 - 2.72 (m, 3H), 2.67 - 2.60 (m, 1H), 1.24 - 1.21 (m, 3H).

### Example 24: preparation of N-methyl-5-(4-(1-(8-methyl-6-oxo-7-(trifluoromethyl)-5,6-dihydro-1,5-naphthyridin-3-yl)ethyl)piperazin-1-yl)picolinamide (compound (24))

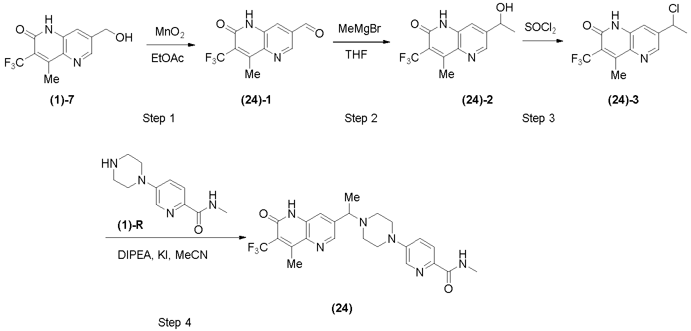

### Step 1. preparation of compound (24)-1

Compound **(1)-7** (1 g) was dissolved in ethyl acetate (20 mL), followed by the addition of activated manganese dioxide (5.05 g). The reaction was carried out at 50°C for 3 hours. After the reaction was completed, the solid was filtered off, and the reaction solution was concentrated. The residue was purified by silica gel column chromatography (eluent: dichloromethane-methanol = 20:1). The eluate was collected and the solvent was evaporated under reduced pressure to obtain a light yellow solid **(24)-1** (550 mg, yield 55%). MS-ESI m/z: 257.1 [M+H]⁺.

### Step 2. preparation of compound (24)-2

Compound **(24)-1** (550 mg) was dissolved in anhydrous tetrahydrofuran (11 mL), and a solution of methylmagnesium bromide (5.4 mL, 1 M in THF) was added dropwise at 0°C. The reaction was carried out at 25°C for 1 hour. After the reaction was completed, the reaction was quenched with a saturated aqueous solution of ammonium chloride, and the pH was adjusted to approximately 7 with a saturated aqueous solution of sodium bicarbonate. The mixture was extracted with ethyl acetate, and the organic phase was washed with saturated brine, dried over anhydrous sodium sulfate, filtered, and concentrated. The residue was purified by silica gel column chromatography (eluent: dichloromethane-methanol = 20:1). The eluate was collected and the solvent was evaporated under reduced pressure to obtain a yellow solid **(24)-2** (558 mg, yield 95%). MS-ESI m/z: 273.1 [M+H]⁺.

### Step 3. preparation of compound (24)-3

Compound **(24)-2** (558 mg) was dissolved in thionyl chloride (10 mL) and the reaction was carried out at 25°C for 1 hour. After the reaction was completed, the reaction mixture was concentrated to dryness to obtain a yellow solid **(24)-3** (590 mg). MS-ESI m/z: 291.0 [M+H]⁺.

### Step 4: preparation of compound (24)

Compound **(24)-3** (568.4 mg) was dissolved in acetonitrile (20 mL), and **(1)-R** (500.0 mg), DIPEA (1.14 mL), and potassium iodide (57.1 mg) were added. The reaction was carried out at 80°C for 16 hours. After the reaction was completed, the mixture was diluted with water and extracted with dichloromethane. The organic phase was dried over anhydrous sodium sulfate, filtered, and concentrated. The residue was purified by silica gel column chromatography (eluent: dichloromethane-methanol = 20:1). The eluate was collected and the solvent was evaporated under reduced pressure to obtain a light yellow solid **(24)** (340.0 mg, yield 42 %). MS-ESI m/z: 475.2 [M+H]⁺. ¹H NMR (400 MHz, DMSO-*d₆*) δ 12.21 (s, 1H), 8.63 (d, J = 2.0 Hz, 1H), 8.44 - 8.35 (m, 1H), 8.25 (d, J = 2.8 Hz, 1H), 7.82 (d, J = 8.8 Hz, 1H), 7.68 (d, J = 2.0 Hz, 1H), 7.37 (dd, J = 8.8, 2.8 Hz, 1H), 3.80 - 3.67 (m, 1H), 3.34 - 3.27 (m, 4H), 2.82 - 2.68 (m, 5H), 2.69 - 2.56 (m, 2H), 2.48 - 2.43 (m, 3H), 1.38 (d, J = 6.8 Hz, 3H).

### Example 90 and 91: preparation of (R)-N-methyl-5-(4-(1-(8-methyl-6-oxo-7-(trifluoromethyl)-5,6-dihydro-1,5-naphthyridin-3-yl)ethyl)piperazin-1-yl)picolinamide (compound (90)) and (S)-N-methyl-5-(4-(1-(8-methyl-6-oxo-7-(trifluoromethyl)-5,6-dihydro-1,5-naphthyridin-3-yl)ethyl)piperazin-1-yl)picolinamide (compound (91))

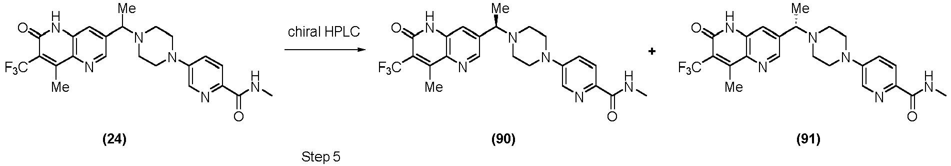

Compound **(24)** (340 mg) was separated by chiral HPLC to obtain two stereoisomeric compounds, compound **(90)** and compound **(91).**

### Example 25: preparation of 5-(4-(cyano(8-methyl-6-oxo-7-(trifluoromethyl)-5,6-dihydro-1,5-naphthyridin-3-yl)methyl)piperazin-1-yl)-N-methylpicolinamide (compound (25))

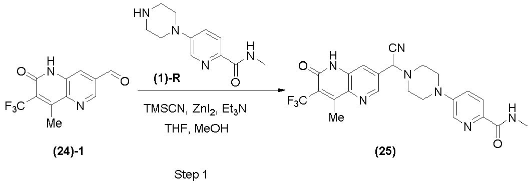

### Step 1. preparation of compound (25)

Compound **(24)-1** (25 mg) was dissolved in tetrahydrofuran (1 mL), and trimethylsilyl cyanide (11.6 mg) and zinc iodide (1.6 mg) were added. The mixture was purged with nitrogen three times and then stirred at 0°C for 5 minutes. A methanol solution (2 mL) of **(1)-R** (23.6 mg) and triethylamine (9.9 mg) was added dropwise, and the reaction was carried out at 50°C for 16 hours. After the reaction was completed, the reaction solution was quenched with a saturated aqueous solution of potassium carbonate and extracted with ethyl acetate. The organic phase was dried over anhydrous sodium sulfate, filtered, and concentrated. The residue was purified by silica gel column chromatography (eluent: dichloromethane-methanol = 15:1). The eluate was collected and concentrated to obtain a white solid **(25)** (1.2 mg, yield 2.5 %). MS-ESI m/z: 486.2 [M+H]⁺. ¹H NMR (400 MHz, DMSO-*d₆*) δ 12.26 (s, 1H), 8.68 (d, J = 2.0 Hz, 1H), 8.43 (q, J = 4.8 Hz, 1H), 8.29 (d, J = 3.2 Hz, 1H), 7.88 (d, J = 2.0 Hz, 1H), 7.84 (d, J = 8.8 Hz, 1H), 7.42 (dd, J = 8.8, 3.2 Hz, 1H), 5.82 (s, 1H), 3.58 - 3.47 (m, 4H), 2.81 - 2.70 (m, 5H), 2.61 - 2.54 (m, 2H), 2.52 - 2.51 (m, 3H).

### Example 26: preparation of N-methyl-6-(3-methyl-4-((8-methyl-6-oxo-7-(trifluoromethyl)-5,6-dihydro-1,5-naphthyridin-3-yl)methyl)piperazin-1-yl)pyridazine-3-carboxamide (compound (26))

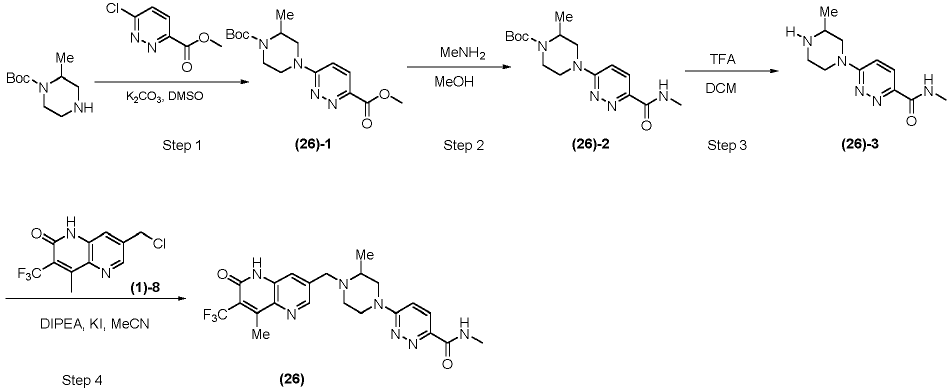

### Step 1 preparation of compound (26)-1

Methyl 6-chloropyridazine-3-carboxylate (200 mg) and tert-butyl 2-methylpiperazine-1-carboxylate (243.7 mg) were dissolved in dimethyl sulfoxide (4 mL), followed by the addition of potassium carbonate (480.5 mg). The reaction was carried out at 100°C for 1 hour. After the reaction was completed, the mixture was diluted with water and extracted with ethyl acetate. The organic phase was washed with saturated brine, dried over anhydrous sodium sulfate, filtered, and concentrated. The residue was purified by silica gel column chromatography (eluent: petroleum ether-ethyl acetate = 5:1). The eluate was collected and the solvent was evaporated under reduced pressure to obtain a white solid **(26)-1** (200.6 mg, yield 51%). MS-ESI m/z: 337.2 [M+H]⁺.

### Steps 2-4. preparation of compound (26)

Using **(26)-1** as the starting material, **compound (26)** was prepared according to a method similar to steps 2-4 in the synthetic route of **compound (3).** MS-ESI m/z: 476.2 [M+H]⁺. ¹H NMR (400 MHz, Chloroform-d) δ 12.53 (s, 1H), 8.65 (d, J = 2.0 Hz, 1H), 8.01 (d, J = 9.6 Hz, 1H), 7.92 - 7.85 (m, 1H), 7.80 (d, J = 2.0 Hz, 1H), 6.95 (d, J = 9.6 Hz, 1H), 4.23 - 4.12 (m, 2H), 4.02 (d, J = 12.8 Hz, 1H), 3.48 (d, J = 14.8 Hz, 1H), 3.45 - 3.37 (m, 1H), 3.22 (dd, J = 13.2, 8.8 Hz, 1H), 3.03 (d, J = 5.2 Hz, 3H), 2.92 - 2.85 (m, 3H), 2.82 (dt, J = 11.6, 3.6 Hz, 1H), 2.77 - 2.67 (m, 1H), 2.39 (ddd, J = 11.6, 9.6, 3.2 Hz, 1H), 1.24 (d, J = 6.0 Hz, 3H).

### Example 28: preparation of N-methyl-5-(3-methyl-4-((8-methyl-6-oxo-7-(trifluoromethyl)-5,6-dihydro-1,5-naphthyridin-3-yl)methyl)piperazin-1-yl)pyrimidine-2-carboxamide (compound (28))

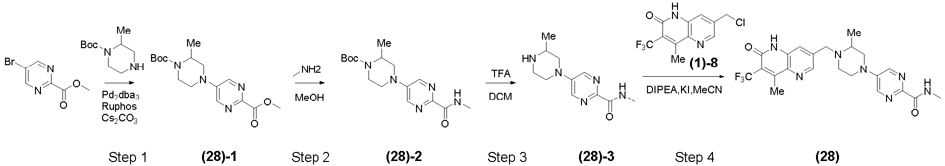

### Steps 1-4. preparation of compound (28)

Using methyl 5-bromopyrimidine-2-carboxylate and *tert*-butyl 2-methylpiperazine-1-carboxylate as the starting material, **compound (28)** was prepared according to a method similar to the synthetic route of **compound (3).** MS-ESI m/z: 476.2 [M+H]⁺. ¹H NMR (400 MHz, DMSO-*d*₆) δ 12.16 (s, 1H), 8.55 *(d, J* = 2.0 Hz, 1H), 8.52 - 8.47 (m, 3H), 7.67 (d, *J* = 2.0 Hz, 1H), 4.10 (d, *J* = 14.8 Hz, 1H), 3.75 (d, *J* = 12.0 Hz, 1H), 3.64 (d, *J* = 8.8 Hz, 1H), 3.42 (d, *J* = 14.8 Hz, 1H), 3.06 - 2.95 (m, 1H), 2.81 (dd, *J* = 12.4, 8.8 Hz, 1H), 2.81 - 2.68 (m,6H), 2.66 - 2.54 (m, 1H), 2.34 - 2.23 (m, 1H), 1.13 (d, *J* = 6.0 Hz, 3H).

### Example 29: preparation of 5-(4-((7,8-dimethyl-6-oxo-5,6-dihydro-1,5-naphthyridin-3-yl)methyl)piperazin-1-yl)-N-methylpicolinamide (compound (29))

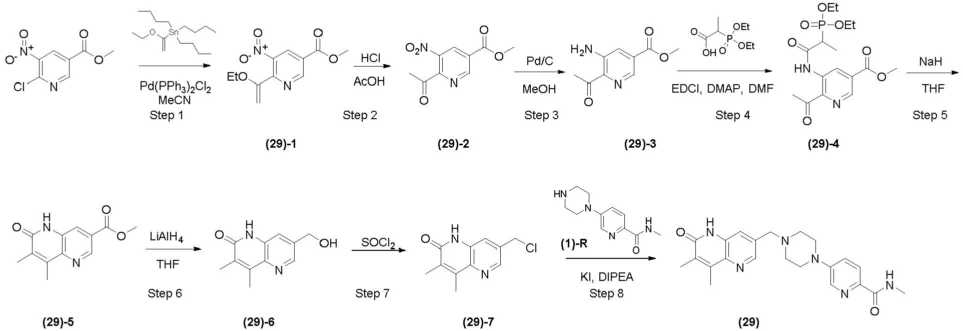

### Steps 1-3. preparation of compound (29)-3

Using methyl 6-chloro-5-nitropyridine-3-carboxylate as the starting material, **compound (29)-3** was prepared according to a method similar to steps 1-3 in the synthetic route of **compound (1).** MS-ESI m/z: 195.1 [M+H]⁺.

### Step 4. preparation of compound (29)-4

Compound **(29)-3** (400 mg) and 2-(diethylphosphoryl)propanoic acid (432.9 mg) were dissolved in DMF (10 mL), followed by the addition of EDCI (790 mg), DMAP (503.3 mg), and triethylamine (625.3 mg). The reaction was carried out at 60°C for 16 hours. After the reaction was completed, the mixture was diluted with water and extracted with ethyl acetate. The organic phase was washed with saturated brine, dried over anhydrous sodium sulfate, filtered, and concentrated. The residue was purified by silica gel column chromatography (eluent: petroleum ether-ethyl acetate = 10:1). The eluate was collected and the solvent was evaporated under reduced pressure to obtain a light yellow solid **(29)-4** (323.3 mg, yield 41%). MS-ESI m/z: 387.1 [M+H]⁺.

### Step 5. preparation of compound (29)-5

Compound **(29)-4** (323.3 mg) was dissolved in tetrahydrofuran (160 mL), and sodium hydride (341.6 mg, 60% wt) was added. The reaction was carried out at room temperature for 8 hours. After the reaction was completed, the mixture was quenched with a saturated aqueous solution of ammonium chloride and extracted with ethyl acetate. The organic phase was washed with saturated brine, dried over anhydrous sodium sulfate, filtered, and concentrated. The residue was purified by silica gel column chromatography (eluent: dichloromethane-methanol = 20:1). The eluate was collected and the solvent was evaporated under reduced pressure to obtain a white solid (29)-5 (76 mg, yield 39%). MS-ESI m/z: 233.1 [M+H]⁺.

### Steps 6-8. preparation of compound (29)

Using **(29)-5** as the starting material, **compound (29)** was prepared according to a method similar to steps 6-8 in the synthetic route of **compound (1).** MS-ESI m/z: 407.2 [M+H]⁺. ¹H NMR (400 MHz, DMSO-*d₆*) δ 11.74 (s, 1H), 8.47 - 8.35 (m, 2H), 8.26 (d, J = 2.8 Hz, 1H), 7.82 (d, J = 8.8 Hz, 1H), 7.60 (d, J = 2.0 Hz, 1H), 7.38 (dd, J = 8.8, 2.8 Hz, 1H), 3.64 (s, 2H), 3.33 (t, J = 5.2 Hz, 4H), 2.77 (d, J = 4.8 Hz, 3H), 2.57 - 2.53 (m, 4H), 2.49 (d, J = 1.2 Hz, 3H), 2.13 (d, J = 1.2 Hz, 3H).

### Example 51: preparation of (R)-N-methyl-5-(3-methyl-4-((8-methyl-6-oxo-7-(trifluoromethyl)-5,6-dihydro-1,5-naphthyridin-3-yl)methyl)piperazin-1-yl)picolinamide (compound (51))

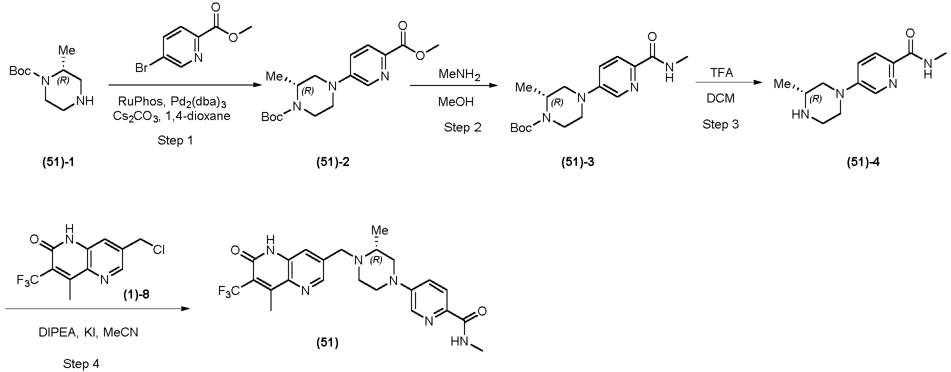

### Steps 1-4. preparation of compound (51)

Using (R)-1-N-Boc-2-methylpiperazine **((51)-1)** as the starting material, **compound (51)** was prepared according to a method similar to the synthetic route of **compound (3).** MS-ESI m/z: 475.2 [M+H]⁺. ¹H NMR (400 MHz, DMSO-*d*₆) δ 12.19 (s, 1H), 8.56 (s, 1H), 8.47-8.27 (m, 1H), 8.25 (d, *J* = 2.9 Hz, 1H), 7.81 (d, *J* = 8.7 Hz, 1H), 7.69 (s, 1H), 7.43 - 7.34 (m, 1H), 4.12 (d, *J* = 14.6 Hz, 1H), 3.70 (d, *J* = 12.0 Hz, 1H), 3.59 (d, *J* = 12.2 Hz, 2H), 3.06 - 2.88 (m, 2H), 2.84-2.64 (m, 7H), 2.61 (s, 1H), 2.40-2.20 (m, 1H), 1.16 (d, *J* = 6.1 Hz, 3H).

### Example 52: preparation of (S)-N-methyl-5-(3-methyl-4-((8-methyl-6-oxo-7-(trifluoromethyl)-5,6-dihydro-1,5-naphthyridin-3-yl)methyl)piperazin-1-yl)picolinamide (compound (52))

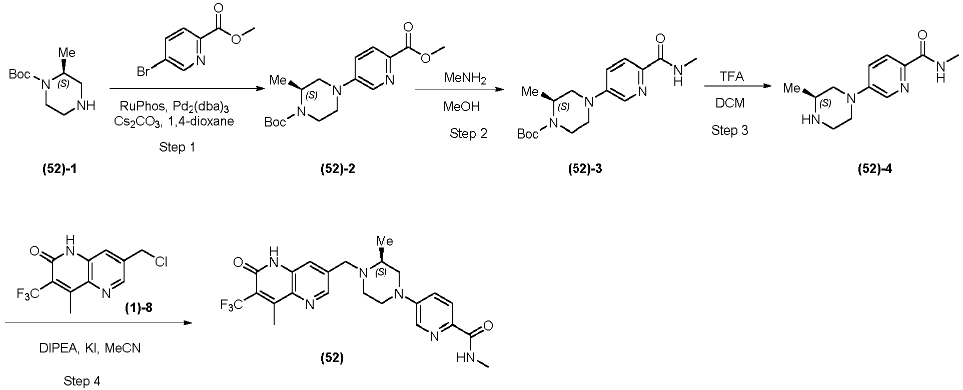

### Steps 1-4. preparation of compound (52)

Using (S)-1-N-Boc-2-methylpiperazine **((52)-1)** as the starting material, **compound (52)** was prepared according to a method similar to the synthetic route of compound (3). MS-ESI m/z: 475.2 [M+H]⁺. ¹H NMR (400 MHz, DMSO-*d*₆) δ 12.19 (s, 1H), 8.56 (s, 1H), 8.47-8.27 (m, 1H), 8.25 (d, *J* = 2.9 Hz, 1H), 7.81 (d, *J* = 8.7 Hz, 1H), 7.69 (s, 1H), 7.43 - 7.34 (m, 1H), 4.12 (d, *J* = 14.6 Hz, 1H), 3.70 (d, *J* = 12.0 Hz, 1H), 3.59 (d, *J* = 12.2 Hz, 2H), 3.06 - 2.88 (m, 2H), 2.84-2.64 (m, 7H), 2.61 (s, 1H), 2.40-2.20 (m, 1H), 1.16 (d, *J* = 6.1 Hz, 3H).

### Example 53: preparation of (S)-N-methyl-5-(2-methyl-4-((8-methyl-6-oxo-7-(trifluoromethyl)-5,6-dihydro-1,5-naphthyridin-3-yl)methyl)piperazin-1-yl)picolinamide (compound (53))

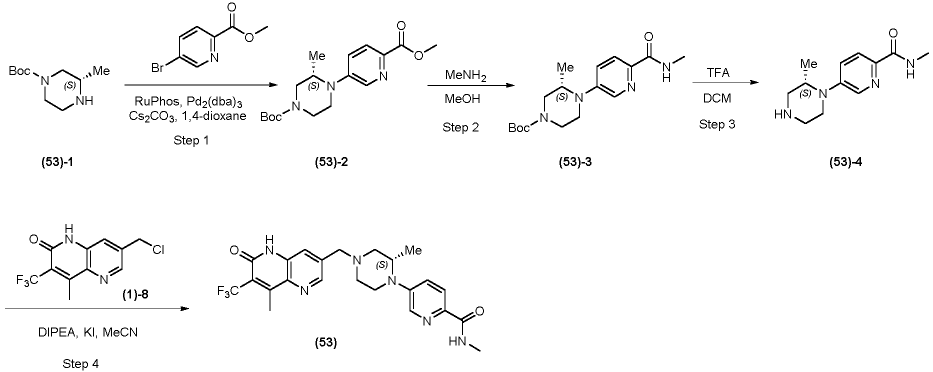

### Steps 1-4. preparation of compound (53)

Using (S)-4-N-*tert*-butyloxycarbonyl-2-methylpiperazine **((53)-1)** as the starting material, **compound (53)** was prepared according to a method similar to the synthetic route of **compound (3).** MS-ESI m/z: 475.2 [M+H]⁺. ¹H NMR (400 MHz, Chloroform-*d*) δ 12.84 (s, 1H), 8.65 (d, *J* = 2.0 Hz, 1H), 8.11 (d, *J* = 2.8 Hz, 1H), 8.05 (d, *J* = 8.8 Hz, 1H), 7.84 (d, *J* = 2.0 Hz, 1H), 7.80 - 7.74 (m, 1H), 7.17 (dd, *J* = 8.8, 2.8 Hz, 1H), 4.15 - 4.06 (m, 1H), 3.80 - 3.63 (m, 2H), 3.48 - 3.40 (m, 1H), 3.31 - 3.20 (m, 1H), 3.01 (d, *J* = 5.2 Hz, 3H), 2.97 - 2.93 (m, 1H), 2.93 - 2.89 (m, 3H), 2.81 - 2.72 (m, 1H), 2.55 (dd, *J =* 11.2, 3.6 Hz, 1H), 2.44 - 2.32 (m, 1H), 1.25 (d, *J* = 6.8 Hz, 3H).

### Example 54: preparation of (R)-N-methyl-5-(2-methyl-4-((8-methyl-6-oxo-7-(trifluoromethyl)-5,6-dihydro-1,5-naphthyridin-3-yl)methyl)piperazin-1-yl)picolinamide (compound (54))

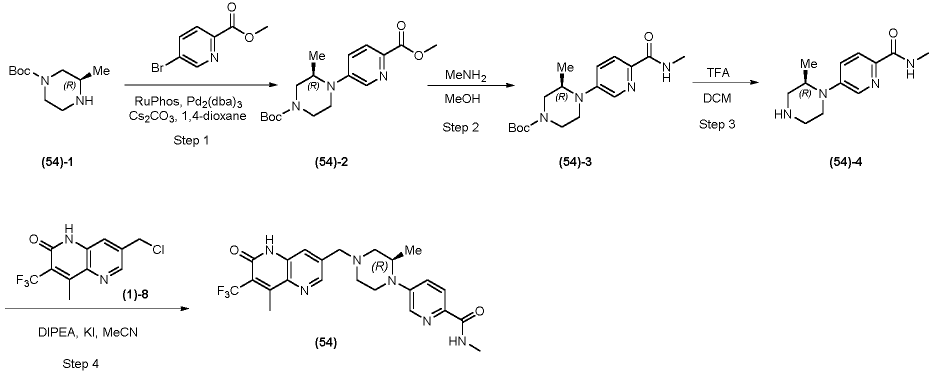

### Steps 1-4. preparation of compound (54)

Using (R)-4-N-*tert*-butyloxycarbonyl-2-methylpiperazine **((54)-1)** as the starting material, **compound** (54) was prepared according to a method similar to the synthetic route of **compound (3).** MS-ESI m/z: 475.2 [M+H]⁺. ¹H NMR (400 MHz, Chloroform-*d*) δ 12.84 (s, 1H), 8.65 (d, *J =* 2.0 Hz, 1H), 8.11 (d, *J* = 2.8 Hz, 1H), 8.05 (d, *J* = 8.8 Hz, 1H), 7.84 (d, *J* = 2.0 Hz, 1H), 7.80 - 7.74 (m, 1H), 7.17 (dd, *J* = 8.8, 2.8 Hz, 1H), 4.15 - 4.06 (m, 1H), 3.80 - 3.63 (m, 2H), 3.48 - 3.40 (m, 1H), 3.31 - 3.20 (m, 1H), 3.01 (d, *J* = 5.2 Hz, 3H), 2.97 - 2.93 (m, 1H), 2.93 - 2.89 (m, 3H), 2.81 - 2.72 (m, 1H), 2.55 (dd, *J* = 11.2, 3.6 Hz, 1H), 2.44 - 2.32 (m, 1H), 1.25 (d, *J* = 6.8 Hz, 3H).

### Examples 55 and 56: preparation of N-methyl-8-((8-methyl-6-oxo-7-(trifluoromethyl)-5,6-dihydro-1,5-naphthyridin-3-yl)methyl)-6,7,8,9-tetrahydropyrido[3',4':4,5]imidazo[1,2-a]pyrazine-3-carboxamide and N-methyl-7-((8-methyl-6-oxo-7-(trifluoromethyl)-5,6-dihydro-1,5-naphthyridin-3-yl)methyl)-6,7,8,9-tetrahydropyrido[4',3':4,5]imidazo[1,2-a]pyrazine-3-carboxamide

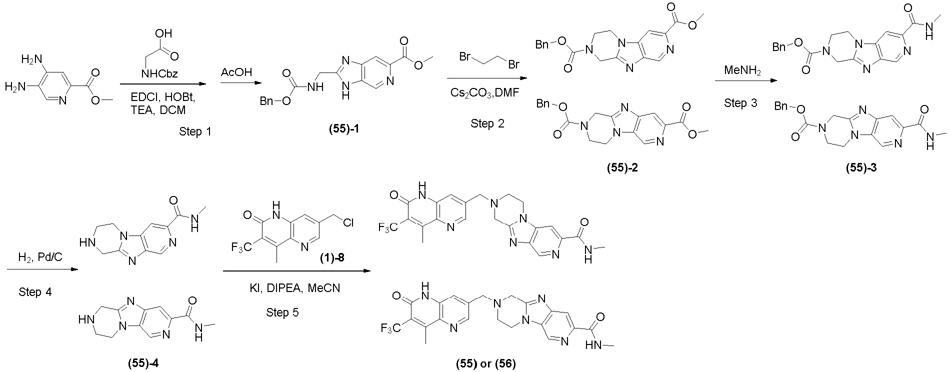

### Step 1. preparation of compound (55)-1

Methyl 4,5-diaminopyridine-carboxylate (900 mg) and benzyloxycarbonyl-glycine (1.24 g) were dissolved in dichloromethane (20 mL), and EDCI (1.67 g), HOBt (1.98 g), and TEA (1.45 g) were added. The reaction was carried out at 40°C for 3 hours. The reaction solution was poured into water and filtered, resulting in a crude white solid (800 mg). MS-ESI m/z: 359.35 [M+H]⁺. The crude product (500 mg) was dissolved in acetic acid (5 mL) and reacted at 120°C for 2 hours. After cooling, the mixture was concentrated, and the residue was purified by silica gel column chromatography (eluent: dichloromethane-methanol = 10:1). The eluate was collected and the solvent was evaporated under reduced pressure to obtain a light yellow oily product **(55)-1** (320 mg, yield: 67%). MS-ESI m/z: 341.34 [M+H]⁺.

### Step 2. preparation of compound (55)-2

Compound **(55)-1** (300 mg) was dissolved in N,N-dimethylformamide (6 mL), and cesium carbonate (574 mg) and 1,2-dibromoethane (497 mg) were added. The reaction was carried out at 80°C for 5 hours. After the reaction was completed, water was added to dilute the mixture, which was then extracted with ethyl acetate. The organic phase was washed with saturated brine, dried, and concentrated. The residue was purified by silica gel column chromatography (eluent: dichloromethane-methanol = 20:1). The eluate was collected and the solvent was evaporated under reduced pressure to obtain a white solid **(55)-2** (220 mg, yield: 68%). MS-ESI m/z: 367.38 [M+H]⁺.

### Step 3. preparation of compound (55)-3

Compound **(55)-2** (220 mg) was dissolved in methanol (5 mL), and an aqueous solution of methylamine (2 mL, 40% wt) was added. The reaction was carried out in a sealed container at 90°C for 1 hour. After the reaction was completed, the mixture was concentrated. The residue was purified by silica gel column chromatography (eluent: dichloromethane-methanol = 10:1). The eluate was collected and the solvent was evaporated under reduced pressure to obtain a light yellow oily product **(55)-3** (180 mg, yield 82%). MS-ESI m/z: 366.39 [M+H]⁺.

### Step 4. preparation of compound (55)-4

Compound **(55)-3** (180 mg) was dissolved in methanol (5 mL), and 10% palladium on carbon (20 mg) was added. The reaction was carried out overnight under a hydrogen atmosphere at room temperature. After the reaction was completed, the mixture was filtered through diatomaceous earth and concentrated to obtain a white solid **(55)-4** (90 mg), which was used directly in the next step. MS-ESI m/z: 232.26 [M+H]⁺.

### Step 5. preparation of compound (55) and (56)

Compound **(55)-4** (90 mg) was dissolved in acetonitrile (5 mL), and **(1)-8** (60 mg), DIPEA (130 mg), and potassium iodide (54 mg) were added. The reaction was carried out at 80°C for 1 hour. After the reaction was completed, the mixture was diluted with water and extracted with dichloromethane. The organic phase was dried over anhydrous sodium sulfate, filtered, and concentrated. The residue was purified by silica gel column chromatography (eluent: dichloromethane-methanol = 10:1). The eluate was collected and concentrated to obtain a white solid **(55)** (18 mg) and a white solid **(56)** (16 mg)(yield 33%).

**(55):** MS-ESI m/z: 472.44 [M+H]⁺. ¹H NMR (400 MHz, DMSO-*d*₆) δ 12.26 (s, 1H), 8.89 (s, 1H), 8.83-8.63 (m, 1H), 8.63 (d, *J* = 1.9 Hz, 1H), 8.17 (s, 1H), 7.74 (d, *J* = 1.9 Hz, 1H), 4.35 (t, *J* = 5.4 Hz, 2H), 4.01 (d, *J* = 10.0 Hz, 4H), 3.10 (t, *J* = 5.4 Hz, 2H), 2.84 (d, *J* = 4.8 Hz, 3H), 2.87-2.67 (m, 3H). (56): MS-ESI m/z: 472.44 [M+H]⁺. ¹H NMR (400 MHz, DMSO-*d*₆) δ 12.26 (s, 1H), 8.87 (d, *J* = 1.0 Hz, 1H), 8.87-8.67 (m, 1H), 8.63 (d, *J* = 1.8 Hz, 1H), 8.25 (d, *J* = 1.0 Hz, 1H), 7.74 (d, *J* = 1.9 Hz, 1H), 4.31 (t, *J* = 5.5 Hz, 2H), 4.00 (d, *J* = 3.3 Hz, 4H), 3.14 - 3.06 (m, 2H), 2.85 (d, *J* = 4.9 Hz, 3H), 2.88-2.78 (m, 3H).

### Example 57: preparation of N-methyl-5-(3-((8-methyl-6-oxo-7-(trifluoromethyl)-5,6-dihydro-1,5-naphthyridin-3-yl)methyl)-3,8-diazabicyclo[3.2.1]octan-8-yl)picolinamide (compound (57))

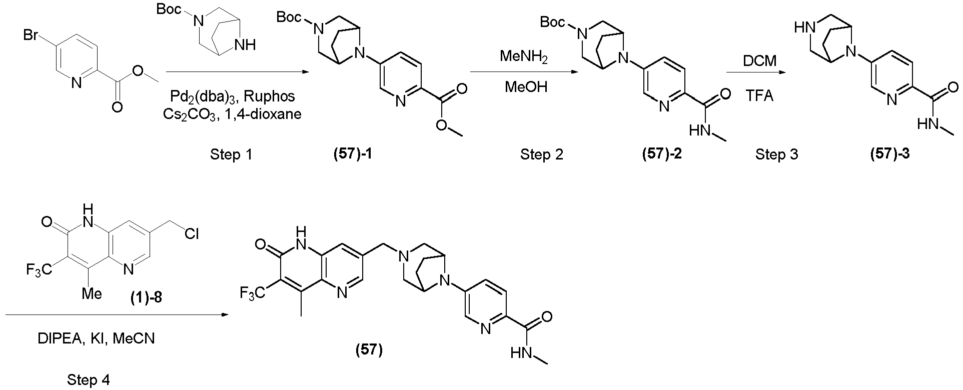

### Steps 1-4. preparation of compound (57)

Using methyl 5-bromopicolinate as the starting material, **compound (57)** was prepared according to a method similar to the synthetic route of **compound (3).** MS-ESI m/z: 487.2 [M+H]⁺. ¹H NMR (400 MHz, DMSO-*d*₆) δ 12.28 (s, 1H), 8.51 (d, *J* = 1.8 Hz, 1H), 8.34 (d, *J* = 4.9 Hz, 1H), 8.16 (d, *J* = 2.8 Hz, 1H), 7.80 (d, *J* = 8.7 Hz, 1H), 7.71 (d, *J* = 1.9 Hz, 1H), 7.29 (d, *J* = 6.0 Hz, 1H), 3.56 (s, 2H), 2.99 (d, *J* = 5.2 Hz, 1H), 2.82 - 2.71 (m, 6H), 2.56 - 2.54 (m, 1H), 2.42 - 2.39 (m, 2H), 2.14 - 2.04 (m, 4H), 1.98 - 1.89 (m, 2H).

### Example 69: preparation of N,6'-dimethyl-1'-((8-methyl-6-oxo-7-(trifluoromethyl)-5,6-dihydro-1,5-naphthyridin-3-yl)methyl)-1',2',3',6'-tetrahydro-[3,4'-bipyridine]-6-carboxamide (compound (69))

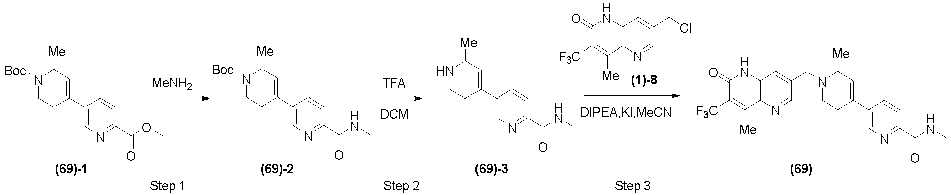

### Steps 1-3: preparation of compound (69)

Using 1'-(*tert*-butyl) 6-methyl 6'-methyl-3',6'-dihydro-[3,4'-bipyridine]-1',6(2'H)-dicarboxylate **((69)-1)** as the starting material, **compound (69)** was prepared according to a method similar to the synthetic route of **compound (14).** MS-ESI m/z: 472.4 [M+H]⁺. ¹H NMR (400 MHz, DMSO-*d*₆) δ 12.18 (s, 1H), 8.74 - 8.65 (m, 2H), 8.55-8.61 (m, 1H), 8.04 - 7.96 (m, 2H), 7.70-7.76 (m, 1H), 6.40 - 6.35 (m, 1H), 3.95 - 3.90 (m, 2H), 3.19 (s, 1H), 3.01 - 2.91 (m, 2H), 2.84 - 2.80 (m, 3H), 2.73-2.79 (m , 3H), 2.28 (s, 1H), 1.94-2.05 (m, 1H), 1.13 (m , 3H).

### Example 70: preparation of N-methyl-5-(2-methyl-1-((8-methyl-6-oxo-7-(trifluoromethyl)-5,6-dihydro-1,5-naphthyridin-3-yl)methyl)piperidin-4-yl)picolinamide (compound (70))

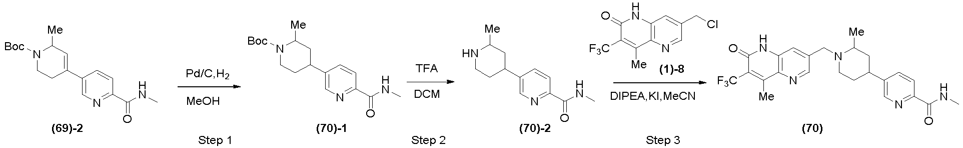

### Step 1: preparation of compound (70)-1

Compound **(69)-2** (49 mg) was dissolved in anhydrous methanol (3 mL), and wt 10% wet palladium on carbon was added. The reaction was carried out under a hydrogen atmosphere at room temperature for 1 hour. After the reaction was completed, the mixture was filtered through diatomaceous earth and concentrated to obtain the crude product **(70)-1** (50 mg), which was used directly in the next step. MS-ESI m/z: 334.4 [M+H]⁺.

### Steps 2-3: preparation of compound (70)

Using **((70)-1)** as the starting material, **compound (70)** was prepared according to a method similar to steps 2-3 in the synthetic route of **compound (69).** MS-ESI m/z: 474.50 [M+H]⁺. ¹H NMR (400 MHz, DMSO-*d*₆) δ 12.19 (s, 1H), 8.69 (d, J = 5.5 Hz, 1H), 8.56-8.62 (m, 1H), 8.49-8.55 (m, 1H), 7.92-7.99 (m, 1H), 7.86 (m, 1H), 7.74 - 7.68 (m, 1H), 4.22 (d, J = 14.5 Hz, 1H), 3.84 (d, J = 14.9 Hz, 1H), 3.72 (d, J = 14.7 Hz, 1H), 3.18 (m, 1H), 2.98-3.1 (m, 1H), 2.85 - 2.73 (m, 6H), 2.62-2.67(m, 1H), 1.99 (m, 1H), 1.79 - 1.59 (m, 3H), 1.17 (m, 3H).

### Example 71: preparation of N-methyl-5-(1-((8-methyl-6-oxo-7-(trifluoromethyl)-5,6-dihydro-1,5-naphthyridin-3-yl)methyl)piperidin-4-yl)picolinamide (compound (71))

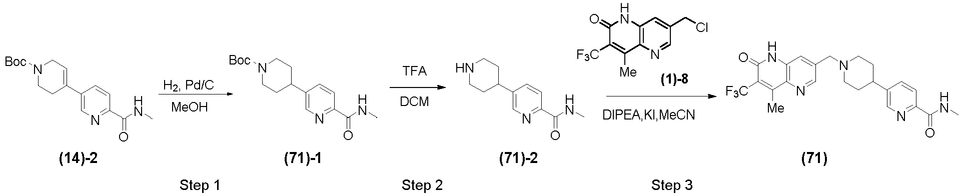

### Steps 1-3: preparation of compound (71)

Using **((14)-2)** as the starting material, **compound (71)** was prepared according to a method similar to the synthetic route of **compound (70).** MS-ESI m/z: 460.2 [M+H]⁺. ¹H NMR (400 MHz, DMSO-*d*₆) δ 12.24 (s, 1H), 8.74 - 8.67 (m, 1H), 8.57 (d, J = 1.8 Hz, 1H), 8.52 (d, J = 2.2 Hz, 1H), 7.95 (d, J = 8.1 Hz, 1H), 7.89 - 7.82 (m, 1H), 7.67 (s, 1H), 3.68 (s, 2H), 3.19 - 3.10 (m, 1H), 2.94 (d, J = 10.6 Hz, 2H), 2.84 - 2.69 (m, 6H), 2.22 - 2.10 (m, 2H), 1.86 - 1.66 (m, 4H).

### Example 72: preparation of 5-(2-cyano-4-((8-methyl-6-oxo-7-(trifluoromethyl)-5,6-dihydro-1,5-naphthyridin-3-yl)methyl)piperazin-1-yl)-N-methylpicolinamide (compound (72))

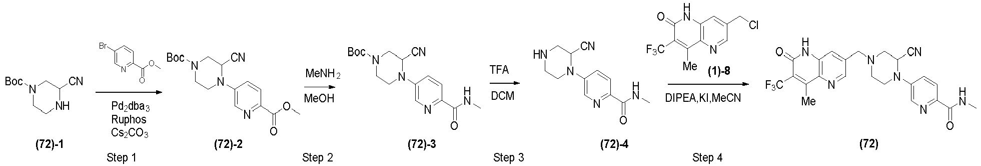

### Steps 1-4: preparation of compound (72)

Using *tert*-butyl 3-cyanopiperazine-1-carboxylate **((72)-1)** as the starting material, **compound (72)** was prepared according to a method similar to the synthetic route of **compound (21).** MS-ESI m/z: 486.2 [M+H]⁺. ¹H NMR (400 MHz, DMSO-*d*₆) δ 8.62 (s, 1H), 8.55 (d, J = 4.9 Hz, 1H), 8.42 (d, J = 2.7 Hz, 1H), 7.94 (d, J = 8.7 Hz, 1H), 7.72 (s, 1H), 7.60 (d, J = 8.9 Hz, 1H), 5.48 (s, 1H), 3.81 (d, J = 14.3 Hz, 2H), 3.15 (d, J = 11.8 Hz, 1H), 3.05 - 2.95 (m, 2H), 2.83 - 2.74 (m, 6H), 2.69 (s, 1H), 2.58 - 2.53 (m, 1H), 2.38 - 2.30 (m, 1H), 2.21 - 2.12 (m, 1H).

### Example 73: preparation of 5-(2-(cyanomethyl)-4-((8-methyl-6-oxo-7-(trifluoromethyl)-5,6-dihydro-1,5-naphthyridin-3-yl)methyl)piperazin-1-yl)-N-methylpicolinamide (compound (73))

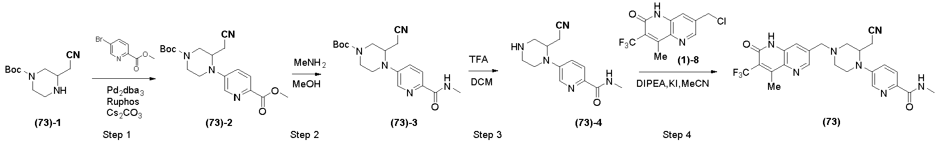

### Steps 1-4: preparation of compound (73)

Using *tert*-butyl 3-(cyanomethyl)piperazine-1-carboxylate **((73)-1)** as the starting material, **compound (73)** was prepared according to a method similar to the synthetic route of **compound (72).** MS-ESI m/z: 500.2 [M+H]⁺. ¹H NMR (400 MHz, DMSO-*d*₆) δ 12.28 (s, 1H), 8.61 (s, 1H), 8.42 (d, J = 4.9 Hz, 1H), 8.31 (d, J = 2.9 Hz, 1H), 7.84 (d, J = 8.8 Hz, 1H), 7.73 (s, 1H), 7.49 - 7.40 (m, 1H), 4.63 (s, 1H), 3.78 (s, 1H), 3.67 (d, J = 11.0 Hz, 2H), 3.21 - 3.10 (m, 1H), 3.05 - 2.64 (m, 9H), 2.43 - 2.24 (m, 3H).

### Example 74: preparation of 5-(2-(methoxymethyl)-4-((8-methyl-6-oxo-7-(trifluoromethyl)-5,6-dihydro-1,5-naphthyridin-3-yl)methyl)piperazin-1-yl)-N-methylpicolinamide (compound (74))

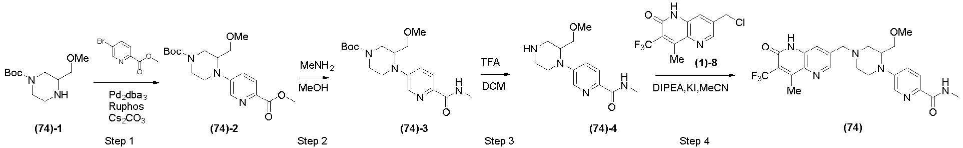

### Steps 1-4: preparation of compound (74)

Using *tert*-butyl 3-(methoxymethyl)piperazine-1-carboxylate **((74)-1)** as the starting material, **compound (74)** was prepared according to a method similar to the synthetic route of **compound (73).** MS-ESI m/z: 505.3 [M+H]⁺. ¹H NMR (400 MHz, Chloroform-d) δ 12.98 (s, 1H), 8.63 (d, J = 1.8 Hz, 1H), 8.13 (d, J = 2.9 Hz, 1H), 8.04 (d, J = 8.7 Hz, 1H), 7.86 (s, 1H), 7.78 (q, J = 5.0 Hz, 1H), 7.18 (dd, J = 8.9, 2.9 Hz, 1H), 4.08 - 4.00 (m, 1H), 3.87 (t, J = 9.0 Hz, 1H), 3.80 - 3.67 (m, 2H), 3.50 (d, J = 12.4 Hz, 1H), 3.35 (dd, J = 8.9, 4.1 Hz, 1H), 3.27 (s, 3H), 3.20 (dd, J = 12.0, 3.5 Hz, 1H), 3.10 (d, J = 11.5 Hz, 1H), 3.00 (d, J = 5.1 Hz, 3H), 2.90 (d, J = 3.3 Hz, 3H), 2.43 (dd, J = 11.3, 3.5 Hz, 1H), 2.39-2.32 (m, J = 11.4, 3.4 Hz, 1H), 2.10-2.02 (m, 1H).

### Example 75: preparation of 5-(2-(hydroxymethyl)-4-((8-methyl-6-oxo-7-(trifluoromethyl)-5,6-dihydro-1,5-naphthyridin-3-yl)methyl)piperazin-1-yl)-N-methylpicolinamide (compound (75))

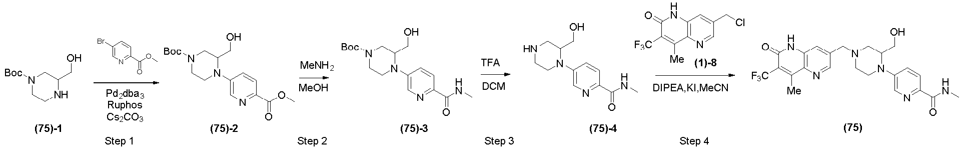

### Steps 1-4: preparation of compound (75)

Using tert-butyl 3-(hydroxymethyl)piperazine-1-carboxylate **((75)-1)** as the starting material, **compound (75)** was prepared according to a method similar to the synthetic route of **compound (21).** MS-ESI m/z: 491.2 [M+H]⁺. ¹H NMR (400 MHz, DMSO-*d*₆) δ 12.23 (s, 1H), 8.56 (d, J = 10.2 Hz, 2H), 8.26 (d, J = 2.8 Hz, 1H), 7.96 (d, J = 8.7 Hz, 1H), 7.66 (s, 1H), 7.51 (dd, J = 8.7, 2.9 Hz, 1H), 6.27 - 6.01 (m, 1H), 4.14 - 3.94 (m, 2H), 3.65 (s, 2H), 3.45 (s, 3H), 3.12 (d, J = 7.8 Hz, 1H), 2.96 - 2.61 (m, 7H), 2.18 -1.93 (m, 2H).

### Example 76: preparation of N-methyl-5-(4-((8-methyl-6-oxo-7-(trifluoromethyl)-5,6-dihydro-1,5-naphthyridin-3-yl)methyl)-2-(trifluoromethyl)piperazin-1-yl)picolinamide (compound (76))

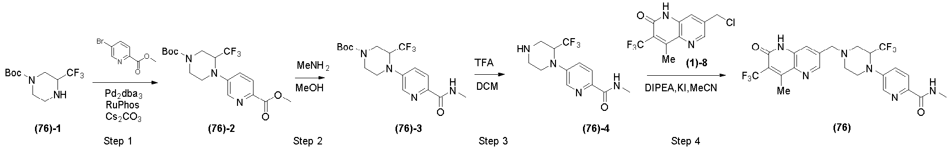

### Steps 1-4: preparation of compound (76)

Using *tert*-butyl 3-(trifluoromethyl)piperazine-1-carboxylate **((76)-1)** as the starting material, **compound (76)** was prepared according to a method similar to the synthetic route of **compound (21).** MS-ESI m/z: 529.2 [M+H]⁺. ¹H NMR (400 MHz, DMSO-*d*₆) δ 12.34 (s, 1H), 8.57 (d, J = 2.0 Hz, 1H), 8.43 (q, J = 4.8 Hz, 1H), 8.37 (d, J = 2.8 Hz, 1H), 7.85 (d, J = 8.8 Hz, 1H), 7.69 (d, J = 2.0 Hz, 1H), 7.50 (dd, J = 8.8, 2.8 Hz, 1H), 5.25 - 5.07 (m, 1H), 3.81 - 3.68 (m, 2H), 3.63 (d, J = 12.4 Hz, 1H), 3.35 - 3.26 (m, 2H), 3.15 (d, J = 12.4 Hz, 1H), 2.96 (d, J = 11.2 Hz, 1H), 2.84 - 2.68 (m, 5H), 2.47 - 2.44 (m, 1H), 2.31 - 2.20 (m, 1H).

### Example 77: preparation of 5-(3,3-dimethyl-4-((8-methyl-6-oxo-7-(trifluoromethyl)-5,6-dihydro-1,5-naphthyridin-3-yl)methyl)piperazin-1-yl)-N-methylpicolinamide (compound (77))

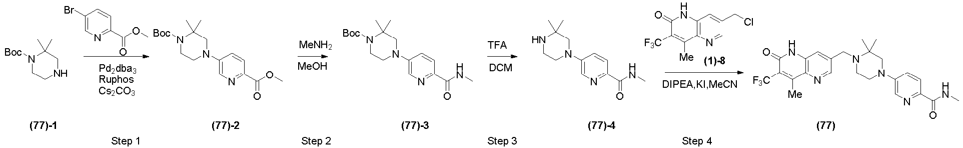

### Steps 1-4: preparation of compound (77)

Using *tert*-butyl 2,2-dimethylpiperazine-1-carboxylate **((77)-1)** as the starting material, **compound (77)** was prepared according to a method similar to the synthetic route of **compound (21).** MS-ESI m/z: 489.2 [M+H]⁺. ¹H NMR (400 MHz, DMSO-*d*₆) δ 12.20 (s, 1H), 8.59 (s, 1H), 8.36 (q, J = 4.8 Hz, 1H), 8.26 (d, J = 2.9 Hz, 1H), 7.81 (d, J = 8.8 Hz, 1H), 7.71 (s, 1H), 7.39 (d, J = 5.9 Hz, 1H), 3.70 (s, 2H), 3.31 - 3.24 (m, 2H), 3.21 (s, 2H), 2.81 - 2.73 (m, 6H), 2.54 - 2.50 (m, 2H), 1.17 (s, 6H).

### Example 78: preparation of N-methyl-5-(4-((8-methyl-6-oxo-7-(trifluoromethyl)-5,6-dihydro-1,5-naphthyridin-3-yl)methyl)-4,7-diazaspiro[2.5]octan-7-yl)picolinamide (compound (78))

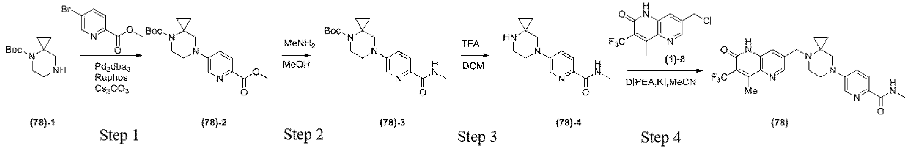

### Steps 1-4: preparation of compound (78)

Using *tert*-butyl 4,7-diazaspiro[2.5]octane-4-carboxylate **((78)-1)** as the starting material, **compound (78)** was prepared according to a method similar to the synthetic route of **compound (21).** MS-ESI m/z: 487.2 [M+H]⁺. ¹H NMR (400 MHz, DMSO-*d*₆) δ 12.17 (s, 1H), 8.55 (d, J = 1.8 Hz, 1H), 8.40 (q, J = 4.9 Hz, 1H), 8.27 (d, J = 2.9 Hz, 1H), 7.84 (d, J = 8.8 Hz, 1H), 7.65 (d, J = 1.9 Hz, 1H), 7.39 (dd, J = 8.9, 2.9 Hz, 1H), 4.04 (s, 2H), 3.45 (s, 3H), 3.26 (s, 2H), 2.91 (t, J = 5.3 Hz, 2H), 2.78 (dd, J = 8.2, 4.2 Hz, 5H), 0.77 - 0.59 (m, 4H).

### Example 79: preparation of N-methyl-5-(1-((8-methyl-6-oxo-7-(trifluoromethyl)-5,6-dihydro-1,5-naphthyridin-3-yl)methyl)-1,2,3,6-tetrahydropyridin-4-yl)pyrazine-2-carboxamide (compound (79))

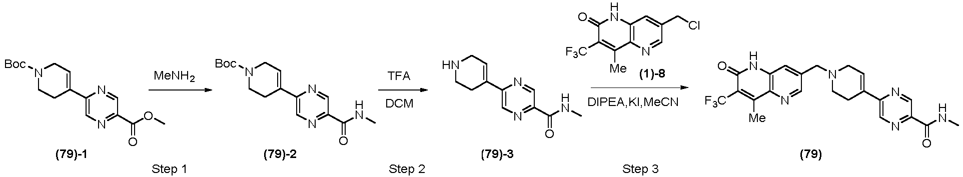

### Steps 1-3: preparation of compound (79)

Using methyl 5-(1-(*tert*-butoxycarbonyl)-1,2,3,6-tetrahydropyridin-4-yl)pyrazine-2-carboxylate **((79)-1)** as the starting material, **compound (79)** was prepared according to a method similar to the synthetic route of **compound (14).** MS-ESI m/z: 459.5 [M+H]⁺. ¹H NMR (400 MHz, DMSO-*d*₆) δ 12.22 (s, 1H), 9.07 (d, J = 1.5 Hz, 1H), 8.89 (d, J = 1.5 Hz, 1H), 8.81 (d, J = 5.0 Hz, 1H), 8.59 (d, J = 1.9 Hz, 1H), 7.72 (d, J= 1.8 Hz, 1H), 7.00 (s, 1H), 3.81 (s, 2H), 3.26 (s, 2H), 2.82 (d, J = 4.8 Hz, 3H), 2.78 (q, J = 3.5 Hz, 3H), 2.72 (d, J = 5.4 Hz, 2H), 2.66 (s, 2H).

### Examples 80 and 81: preparation of N,3'-dimethyl-1'-((8-methyl-6-oxo-7-(trifluoromethyl)-5,6-dihydro-1,5-naphthyridin-3-yl)methyl)-1',2',3',6'-tetrahydro-[3,4'-bipyridine]-6-carboxamide (compound (80)) and N,5'-dimethyl-1'-((8-methyl-6-oxo-7-(trifluoromethyl)-5,6-dihydro-1,5-naphthyridin-3-yl)methyl)-1',2',3',6'-tetrahydro-[3,4'-bipyridine]-6-carboxamide (compound (81))

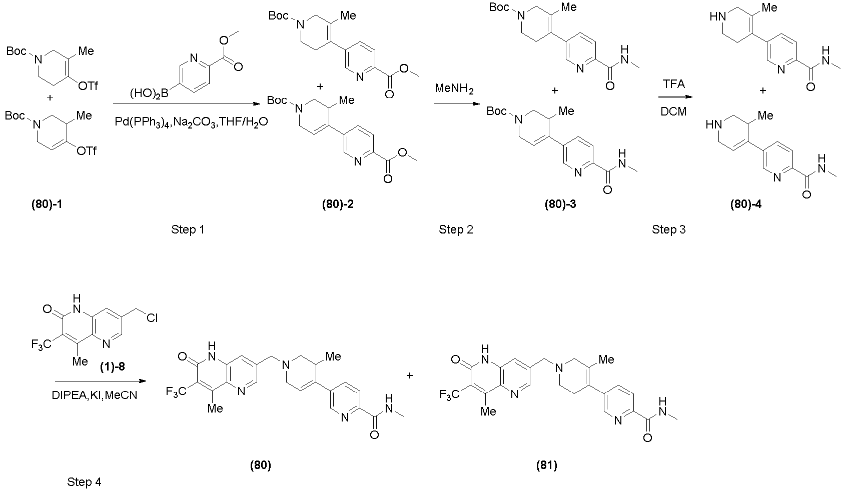

### Step 1. preparation of compound (80)-2

Compound **((80)-1)** (250 mg), which contains two double bond isomers, (6-(methoxycarbonyl)pyridin-3-yl)boronic acid (157.2 mg), Pd(PPh₃)₄ (83 mg), Na₂CO₃ (153 mg), and LiCl (61 mg) were sequentially added to a round-bottom flask. Tetrahydrofuran (5 mL) and water (1 mL) were added, and the reaction was carried out at 80°C under a nitrogen atmosphere for 2 hours. After the reaction was completed, water was added, and the mixture was extracted with ethyl acetate. The organic phase was dried, filtered, and concentrated. The residue was purified by silica gel column chromatography (eluent: petroleum ether-ethyl acetate = 1:1). The eluate was collected and concentrated to obtain a light yellow oily product (100 mg, yield: 41%). MS-ESI m/z: 333.40 [M+H]⁺.

### Steps 2-4. preparation of compound (80) and compound (81)

Using **((80)-2),** which contains two double bond isomers, as the starting material, compound **(80)** and compound **(81)** were prepared according to a method similar to the synthetic route of **compound (14).**
compound **(80):** MS-ESI m/z: 472.4 [M+H]⁺. ¹H NMR (400 MHz, DMSO-*d*₆) δ 12.28 (s, 1H), 8.71 (s, 1H), 8.66 (d, J = 9.5 Hz, 1H), 8.60 (s, 1H), 7.97 (s, 2H), 7.74 (s, 1H), 6.21 (s, 1H), 3.83 (d, J = 14.3 Hz, 1H), 3.72 (d, J = 14.2 Hz, 1H), 3.61 (s, 2H), 3.14 (s, 2H), 2.99 (s, 1H), 2.81 (d, J = 4.9 Hz, 3H), 2.78 (d, J = 3.4 Hz, 3H), 0.99 (d, J = 6.9 Hz, 3H).
compound **(81):** MS-ESI m/z: 472.4 [M+H]⁺. ¹H NMR (400 MHz, DMSO-*d*₆) δ 12.23 (s, 1H), 8.73 (s, 1H), 8.59 (s, 1H), 8.46 (s, 2H), 7.98 (s, 1H), 7.77 (d, J = 49.1 Hz, 1H), 3.77 (s, 2H), 3.61 (m, 2H), 3.14 (m, J = 4.6 Hz, 2H), 3.01 (m, 2H), 2.81 (d, J = 4.4 Hz, 3H), 2.78 (m, 3H), 1.23 - 1.16 (s, 3H).

### Example 82: preparation of N-methyl-1'-((8-methyl-6-oxo-7-(trifluoromethyl)-5,6-dihydro-1,5-naphthyridin-3-yl)methyl)-1',2',3',6'-tetrahydro-[2,4'-bipyridine]-5-carboxamide (compound (82))

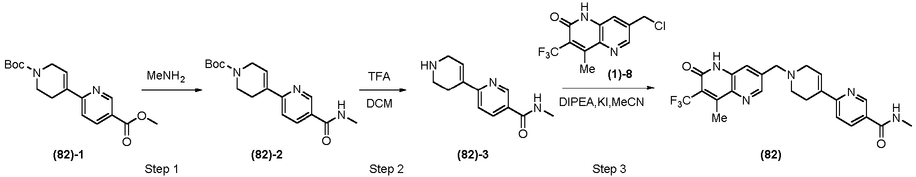

### Steps 1-3: preparation of compound (82)

Using 1'-(tert-butyl) 5-methyl 3',6'-dihydro-[2,4'-bipyridine]-1',5(2'H)-dicarboxylate **((82)-1)** as the starting material, **compound (82)** was prepared according to a method similar to the synthetic route of **compound (14).** MS-ESI m/z: 458.2 [M+H]⁺. ¹H NMR (400 MHz, DMSO-*d*₆) δ 12.22 (s, 1H), 8.93 (d, J = 2.3 Hz, 1H), 8.66 - 8.51 (m, 2H), 8.13 (dd, J = 8.4, 2.3 Hz, 1H), 7.71 (d, J = 1.9 Hz, 1H), 7.63 (d, J = 8.4 Hz, 1H), 6.82 (s, 1H), 3.79 (s, 2H), 3.37 (s, 2H), 3.21 (s, 2H), 2.80 - 2.75 (m, 3H), 2.51 (s, 3H), 2.73 - 2.61 (m, 2H).

### Example 83: preparation of 5-methyl-2-(4-((8-methyl-6-oxo-7-(trifluoromethyl)-5,6-dihydro-1,5-naphthyridin-3-yl)methyl)piperazin-1-yl)-5,6-dihydro-4H-pyrrolo[3,4-d]thiazol-4-one (compound (83))

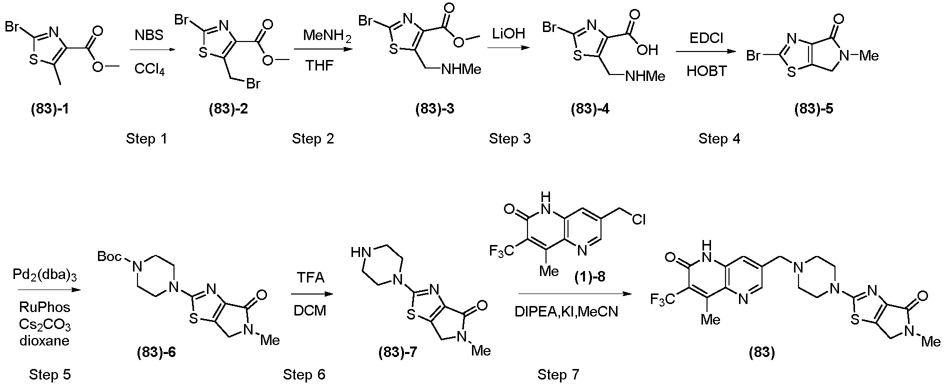

### Steps 1-7: preparation of compound (83)

Using methyl 2-bromo-5-methylthiazole-4-carboxylate **((83)-1)** as the starting material, **compound (83)** was prepared according to a method similar to steps 1-7 in the synthetic route of **compound (17).** MS-ESI m/z: 479.1 [M+H]⁺. ¹H NMR (400 MHz, DMSO-*d*₆) δ 12.26 (s, 1H), 8.58 (s, 1H), 7.69 (s, 1H), 4.38 (s, 2H), 3.72 (s, 2H), 3.49 - 3.44 (m, 4H), 2.99 (s, 3H), 2.77 (q, J = 3.5 Hz, 3H), 2.56 (t, J = 5.1 Hz, 4H).

### Example 84: preparation of 4-methyl-7-((4-(2-methyl-3-oxo-2,3-dihydro-1H-pyrrolo[3,4-c]pyridin-6-yl)piperazin-1-yl)methyl)-3-(trifluoromethyl)-1,5-naphthyridin-2(1H)-one (compound (84))

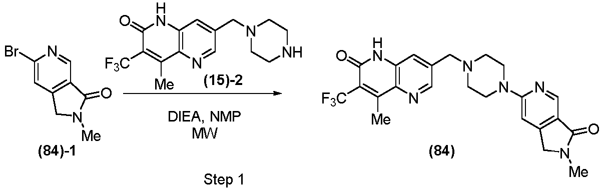

### Step 1. preparation of compound (84)

Using 6-bromo-2-methyl-1,2-dihydro-3H-pyrrolo[3,4-c]pyridin-3-one **((84)-1)** as the starting material, **compound (84)** was prepared according to a method similar to step 3 in the synthetic route of **compound (15).** MS-ESI m/z: 473.4 [M+H]⁺. ¹H NMR (400 MHz, Chloroform-d) δ 11.5 (s, 1H) 8.66 (d, J = 14.4 Hz, 1H), 7.72 (s, 1H), 6.99 (d, J = 8.3 Hz, 1H), 6.63 (s, 1H), 4.31 (s, 2H), 3.73 (d, J = 11.5 Hz, 4H), 3.12 (d, J = 26.9 Hz, 2H), 2.86 (d, J = 50.8 Hz, 2H), 2.64 (s, 2H), 2.33 (d, J = 40.0 Hz, 3H), 2.06 (s, 3H).

### Example 85: preparation of 5-(4-((7-cyano-8-methyl-6-oxo-5,6-dihydro-1,5-naphthyridin-3-yl)methyl)piperazin-1-yl)-N-methylpicolinamide (compound (85))

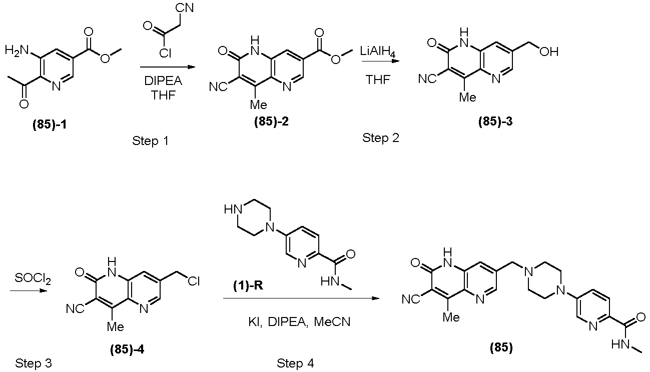

### Step 1. preparation of compound (85)-2

**((85)-1)** (50 mg) and 2-cyanacetyl chloride (53 mg) were dissolved in THF (2 mL) and reacted at 0 °C for 5 min. DIPEA (66 mg) was added, the mixture was allowed to return to room temperature and the reaction was continued for 2h. Upon completion, water was added to quench the reaction, and the mixture was extracted with ethyl acetate. The organic phase was washed with saturated brine, dried over anhydrous sodium sulfate, filtered, and concentrated. The redidue was purified by column chromatography [eluent: petroleum ether-ethyl acetate = 9:1], the eluate was collected, and the solvent was evaporated under reduced pressure to obtain a white solid **((85)-2)** (40 mg, yield: 64%). MS-ESI m/z: 244.1 [M+H]⁺.

### Steps 2-4. preparation of compound (85)

Using methyl 6-acetyl-5-aminonicotinate **((85)-2)** as the starting material, **compound (85)** was prepared according to a method similar to steps 6-8 in the synthetic route of **compound (1).** MS-ESI m/z: 418.2 [M+H]⁺. ¹H NMR (400 MHz, Chloroform-d) δ 12.58 (s, 1H), 8.71 (d, J = 1.8 Hz, 1H), 8.15 (d, J = 2.8 Hz, 1H), 8.05 (d, J = 8.7 Hz, 1H), 7.90 (d, J = 1.8 Hz, 1H), 7.79 (d, J = 5.3 Hz, 1H), 7.21 (dd, J = 8.8, 2.9 Hz, 1H), 3.79 (s, 2H), 3.35 (t, J = 5.1 Hz, 4H), 3.01 (d, J = 5.1 Hz, 3H), 2.93 (s, 3H), 2.69 (t, J = 5.0 Hz, 4H).

### Example 86: preparation of (S)-5-(2-cyano-4-((8-methyl-6-oxo-7-(trifluoromethyl)-5,6-dihydro-1,5-naphthyridin-3-yl)methyl)piperazin-1-yl)-N-methylpicolinamide (compound (86))

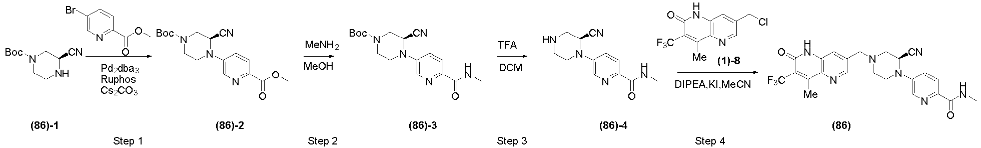

### Steps 1-4. preparation of compound (86)

Using *tert*-butyl (S)-3-cyanopiperazine-1-carboxylate **((86)-1)** as the starting material, **compound (86)** was prepared according to a method similar to the synthetic route of **compound (21).** MS-ESI m/z: 486.1 [M+H]⁺. ¹H NMR (400 MHz, DMSO-*d*₆) δ 12.35 (s, 1H), 8.63 (d, J = 1.9 Hz, 1H), 8.56 (q, J = 4.8 Hz, 1H), 8.43 (d, J = 2.8 Hz, 1H), 7.95 (d, J = 8.8 Hz, 1H), 7.72 (d, J = 1.9 Hz, 1H), 7.60 (dd, J = 8.9, 2.9 Hz, 1H), 5.42 (s, 1H), 3.85 - 3.70 (m, 3H), 3.16 (d, J = 11.9 Hz, 1H), 3.01 (d, J = 11.0 Hz, 3H), 2.82 - 2.76 (m, 5H), 2.56 (dd, J = 12.1, 3.3 Hz, 1H), 2.40 - 2.32 (m, 1H).

### Example 87: preparation of (R)-N-methyl-5-(3-methyl-4-((4-methyl-2-oxo-3-(trifluoromethyl)-1,2-dihydro-1,6-naphthyridin-7-yl)methyl)piperazin-1-yl)picolinamide ((compound (87))

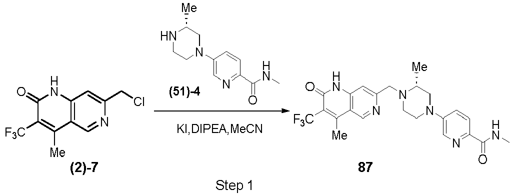

### Step 1. preparation of compound (87)

Using **((2)-7)** and **(51)-4** as the starting materials, **compound (87)** was prepared according to a method similar to step 7 in the synthetic route of **compound (2).** MS-ESI m/z: 475.4 [M+H]⁺. ¹H NMR (400 MHz, DMSO-*d*₆) δ 12.25 (s, 1H), 9.12 (s, 1H), 8.39 (d, J = 4.9 Hz, 1H), 8.28 (d, J = 2.8 Hz, 1H), 7.83 (d, J = 8.8 Hz, 1H), 7.44 - 7.36 (m, 2H), 4.09 (d, J = 15.3 Hz, 1H), 3.73 (d, J = 10.4 Hz, 1H), 3.66 (d, J = 12.4 Hz, 1H), 3.58 (d, J = 15.3 Hz, 1H), 3.02 (t, J = 9.6 Hz, 1H), 2.86 - 2.84 (m, 2H), 2.82 - 2.75 (m, 4H), 2.74 - 2.70 (m, 3H), 2.70 - 2.65 (m, 1H), 1.16 (d, J = 6.1 Hz, 3H).

### Example 88: preparation of (R)-N-methyl-5-(2-methyl-4-((4-methyl-2-oxo-3-(trifluoromethyl)-1,2-dihydro-1,6-naphthyridin-7-yl)methyl)piperazin-1-yl)picolinamide ((compound (88))

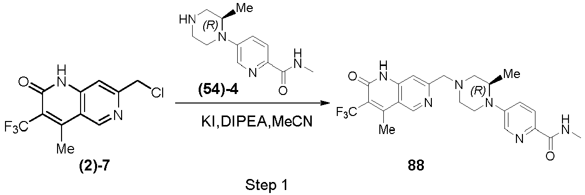

### Step 1. preparation of compound (88)

Using **((2)-7)** and **(54)-4** as the starting materials, **compound (88)** was prepared according to a method similar to step 7 in the synthetic route of **compound (2).** MS-ESI m/z: 475.4 [M+H]⁺. ¹H NMR (400 MHz, DMSO-*d*₆) δ 12.87 - 11.74 (m, 1H), 9.12 (s, 1H), 8.38 (d, J = 4.9 Hz, 1H), 8.23 (d, J = 2.7 Hz, 1H), 7.84 (d, J = 8.8 Hz, 1H), 7.46 (s, 1H), 7.36 (dd, J = 8.9, 2.7 Hz, 1H), 4.25 (s, 1H), 3.79 (d, J = 15.4 Hz, 1H), 3.64 (dd, J = 13.6, 8.0 Hz, 2H), 3.15 - 3.07 (m, 1H), 2.99 (d, J = 11.2 Hz, 1H), 2.79 (d, J = 4.8 Hz, 4H), 2.72 (d, J = 2.6 Hz, 3H), 2.42 (dd, J = 11.2, 3.3 Hz, 1H), 2.37 - 2.26 (m, 1H), 1.20 (d, J = 6.5 Hz, 3H).

### Example 89: preparation of N-methyl-1'-((4-methyl-2-oxo-3-(trifluoromethyl)-1,2-dihydro-1,6-naphthyridin-7-yl)methyl)-1',2',3',6'-tetrahydro-[3,4'-bipyridine]-6-carboxamide ((compound (89))

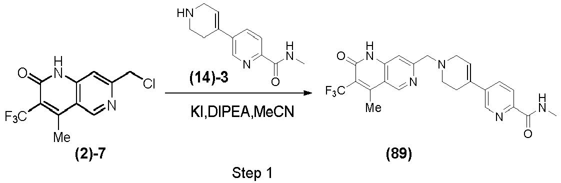

### Step 1. preparation of compound (89)

Using **((2)-7)** and **(14)-3** as the starting materials, **compound (89)** was prepared according to a method similar to step 7 in the synthetic route of **compound (2).** MS-ESI m/z: 458.4 [M+H]⁺. ¹H NMR (400 MHz, DMSO-*d*₆) δ 12.30 (s, 1H), 9.13 (s, 1H), 8.77 - 8.68 (m, 1H), 8.06 - 7.95 (m, 2H), 7.40 (s, 1H), 6.47 (s, 1H), 6.04 (d, J = 6.3 Hz, 1H), 3.82 (s, 2H), 3.57 (s, 1H), 3.25 (d, J = 2.8 Hz, 2H), 2.82 (d, J = 4.9 Hz, 3H), 2.77 - 2.74 (m, 1H), 2.74 - 2.70 (m, 3H), 2.65 - 2.60 (m, 2H).

### Example 92: preparation of (S)-5-(3-cyano-4-((8-methyl-6-oxo-7-(trifluoromethyl)-5,6-dihydro-1,5-naphthyridin-3-yl)methyl)piperazin-1-yl)-N-methylpicolinamide ((compound (92))

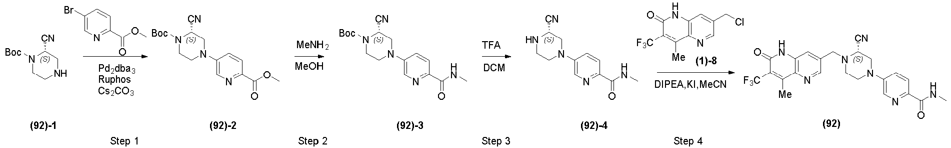

### Steps 1-4. preparation of compound (92)

Using *tert*-butyl (S)-2-cyanopiperazine-1-carboxylate **((92)-1)** as the starting material, **compound (92)** was prepared according to a method similar to the synthetic route of **compound (21).** MS-ESI m/z: 486.1 [M+H]⁺. ¹H NMR (400 MHz, DMSO-*d*₆) δ 12.30 (s, 1H), 8.62 (d, J = 1.9 Hz, 1H), 8.48 (d, J = 4.9 Hz, 1H), 8.33 (d, J = 2.9 Hz, 1H), 7.87 (d, J = 8.7 Hz, 1H), 7.71 (d, J = 1.9 Hz, 1H), 7.48 (dd, J = 8.8, 2.9 Hz, 1H), 4.33 (s, 1H), 4.23 - 4.11 (m, 2H), 3.95 - 3.80 (m, 3H), 3.19 - 3.08 (m, 2H), 2.94 - 2.81 (m, 3H), 2.81 - 2.74 (m, 4H).

### Example 93: preparation of 6-fluoro-N-methyl-5-(4-((8-methyl-6-oxo-7-(trifluoromethyl)-5,6-dihydro-1,5-naphthyridin-3-yl)methyl)piperazin-1-yl)picolinamide ((compound (93))

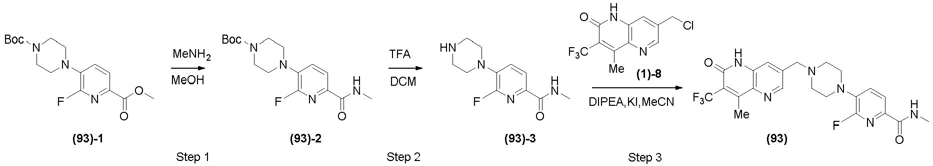

### Steps 1-3. preparation of compound (93)

Using *tert*-butyl 4-(2-fluoro-6-(methoxycarbonyl)pyridin-3-yl)piperazine-1-carboxylate as the starting material, **compound (93)** was prepared according to a method similar to steps 2-4 in the synthetic route of **compound (21).** MS-ESI m/z: 479.5 [M+H]⁺. ¹H NMR (400 MHz, DMSO-*d*₆) δ 12.18 (s, 1H), 8.58 (s, 1H), 8.42 (d, J = 4.9 Hz, 1H), 7.85 (d, J = 7.7 Hz, 1H), 7.69 (s, 1H), 7.64 - 7.52 (m, 1H), 3.73- 7.68 (m, 3H), 3.19 (s, 4H), 2.77 (t, J = 4.0 Hz, 6H), 2.60 (s, 3H).

### Example 94: preparation of 5-(4-((4-fluoro-3-methyl-2-oxo-1,2-dihydroquinolin-7-yl)methyl)piperazin-1-yl)-N-methylpicolinamide ((compound (94))

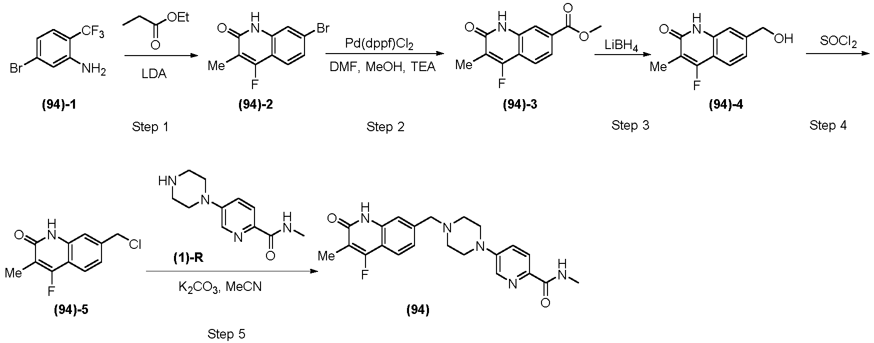

### Step 1. preparation of compound ((94)-2)

In a three-necked flask, ethyl n-propionate (3.82 g) and ultradry tetrahydrofuran (150 mL) were added and the mixture was cooled to -78°C under nitrogen protection. Lithium diisopropylamide (2 M, 28.12 mL) was added dropwise to the reaction system, followed by the slow addition of a tetrahydrofuran solution of **((94)-1)** (2.24 g, 40 mL). The reaction mixture was then allowed to slowly warm to room temperature and react overnight. The reaction was quenched with a saturated aqueous solution of ammonium chloride, filtered, and extracted with ethyl acetate. The solvent was evaporated under reduced pressure to obtain a crude product, which was purified by column chromatography [eluent: petroleum ether-ethyl acetate (20:1-10:1)]. A yellow solid **((94)-2)** was obtained **((94)-2)** (540 mg, yield: 22.5%). MS-ESI m/z: 256.2 [M+H]⁺.

### Step 2. preparation of compound ((94)-3)

In an autoclave, **((94)-2)** (520 mg), Pd(dppf)Cl₂ (149 mg), triethylamine (618 mg), N,N-dimethylformamide (10 mL), and methanol (5 mL) were sequentially added. The mixture was heated to 100°C under a carbon monoxide atmosphere (2 MPa) and stirred overnight. After the reaction was completed, the solvent was evaporated under reduced pressure to obtain a crude product, which was purified by silica gel column chromatography [eluent: petroleum ether-ethyl acetate (10:1-3:1)]. The eluate was collected and the solvent was evaporated under reduced pressure to obtain a yellow solid **((94)-3)** (270 mg, yield: 56.4%). MS-ESI m/z: 236.3 [M+H]⁺.

### Step 3. preparation of compound ((94)-4)

In a reaction flask, **((94)-3)** (270 mg) and tetrahydrofuran (6 mL) were added. Under an ice bath, a tetrahydrofuran solution of LiBH₄ (1.73 mL, 2 M in THF) was added dropwise and the mixture was stirred at room temperature for 1 hour. After the reaction was completed, the reaction solution was poured into a saturated ammonium chloride solution and extracted with dichloromethane. The solvent was evaporated under reduced pressure to obtain a crude product, which was purified by silica gel column chromatography [eluent: dichloromethane-methanol (100:1-10:1)]. The eluate was collected and the solvent was evaporated under reduced pressure to obtain a yellow solid **((94)-4)** (150 mg, yield: 63.1%) MS-ESI m/z: 208.4 [M+H]⁺.

### Step 4. preparation of compound ((94)-5)

In a reaction flask, **((94)-4)** (150 mg) and thionyl chloride (5 mL) were added and the mixture was heated to 70°C and stirred for 1 hour. After the reaction was completed, the solvent was evaporated under reduced pressure to obtain a light yellow solid **((94)-5)** (165 mg, yield: 100%). MS-ESI m/z: 226.3 [M+H]⁺.

### Step 5. preparation of compound (94)

In a reaction flask, **((94)-5)** (165 mg), **(1)-R** (240 mg), potassium carbonate (250 mg), and acetonitrile (10 mL) were added, and the mixture was heated to 80°C and stirred overnight. After the reaction was completed, the solvent was evaporated under reduced pressure to obtain a crude product, which was purified by silica gel column chromatography [eluent: dichloromethane-methanol (100:1-10:1)] and preparative HPLC to obtain a white solid (94) (60 mg, yield: 20.3%). MS-ESI m/z: 410.3 [M+H]⁺. ¹H NMR (400 MHz, DMSO-*d*₆) δ: 11.91 (s, 1H), 8.42-8.37 (m, 1H), 8.27 (d, J = 2.8 Hz, 1H), 7.84 (d, J = 8.8 Hz, 1H), 7.69 (d, J = 8.4 Hz, 1H), 7.41-7.36 (m, 2H), 7.26 (dd, J = 9.6, 6.8 Hz, 1H), 3.62 (s, 2H), 3.38-3.33 (m, 4H), 2.78 (d, J = 4.8 Hz, 3H), 2.56-2.53 (m, 4H), 2.02 (d, J = 2.4 Hz, 3H).

### Example 95: preparation of N-(methyl-d₃)-5-(4-((8-methyl-6-oxo-7-(trifluoromethyl)-5,6-dihydro-1,5-naphthyridin-3-yl)methyl)piperazin-1-yl)picolinamide ((compound (95))

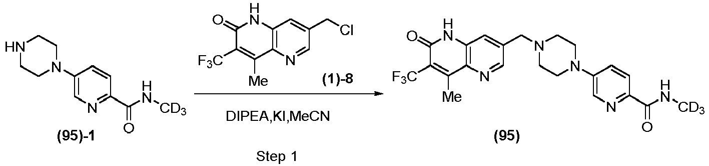

### Step 1. preparation of compound (95)

Using N-(methyl-*d₃*)-5-(piperazin-1-yl)picolinamide **((95)-1)** as the starting material, **compound (95)** was prepared according to a method similar to step 8 in the synthetic route of **compound (1).** MS-ESI m/z: 464.2 [M+H]⁺. ¹H NMR (400 MHz, DMSO-*d*₆) δ 12.23 (s, 1H), 8.58 (d, J = 2.0 Hz, 1H), 8.36 (s, 1H), 8.27 (d, J = 2.8 Hz, 1H), 7.83 (d, J = 8.8 Hz, 1H), 7.69 (d, J = 2.0 Hz, 1H), 7.39 (dd, J = 8.8, 2.9 Hz, 1H), 3.72 (s, 2H), 3.38 - 3.26 (m, 6H), 2.83 - 2.72 (m, 2H), 2.57- 2.52 (m, 3H).

### Example 96: preparation of N-(2-hydroxyethyl)-5-(4-((8-methyl-6-oxo-7-(trifluoromethyl)-5,6-dihydro-1,5-naphthyridin-3-yl)methyl)piperazin-1-yl)picolinamide ((compound (96))

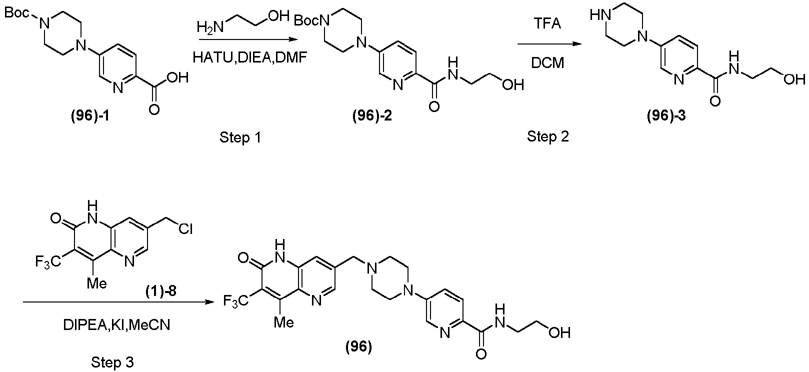

### Step 1. preparation of compound (96)-2

Compound **((96)-1)** (100 mg) was dissolved in DMF (2 mL), followed by the addition of ethanolamine (38 mg), HATU (237 mg), and diisopropylethylamine (201 mg). The reaction was allowed to proceed at room temperature for 2 hours. After the reaction was completed, dichloromethane and water were added for phase separation. The aqueous phase was extracted three times with dichloromethane. The combined organic phases were dried over anhydrous sodium sulfate, filtered, and concentrated. The residue was purified on a preparative chromatographic plate [eluent: petroleum ether-ethyl acetate = 1:2], to obtain a white solid **((96)-2)** (100 mg, yield: 88%). MS-ESI m/z: 351.2 [M+H]⁺.

### Steps 2-3. preparation of compound (96)

Using 5-(4-(*tert*-butoxycarbonyl)piperazin-1-yl)picolinic acid **((96)-2)** as the starting material, **compound (96)** was prepared according to a method similar to steps 3-4 in the synthetic route of **compound (21).** MS-ESI m/z: 491.2 [M+H]⁺. ¹H NMR (400 MHz, DMSO-*d*₆) δ 12.23 (s, 1H), 8.58 (d, J = 1.6 Hz, 1H), 8.34 (t, J = 6.0 Hz, 1H), 8.29 (d, J = 2.8 Hz, 1H), 7.84 (d, J = 8.8 Hz, 1H), 7.69 (d, J = 1.6 Hz, 1H), 7.40 (dd, J = 8.8, 2.8 Hz, 1H), 4.78 (t, J = 5.2 Hz, 1H), 3.72 (s, 2H), 3.56 - 3.45 (m, 2H), 3.39 - 3.26 (m, 6H), 2.85 - 2.74 (m, 3H), 2.58 - 2.55 (m, 3H).

### Example 97: preparation of 5-(4-((8-methyl-6-oxo-7-(trifluoromethyl)-5,6-dihydro-1,5-naphthyridin-3-yl)methyl)piperazin-1-yl)-N-(2,2,2-trifluoroethyl)picolinamide ((compound (97))

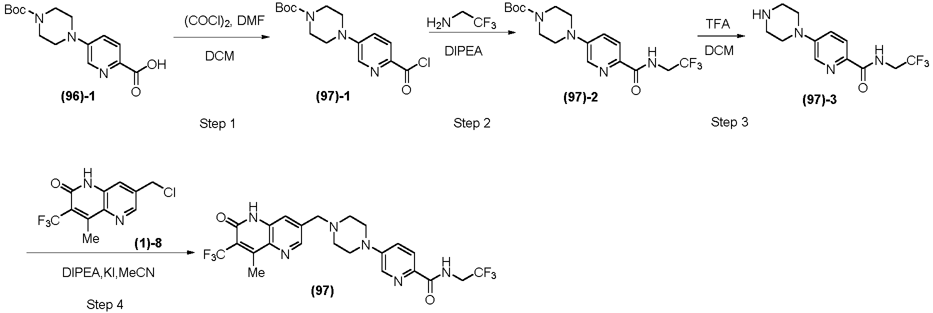

### Step 1. preparation of compound (97)-1

Compound **((96)-1)** (400 mg) was dissolved in dichloromethane (4 mL). DMF (20 mg) and oxalyl chloride (330 mg) were added dropwise under an ice bath, and the reaction was allowed to proceed at room temperature for 20 minutes. The mixture was then concentrated to obtain the crude product ((97)-1), which was used directly in the next step.

### Step 2. preparation of compound (97)-2

The crude product **((97)-1)** (200 mg) was dissolved in acetonitrile (4 mL), and DIPEA (238 mg) was added. Trifluoroethylamine (121.6 mg) was added dropwise slowly under an ice-water bath, and the mixture was stirred at room temperature for 20 minutes. The reaction was quenched with water, and the mixture was extracted three times with dichloromethane. The organic phases were combined, dried over anhydrous sodium sulfate, filtered, and concentrated. The residue was purified by silica gel column chromatography [eluent: dichloromethane-methanol = 15:1] to obtain a white solid ((97)-2) (30 mg). MS-ESI m/z: 389.2 [M+H]⁺.

### Steps 3-4. preparation of compound (97)

Using **(97)-2** as the starting material, **compound (97)** was prepared according to a method similar to steps 2-3 in the synthetic route of **compound (96).** MS-ESI m/z: 529.2 [M+H]⁺. ¹H NMR (400 MHz, DMSO-*d₆*) δ 12.24 (s, 1H), 8.92 (t, J = 6.8 Hz, 1H), 8.58 (d, J = 1.6 Hz, 1H), 8.33 (d, J = 3.2 Hz, 1H), 7.88 (d, J = 8.8 Hz, 1H), 7.72 - 7.67 (m, 1H), 7.43 (dd, J = 8.8, 3.2 Hz, 1H), 4.16 - 3.96 (m, 2H), 3.73 (s, 2H), 3.44 - 3.36 (m, 3H), 3.31 (s, 1H), 2.83 - 2.74 (m, 4H), 2.63 - 2.55 (m, 3H).

### Example 98: preparation of N-ethyl-5-(4-((8-methyl-6-oxo-7-(trifluoromethyl)-5,6-dihydro-1,5-naphthyridin-3-yl)methyl)piperazin-1-yl)picolinamide ((compound (98))

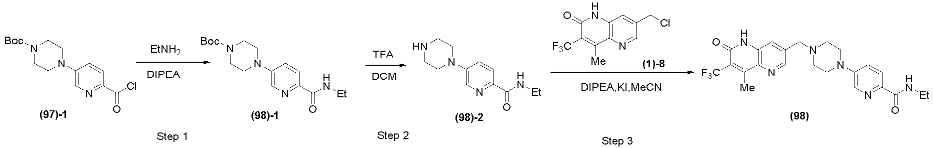

### Steps 1-3. preparation of compound (98)

Using **((97)-1)** and EtNH₂ as the starting materials, **compound (98)** was prepared according to a method similar to steps 2-4 in the synthetic route of **compound (97).** MS-ESI m/z: 475.2 [M+H]⁺. ¹H NMR (400 MHz, DMSO-*d*₆) δ 12.23 (s, 1H), 8.58 (d, J = 1.8 Hz, 1H), 8.42 (t, J = 6.0 Hz, 1H), 8.27 (d, J = 2.8 Hz, 1H), 7.83 (d, J = 8.7 Hz, 1H), 7.70 (s, 1H), 7.40 (dd, J = 9.0, 2.8 Hz, 1H), 3.72 (s, 2H), 3.31 - 3.2 (m, 8H), 2.87 - 2.73 (m, 2H), 2.60- 2.55 (m, 3H), 1.25 - 1.08 (m, 3H).

### Example 99: preparation of N-cyclopropyl-5-(4-((8-methyl-6-oxo-7-(trifluoromethyl)-5,6-dihydro-1,5-naphthyridin-3-yl)methyl)piperazin-1-yl)picolinamide ((compound (99))

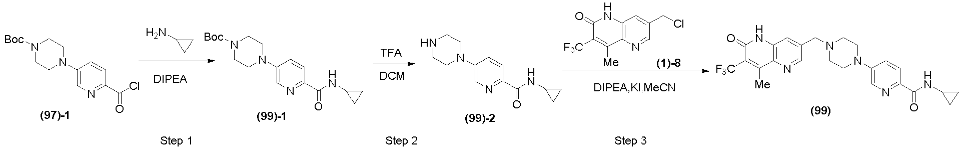

### Steps 1-3. preparation of compound (99)

Using **((97)-1)** as the starting material, **compound (99)** was prepared according to a method similar to steps 2-4 in the synthetic route of **compound (97).** MS-ESI m/z: 487.2 [M+H]⁺. ¹H NMR (400 MHz, DMSO-*d₆*) δ 12.23 (s, 1H), 8.58 (d, J = 1.8 Hz, 1H), 8.42 (t, J = 6.0 Hz, 1H), 8.27 (d, J = 2.8 Hz, 1H), 7.83 (d, J = 8.7 Hz, 1H), 7.70 (s, 1H), 7.40 (dd, J = 9.0, 2.8 Hz, 1H), 3.72 (s, 1H), 3.31 - 3.2 (m, 9H), 2.87 - 2.73 (m, 1H), 2.60- 2.55 (m, 3H), 1.25 - 1.08 (m, 4H).

### Example 100: preparation of 5-(4-((7-(difluoromethyl)-8-methyl-6-oxo-5,6-dihydro-1,5-naphthyridin-3-yl)methyl)piperazin-1-yl)-N-methylpicolinamide ((compound (100))

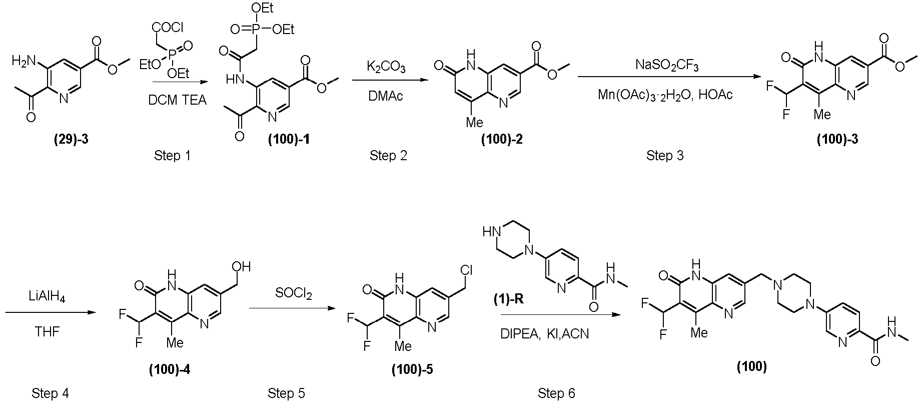

### Step 1. preparation of compound ((100)-1)

Compound **((29)-3)** (1 g) was dissolved in dichloromethane (40 mL), and diethyl (2-chloro-2-oxoethyl)phosphonate (3.3 g) was added at 0°C. Triethylamine (2.61 g) was then slowly added dropwise, and the mixture was allowed to return to room temperature. After the reaction was completed, the reaction was quenched with water and extracted with dichloromethane. The organic phase was washed with saturated brine, dried over anhydrous sodium sulfate, filtered, and concentrated. The crude product was purified by silica gel column chromatography [eluent: EA/PE = (10%-40%)]. The eluate was collected and the solvent was evaporated under reduced pressure to obtain a deep yellow liquid **((100)-1)** (860 mg, yield 44.8 %). MS-ESI m/z: 373.2 [M+H]⁺.

### Step 2. preparation of compound ((100)-2)

Compound **((100)-1)** (410 mg) and potassium carbonate (760.9 mg) were dissolved in N,N-dimethylacetamide (15 mL) and reacted at 80°C for 1 hour. After the reaction was completed, the mixture was concentrated and purified by silica gel column chromatography [eluent: DCM/MeOH = 20:1]. The eluate was collected and the solvent was evaporated under reduced pressure to obtain a white solid **((100)-2)** (150 mg, yield:62.5%). MS-ESI m/z: 219.2 [M+H]⁺.

### Step 3. preparation of compound ((100)-3)

Compound **((100)-2)** (50 mg), sodium trifluoromethanesulfinate (63.3 mg), and Mn(OAc)₃·2H₂O (184.3 mg) were dissolved in acetic acid (2 mL) and reacted at 50°C for 15 minutes. After the reaction was completed, the reaction solution was concentrated, diluted with water, and extracted twice with ethyl acetate. The organic phase was concentrated, and purified by silica gel plate chromatography [eluent: PE/EA = 1:1] to obtain a brown solid **((100)-3)** (16 mg, yield 26.2%). MS-ESI m/z: 269.2 [M+H]⁺.

### Steps 4-6. preparation of compound (100)

Using **((100)-3)** as the starting material, **compound (100)** was prepared according to a method similar to steps 6-8 in the synthetic route of **compound (1).** MS-ESI m/z: 443.2 [M+H]⁺. ¹H NMR (400 MHz, DMSO-*d*₆) δ 12.10 (s, 1H), 8.55 (s, 1H), 8.42 - 8.37 (m, 1H), 8.27 (d, J = 2.9 Hz, 1H), 7.83 (d, J = 8.7 Hz, 1H), 7.69 (s, 1H), 7.47 - 7.33 (m, 1H), 3.75 - 3.65 (m, 3H), 2.81- 2.75 (m, 4H), 2.72- 2.65 (m, 4H), 2.61 - 2.54 (m, 3H), 2.49 - 2.45 (m, 3H).

### Example 101: preparation of 5-(4-((4-fluoro-8-methyl-6-oxo-7-(trifluoromethyl)-5,6-dihydro-1,5-naphthyridin-3-yl)methyl)piperazin-1-yl)-N-methylpicolinamide ((compound (101))

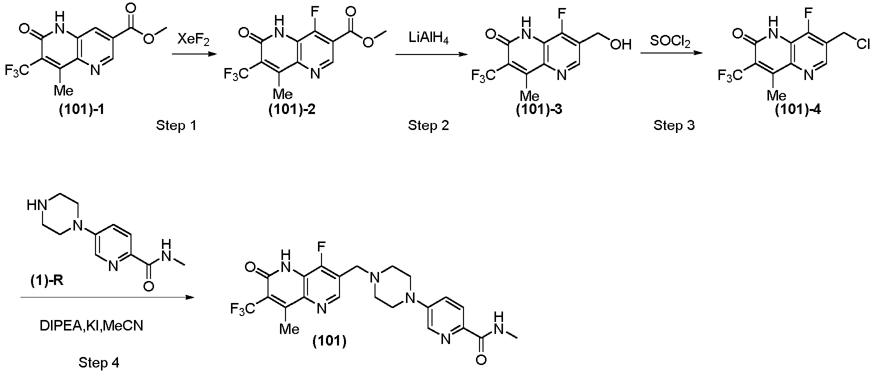

### Step 1. preparation of compound ((101)-2)

Compound **((101)-1)** (200 mg) was suspended in dichloromethane (6 mL). Under ice bath conditions, boron trifluoride etherate (990 mg) and xenon difluoride (233.6 mg) were added, and the reaction was allowed to proceed overnight at room temperature. After the reaction was completed, saturated sodium bicarbonate solution was added to adjust the pH to ~7. The mixture was extracted with dichloromethane, and the organic phases were combined, dried, and concentrated. The residue was purified by silica gel column chromatography [eluent: DCM/MeOH = 20:1]. The eluate was collected and the solvent was evaporated under reduced pressure to obtain a pale yellow solid **((101)-2)** (90 mg, yield: 42%). MS-ESI m/z: 305.2 [M+H]⁺

### Steps 2-4. preparation of compound (101)

Using **((100)-2)** as the starting material, **compound (101)** was prepared according to a method similar to steps 6-8 in the synthetic route of **compound (1).** MS-ESI m/z: 479.4 [M+H]⁺. ¹H NMR (400 MHz, DMSO-*d*₆) δ 12.60 - 12.04 (m, 1H), 8.41 (d, J = 4.9 Hz, 1H), 8.29 (d, J = 2.8 Hz, 1H), 7.98 (d, J = 8.4 Hz, 1H), 7.84 (d, J = 8.8 Hz, 1H), 7.41 (dd, J = 8.9, 2.9 Hz, 1H), 3.71 (s, 2H), 3.37 (s, 4H), 2.78 (d, J = 4.9 Hz, 3H), 2.69 - 2.60 (m, 7H).

### Example 102: preparation of 5-(4-((4-fluoro-2-oxo-3-(trifluoromethyl)-1,2-dihydroquinolin-7-yl)methyl)piperazin-1-yl)-N-methylpicolinamide ((compound (102))

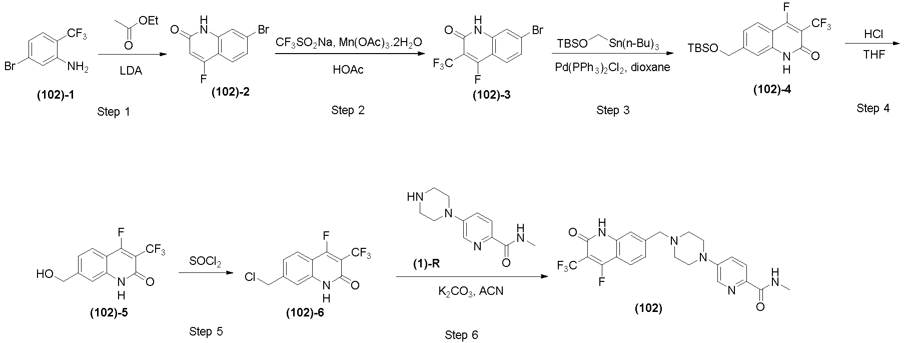

### Step 1. preparation of compound ((102)-2)

In a three-neck flask, ethyl acetate (3.52 g) and ultradry tetrahydrofuran (150 mL) were added and cooled to -78°C under nitrogen protection. Lithium diisopropylamide (2 M, 30.0 mL) was added dropwise to the reaction system, followed by the slow dropwise addition of a tetrahydrofuran solution of **((102)-1)** (2.39 g, 40 mL). The mixture was slowly warmed to room temperature and reacted overnight. The reaction was quenched with saturated aqueous solution of ammonium chloride, filtered, and extracted with ethyl acetate. The solvent was evaporated under reduced pressure to obtain the crude product, which was purified by column chromatography [eluent: petroleum ether-ethyl acetate = 20:1] to obtain a yellow solid **((102)-2)** (570 mg, yield:23.7%). MS-ESI m/z: 242.2 [M+H]⁺.

### Step 2. preparation of compound ((102)-3)

In a reaction flask, **((102)-2)** (492 mg), sodium trifluoromethanesulfinate (955 mg), and acetic acid (10 mL) were added, followed by the slow addition of Mn(OAc)₃·2H₂O (2.19 g). The mixture was stirred at room temperature overnight. The solvent was evaporated under reduced pressure, and the mixture was extracted with ethyl acetate. The organic phases were combined and dried to obtain the crude product, which was purified by silica gel column chromatography [eluent: petroleum ether-ethyl acetate = 20:1]. The eluate was collected and the solvent was evaporated under reduced pressure to obtain a white solid **((102)-3)** (140 mg, yield: 22.2%) MS-ESI m/z: 310.0 [M+H]⁺.

### Step 3. preparation of compound ((102)-4)

In a reaction flask, **((102)-3)** (119 mg, 0.39 mmol), bis(triphenylphosphine)palladium(II) dichloride (27 mg), tert-butyldimethyl[(tributylstannyl)methoxy]silane (335 mg), and 1,4-dioxane (5 mL) were added. The mixture was heated to 100°C under nitrogen protection and stirred overnight. After the reaction was completed, the mixture was cooled to room temperature, and 10% KF aqueous solution was slowly added dropwise to quench the reaction. The precipitated solid was filtered, and the filtrate was extracted with ethyl acetate. The organic phases were combined, dried over anhydrous sodium sulfate, and the solvent was evaporated under reduced pressure to obtain the crude product, which was purified by silica gel column chromatography [eluent: petroleum ether-ethyl acetate = 30:1]. The eluate was collected and the solvent was evaporated under reduced pressure to obtain a yellow solid **((102)-4)** (90 mg, yield: 62.5%) MS-ESI m/z: 376.1 [M+H]⁺.

### Step 4. preparation of compound ((102)-5)

In a reaction flask, **((102)-4)** (90 mg), tetrahydrofuran (3 mL), and an aqueous solution of hydrochloric acid a (2M, 1 mL) were added and stirred at room temperature for 1 hour. After the reaction was completed, a saturated sodium bicarbonate solution was added, and the mixture was extracted with dichloromethane. The organic phases were combined, dried over anhydrous sodium sulfate, and the solvent was evaporated under reduced pressure to obtain the crude product, which was purified by silica gel column chromatography [eluent: dichloromethane-methanol = 100:1]. The eluate was collected and the solvent was evaporated under reduced pressure to obtain a pale yellow solid **((102)-5)** (51 mg, yield: 81.5%). MS-ESI m/z: 262.1 [M+H]⁺.

### Steps 5-6. preparation of compound (102)

Using **((102)-5)** as the starting material, **compound (102)** was prepared according to a method similar to steps 7-8 in the synthetic route of **compound (1).** MS-ESI m/z: 464.2 [M+H]⁺. ¹H NMR (400 MHz, DMSO-*d*₆) δ: 12.75 (s, 1H), 8.50-8.45 (m, 1H), 8.34 (d, J = 3.2 Hz, 1H), 8.03 (d, J = 8.4 Hz, 1H), 7.90 (d, J = 8.8 Hz, 1H), 7.70 (d, J = 8.4 Hz, 1H), 7.55 (s, 1H), 7.50 (dd, J = 11.6, 6.0 Hz, 1H), 4.53 (s, 1H), 4.13-4.02 (m, 2H), 3.43-3.31 (m, 4H), 3.25-3.16 (m, 3H), 2.80 (d, J = 5.2 Hz, 3H).

### Example 103: preparation of 5-(4-((8-fluoro-7-methyl-6-oxo-5,6-dihydro-1,5-naphthyridin-3-yl)methyl)piperazin-1-yl)-N-methylpicolinamide ((compound (103))

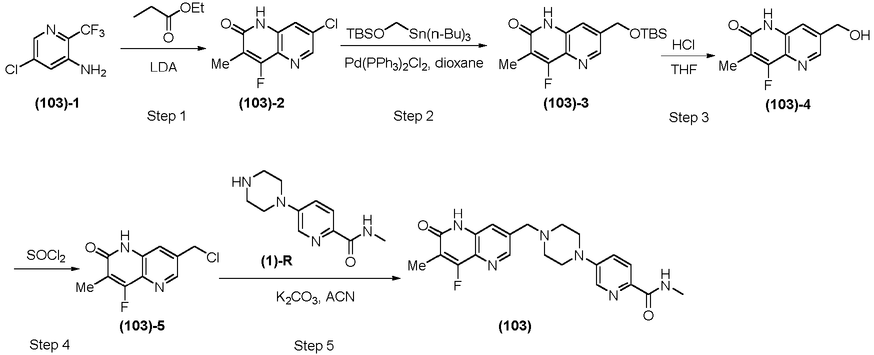

### Steps 1-5. preparation of compound (103)

Using 5-chloro-2-(trifluoromethyl)pyridin-3-amine **((103)-1)** as the starting material, **compound (103)** was prepared according to a method similar to step 1 and steps 3-6 in the synthetic route of **compound (102).** MS-ESI m/z: 411.4 [M+H]⁺. ¹H NMR (400 MHz, DMSO-*d*₆) δ: 12.02 (s, 1H), 8.48 (d, J = 1.6 Hz, 1H), 8.41 (d, J = 4.8 Hz, 1H), 8.27 (d, J = 2.8 Hz, 1H), 7.84 (d, J = 8.8 Hz, 1H), 7.70 (s, 1H), 7.41 (dd, J = 12.0, 6.4 Hz, 1H), 3.69 (s, 2H), 3.44-3.34 (m, 4H), 2.79 (d, J = 4.8 Hz, 3H), 2.58-2.56 (m, 4H), 2.07 (d, J = 2.8, 3H).

### Example 104: preparation of (R)-5-(4-((7-(difluoromethyl)-8-methyl-6-oxo-5,6-dihydro-1,5-naphthyridin-3-yl)methyl)-3-methylpiperazin-1-yl)-N-methylpicolinamide ((compound (104))

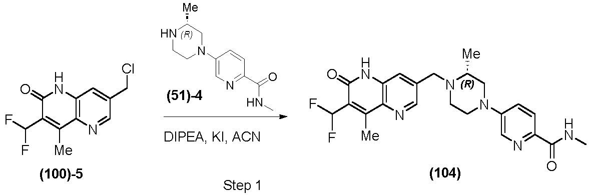

### Step 1. preparation of compound (104)

Using **((100)-5)** as the starting material, **compound (104)** was prepared according to a method similar to the synthetic route of **compound (1).** MS-ESI m/z: 457.2 [M+H]⁺. ¹H NMR (400 MHz, DMSO-*d*₆) δ 12.08 (s, 1H), 8.56 (d, J = 1.9 Hz, 1H), 8.36 (d, J = 5.0 Hz, 1H), 8.26 (d, J = 2.8 Hz, 1H), 7.82 (d, J = 8.7 Hz, 1H), 7.70 (d, J = 1.9 Hz, 1H), 7.43 - 7.34 (m, 1H), 4.13 (d, J = 14.5 Hz, 1H), 3.70 (d, J = 12.0 Hz, 1H), 3.59 (d, J = 12.3 Hz, 1H), 3.43 (d, J = 14.5 Hz, 2H), 3.06 - 2.87 (m, 2H), 2.78 (d, J = 4.9 Hz, 3H), 2.71 (s, 3H), 2.69 - 2.56 (m, 3H), 1.17 (d, J = 6.1 Hz, 3H).

### Example 105: preparation of (R)-5-(4-((7-(difluoromethyl)-8-methyl-6-oxo-5,6-dihydro-1,5-naphthyridin-3-yl)methyl)-2-methylpiperazin-1-yl)-N-methylpicolinamide ((compound (105))

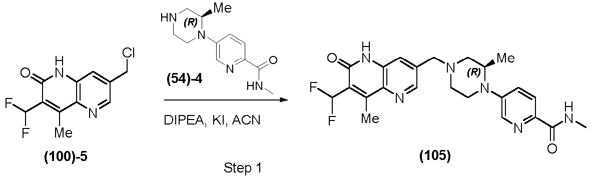

### Step 1. preparation of compound (105)

Using **((100)-5)** as the starting material, **compound (105)** was prepared according to a method similar to step 8 in the synthetic route of **compound (1).** MS-ESI m/z: 457.2 [M+H]⁺. ¹H NMR (400 MHz, DMSO-*d₆*) δ 12.19 (s, 1H), 8.56 (d, J = 1.8 Hz, 1H), 8.37 - 8.35 (m, 1H), 8.20 (d, J = 2.8 Hz, 1H), 7.82 (d, J = 8.8 Hz, 1H), 7.73 (d, J = 1.9 Hz, 1H), 7.33 (dd, J = 8.9, 2.9 Hz, 1H), 4.22 (d, J = 7.6 Hz, 1H), 3.75 (d, J = 14.4 Hz, 1H), 3.66 - 3.54 (m, 2H), 3.20 - 3.04 (m, 2H), 2.94 (d, J = 10.7 Hz, 1H), 2.78 (d, J = 4.8 Hz, 3H), 2.72 (d, J = 2.5 Hz, 3H), 2.40- 2.35 (m, 2H), 2.30 - 2.20 (m, 1H), 1.14 (d, J = 6.5 Hz, 3H).

### Example 106: preparation of 1'-((7-(difluoromethyl)-8-methyl-6-oxo-5,6-dihydro-1,5-naphthyridin-3-yl)methyl)-N-methyl-1',2',3',6'-tetrahydro-[3,4'-bipyridine]-6-carboxamide ((compound (106))

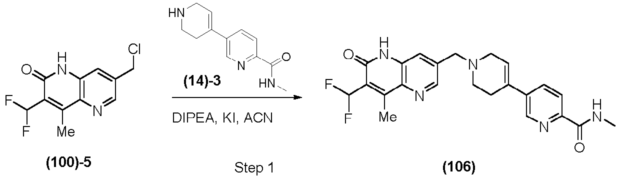

### Step 1. preparation of compound (106)

Using **((100)-5)** as the starting material, **compound (106)** was prepared according to a method similar to step 8 in the synthetic route of **compound (1).** MS-ESI m/z: 440.2 [M+H]⁺. ¹H NMR (400 MHz, DMSO-*d₆*) δ 12.13 (s, 1H), 8.70 - 8.67 (m, 2H), 8.56 (d, J = 1.9 Hz, 1H), 7.98 (dd, J = 3.4, 1.5 Hz, 2H), 7.70 (d, J = 1.9 Hz, 1H), 7.23 (m, 1H), 6.42 (s, 1H), 3.77 (s, 2H), 3.32-3.29 (m, 2H), 3.17 (dd, J = 5.8, 3.0 Hz, 2H), 2.81 (d, J = 4.9 Hz, 3H), 2.71 (s, 3H), 2.58-2.54 (m, 2H).

### Example 107: preparation of (R)-5-(4-((7,8-dimethyl-6-oxo-5,6-dihydro-1,5-naphthyridin-3-yl)methyl)-3-methylpiperazin-1-yl)-N-methylpicolinamide (compound (107))

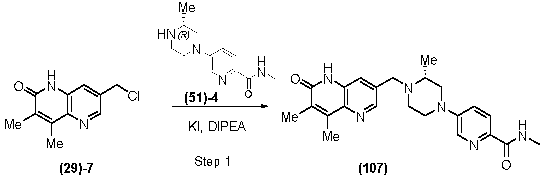

### Step 1: preparation of compound (107)

Using **(29)-7** and **(51)-4** as the starting materials, **compound (107)** was prepared according to a method similar to step 8 in the synthetic route of **compound (29).** MS-ESI m/z: 421.2 [M+H]⁺. ¹H NMR (400 MHz, DMSO-*d*₆) δ 11.70 (s, 1H), 8.44 (d, J = 2.0 Hz, 1H), 8.38 (q, J = 4.8 Hz, 1H), 8.26 (d, J = 2.8 Hz, 1H), 7.82 (d, J = 8.8 Hz, 1H), 7.62 (d, J = 2.0 Hz, 1H), 7.39 (dd, J = 8.8, 2.8 Hz, 1H), 4.10 (d, J = 14.4 Hz, 1H), 3.70 (d, J = 12.0 Hz, 1H), 3.59 (d, J = 12.0 Hz, 1H), 3.41 - 3.38 (m, 2H), 3.02 - 2.91 (m, 1H), 2.84 - 2.69 (m, 4H), 2.64 - 2.55 (m, 1H), 2.52 - 2.51 (m, 3H), 2.34 - 2.24 (m, 1H), 2.13 (s, 3H), 1.19 (d, J = 6.0 Hz, 3H).

### Example 108: preparation of (S)-5-(4-((7,8-dimethyl-6-oxo-5,6-dihydro-1,5-naphthyridin-3-yl)methyl)-3-methylpiperazin-1-yl)-N-methylpicolinamide (compound (108))

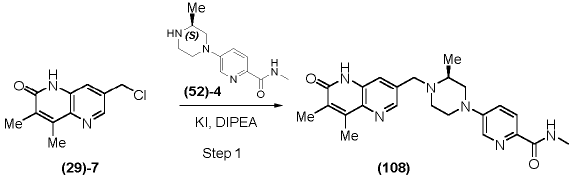

### Step 1: preparation of compound (108)

Using **(29)-7** and **(52)-4** as the starting materials, **compound (108)** was prepared according to a method similar to step 8 in the synthetic route of **compound (29).** MS-ESI m/z: 421.2 [M+H]⁺. ¹H NMR (400 MHz, DMSO-*d*₆) δ 11.70 (s, 1H), 8.44 (d, J = 2.0 Hz, 1H), 8.38 (q, J = 4.8 Hz, 1H), 8.26 (d, J = 2.8 Hz, 1H), 7.82 (d, J = 8.8 Hz, 1H), 7.62 (d, J = 2.0 Hz, 1H), 7.39 (dd, J = 8.8, 2.8 Hz, 1H), 4.10 (d, J = 14.4 Hz, 1H), 3.70 (d, J = 12.0 Hz, 1H), 3.59 (d, J = 12.0 Hz, 1H), 3.41 - 3.38 (m, 2H), 3.02 - 2.91 (m, 1H), 2.84 - 2.69 (m, 4H), 2.64 - 2.55 (m, 1H), 2.52 - 2.51 (m, 3H), 2.34 - 2.24 (m, 1H), 2.13 (s, 3H), 1.19 (d, J = 6.0 Hz, 3H).

### Biological Assay

### Experimental Example 1: PARP Enzyme Activity Assay and Trapping Assay

### 1. PARP Enzyme Activity Assay

**Experimental Materials:** The PARP1 assay kit was purchased from BPS Bioscience, USA, specifically the 384-well chemiluminescent assay kit (Catalog No.: #80551).

**Experimental Principle:** PARP can catalyze the transfer of ADP-ribose residues from NAD+ to target substrates, thereby modifying proteins through ribosylation. This experiment measures PARP1 enzyme activity by detecting the poly(ADP-ribosylation) of histones by PARP1.

**Preparation of Reagents and Test Compounds:** The 10x assay buffer included in the kit was diluted to 1x with distilled water. The test compound (stock concentration of 10 mM) was diluted to 10 µM with a DMSO solution and then serially diluted in a 1:3 ratio. The serially diluted compounds were further diluted 10-fold with the 1x assay buffer.

### Experimental Method: The procedure was carried out according to the kit's instructions:

**1.** The 5x histone mixture was diluted to 1x with PBS, and 25 µL was added to each well of a 384-well plate, incubating overnight at 4°C.
**2.** After incubation, 100 µL of PBST (0.05% Tween 20) was added to each well for washing, repeated three times. Then, 100 µL of blocking solution was added to each well and incubated at room temperature for 90 minutes.
**3.** After incubation, the wells were washed three times with PBST, and then 1.25 µL of 10x assay buffer, 1.25 µL of 10x PARP assay mixture, 2.5 µL of activation DNA (5x), and 7.5 µL of distilled water were added to each well.
**4.** Then, 2.5 µL of the diluted test compound was added to each well. The positive control group and the negative control group were added with 1x assay buffer containing the same concentration of DMSO.
**5.** Next, 10 µL of 2.5 ng/µL PARP1 enzyme was added to the test compound and positive control groups, while 10 µL of 1x assay buffer was added to the negative control group, and the mixture was incubated at room temperature for 60 minutes.
**6.** Streptavidin-HRP was diluted 1:50 with the blocking solution. After incubation, the plate was washed three times with PBST, and 25 µL of the diluted streptavidin-HRP solution was added to each well, incubating at room temperature for 30 minutes.
**7.** After incubation, the plate was washed three times with PBST, and 50 µL of the mixed substrate A and substrate B in equal volumes was added. The luminescence signal was immediately read on a Synergy HTX multi-mode reader.
**8.** Data analysis was performed using GraphPad Prism 8.0 software. The inhibition rate % = 100 * (average luminescence signal of the positive control group - luminescence signal of the compound) / (average luminescence signal of the positive control group - average luminescence signal of the negative control group). The IC₅₀ value was obtained by plotting the logarithm of the compound concentration against the percentage inhibition rate.

**Test Results:** The enzyme inhibitory effect of the compounds on PARP1 is shown in Table 1.

**Table 1: Enzyme Inhibitory Activity of Compounds on PARP1**

| **Test Compound** | **PARP-1 IC50 (nM)** |
|---|---|
| **1** | 2.78 |
| **2** | 3.14 |
| **3** | 1.53 |
| **5** | 27.18 |
| **6** | 34.32 |
| **8** | 2.55 |
| **10** | 2.79 |
| **11** | 0.96 |
| **12** | 2.25 |
| **13** | 7.37 |
| **14** | 0.83 |
| **15** | 3.48 |
| **18** | 15.91 |
| **19** | 5.48 |
| **20** | 1.26 |

### 2. PARP1/PARP2 Trapping Assay

**Experimental Materials and Reagents:**

| Reagent | Supplier | Catalog No. |
|---|---|---|
| PARP1 | BPS Bioscience | 80501 |
| PARP2 | BPS Bioscience | 80502 |
| NAD | Sigma | 10127965001 |
| PARP1 Probe | Generay | N/A |
| PARP2 Probe | Generay | N/A |
| GST-Tb Antibody | Cisbio | 61GSTTLA |

### Buffer Preparation:

10 mM potassium phosphate (pH=7.9), 50 mM sodium chloride, 1 mM ethylenediaminetetraacetic acid, 0.05% polyethylene glycol dodecyl ether, 1 mM dithiothreitol.

### Experimental Method:

**1.** A 4x enzyme reaction mixture, comprising PARP1, PARP2, and GST-Tb antibody, was prepared with the buffer and 4 µL of the enzyme reaction mixture was added to a 384-well plate.
**2.** A 4x PARP1 and PARP2 probe reaction mixture was prepared with the buffer and 4 µL of the probe reaction mixture was added to the 384-well plate.
**3.** A series of gradient-diluted compounds were prepared and 4 µL of the diluted compounds were added to the 384-well plate, and incubated at room temperature for 45 minutes.
**4.** After incubation, 4 µL of 4x NAD reaction mixture was added to the 384-well plate and incubated at room temperature for 10 minutes.
**5.** After incubation, the plate was read using a plate reader.
**6.** Data Analysis: The percentage inhibition rate was calculated as follows: Inhibition Rate (%) = (Reading of Compound Group - Reading of Blank Group) / (Reading of Vehicle Control Group - Reading of Blank Group).

**Test Results:** The inhibitory effects of the compounds on PARP1 and PARP2 trapping are shown in Tables 2-1 and 2-2, respectively.

**Table 2-1: Inhibitory Effects of Compounds on PARP1 Trapping**

| **Test Compound** | **Inhibitory Effect on PARP1 Trapping IC₅₀ (nM)** |
|---|---|
| **1** | 1.33 |
| **2** | 1.22 |
| **14** | 1.38 |
| **52** | 1.60 |
| **54** | 1.31 |
| **87** | 2.57 |
| **88** | 1.55 |
| **89** | 1.53 |
| **90** | 4.33 |
| **91** | 2.90 |
| **93** | 1.32 |
| **99** | 1.53 |
| **100** | 2.58 |

**Table 2-2: Inhibitor Effects of Compounds on PARP2 Trapping**

| **Test Compound** | **Inhibitory Effect on PARP2 Trapping IC₅₀ (nM)** |
|---|---|
| **1** | 164.4 |
| **2** | 137.1 |
| **14** | 176.1 |
| **52** | 270.5 |
| **54** | 192.3 |

**Experimental Conclusion:** The Example compounds in the present application exhibit significant inhibitory effects on PARP1 enzyme activity. In the trapping assays for PARP1 and PARP2, the Example compounds show a clear selectivity for inhibiting PARP1 function over PARP2.

### Experimental Example 2: Cell Proliferation Assay

**Experimental Materials:** MDA-MB-436 or UWB1.289 cell lines were purchased from Cobioer Biosciences Co. Ltd. (Nanjing, China). The STR identification results were a complete match, and mycoplasma testing was negative. MDA-MB-436 cells were cultured in DMEM complete medium containing 10% fetal bovine serum, 1% penicillin-streptomycin, 1% insulin, and 16 µg/mL glutathione; UWB1.289 cells were cultured in RMPI1640 complete medium containing 10% fetal bovine serum and 1% penicillin-streptomycin.

### Experimental Method:

**1.** MDA-MB-436 or UWB1.289 cells were cultured in the aforementioned medium (culture conditions: 37°C, 5.0% CO₂ in air) until they reached the logarithmic growth phase. The cells were then digested with trypsin containing 0.25% EDTA, centrifuged at 1000 rpm, and the supernatant was discarded. Cell culture medium was added, and the cell suspension was adjusted to 2 × 10⁴/mL after counting. 135 µL of the cell suspension per well was taken and cultured in a 96-well cell culture plate for 16 hours (culture conditions: 37°C, 5.0% CO₂ in air).
**2.** The test compounds, stored at a concentration of 10 mM, were diluted 2.5 times with DMSO and then serially diluted in a 1:3 gradient (9 gradients). The gradient-diluted samples were further diluted 40 times with serum-free medium, and 15 µL of each was added to the 96-well cell culture plate. The positive control group received serum-free medium containing the same concentration of DMSO, and the negative control group received medium without cells. The cells were then cultured for an additional 7 days.
**3.** After the culture period, the cell culture plate was placed at room temperature for 30 minutes. Then, 100 µL of CellCounting-Lite 2.0 reagent (Vazyme, #DD1101) was added to each well, mixed thoroughly, and incubated in the dark at room temperature for 10 minutes. The luminescence signal was read using a Synergy HTX multi-mode reader.
**4.** Data analysis was performed using GraphPad Prism 8.0 software. The inhibition rate (%) was calculated as 100 * (average luminescence signal of the positive control group - luminescence signal of the compound)/(average luminescence signal of the positive control group - average luminescence signal of the negative control group). The IC₅₀ value was obtained by plotting the logarithm of the compound concentration against the percentage inhibition rate.

**Test Results:** The inhibitory activity of the compounds on the proliferation of UWB1.289 ovarian cancer cells is shown in Table 3.

**Table 3: Inhibitory Activity on Cell Proliferation**

| **Test Compound** | **UWB1.289 Cell IC₅₀ (nM)** |
|---|---|
| **1** | 0.66 |
| **2** | 2.76 |
| **8** | 12.62 |
| **10** | 14.98 |
| **11** | 28.50 |
| **14** | 4.93 |
| **20** | 26.58 |
| **52** | 11.17 |
| **53** | 13.62 |
| **54** | 3.34 |
| **71** | 8.13 |
| **93** | 2.67 |

The inhibitory activity of the compounds on the proliferation of MDA-MB-436 breast cancer cells is shown in Table 4.

**Table 4: Inhibitory Activity on Cell Proliferation**

| **Test Compound** | **MDA-MB-436 Cell IC₅₀ (nM)** |
|---|---|
| **1** | 1.59 |
| **2** | 2.32 |
| **14** | 2.38 |
| **24** | 4.32 |
| **52** | 5.05 |
| **54** | 4.32 |
| **72** | 8.18 |
| **73** | 14.52 |
| **79** | 18.53 |
| **80** | 13.91 |
| **81** | 13.16 |
| **82** | 5.83 |
| **85** | 16.45 |
| **86** | 14.83 |
| **87** | 5.73 |
| **88** | 5.75 |
| **89** | 1.06 |
| **90** | 7.26 |
| **91** | 3.24 |
| **93** | 0.83 |
| **94** | 5.69 |
| **95** | 2.13 |
| **96** | 9.34 |
| **97** | 2.12 |
| **98** | 1.22 |
| **99** | 1.69 |
| **100** | 2.05 |
| **101** | 8.99 |
| **103** | 3.76 |
| **104** | 6.50 |
| **105** | 4.93 |
| **106** | 0.78 |

**Experimental Conclusion:** The compounds in the examples of the present application exhibit strong inhibitory activity on the proliferation of UWB1.289 ovarian cancer cells and MDA-MB-436 breast cancer cells.

### Experimental Example 3: Liver Microsome Metabolism Assay

**Experimental Materials:** Mixed CD1 mouse liver microsomal enzyme protein was purchased from Xenotech (#M1000), and NADPH was purchased from Abmole (#M9076).

### Experimental Method:

**1.** To the liver microsomal enzyme reaction system, 210 µL of phosphate buffer was added, followed by 12.5 µL of liver microsomal enzyme at a concentration of 20 mg/mL. Then, 25 µL of NADPH at a concentration of 10 mM was added to the system, mixed well, and incubated in a 37°C water bath with shaking for 10 minutes.
**2.** The test compound, stored at a concentration of 10 mM, was diluted to 100 µM with DMSO. Then, 2.5 µL of the diluted test compound was added to the reaction system, mixed well, and incubated in a 37°C water bath with shaking.
**3.** At 0.5, 5, 10, 20, 30, and 60 minutes, 25 µL of the enzyme reaction mixture was taken and added to 125 µL of cold acetonitrile containing dexamethasone as an internal standard. The mixture was centrifuged at 4000 g for 20 minutes, and the supernatant was mixed with an equal volume of distilled water. The mixed sample was then subjected to quantitative analysis by liquid chromatography-mass spectrometry.
**4.** Calculation of *in vitro* drug metabolism half-life and drug clearance rate:
   t_{1/2} = -0.693/k. (k: the slope of the linear regression of the natural logarithm of the percentage of drug remaining versus incubation time).
   *In vitro* drug clearance rate = (0.693/t_{1/2}) * (enzyme reaction volume / liver microsomal enzyme content).

**Test Results:** See Table 5.

**Table 5: Mouse Liver Microsomal Enzyme Clearance Rate Data**

| **Test Compound** | **CLint (in mouse liver microsomal) (uL/min/mg protein)** |
|---|---|
| **AZD5305*** | 1.23 |
| **1** | 0.32 |
| **2** | 0.39 |
| **15** | 0.75 |
| **24** | 0.40 |
| **72** | 0.60 |
| **79** | 0.69 |
| **84** | 0.72 |
| **85** | 0.86 |
| **87** | 1.01 |
| **88** | 0.84 |
| **89** | 0.81 |
| **90** | 0.21 |
| **91** | 0.30 |

| | |
|---|---|
| AZD5305 (see Jeffrey W Johannes et al., J. Med. Chem., 2021, 64 14498-14612 was used as a reference compound, and has the following structure 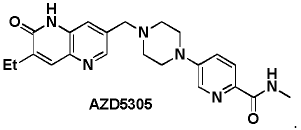 | |

The test results show that the example compounds of the present application exhibit good pharmacokinetic properties.

### Experimental Example 4: Evaluation of Pharmacokinetics in Mice

**Test Objective:** The pharmacokinetics of the example compounds of the present application were tested in mice. By measuring the drug concentration in mouse plasma, the pharmacokinetic behavior of the compounds of the present invention was studied to evaluate their pharmacokinetic characteristics.

### Test Animals: 6-8 week old male ICR (CD1) mice

### Experimental Method:

Oral Gavage Drug Preparation: An appropriate amount of the test compound was first weighed and dissolved in DMSO (Macklin cas: 67-68-5) to prepare a 20 mg/mL solution. Then, 75 µL of this solution was added to 150 µL of Solutol (Sigma cas: 70142-34-6), followed by 1.275 mL of 20% Captisol (Selleck cas: 182410-00-0), resulting in a final drug concentration of 1 mg/mL.

Intravenous Injection Drug Preparation: An appropriate amount of the test compound was first weighed and dissolved in DMSO (Macklin cas: 67-68-5) to prepare a 30 mg/mL solution. Then, 15 µL of this solution was added to 150 µL of Solutol (Sigma cas: 70142-34-6), followed by 1.335 mL of 20% Captisol (Selleck cas: 182410-00-0), resulting in a final drug concentration of 0.3 mg/mL.

Procedures: The mice were fasted overnight with free access to water before the experiment. Food was restored two hours after dosing. Blood samples were collected at different time points after intravenous injection or oral gavage of the test compound in mice, and the compound concentration in plasma was measured. Approximately 50 µL of blood was collected from each animal using a capillary sampling tube via the orbital venous plexus, with heparin sodium as an anticoagulant. Blood samples were placed on ice, centrifuged at 3500 r/min for 10 minutes to separate the plasma, and 20 µL of the collected plasma was added to 200 µL of acetonitrile (Merck cas: 75-05-8) containing 200 nM dexamethasone (Selleck cas: 50-02-2) as an internal standard. The mixture was centrifuged at 8000 r/min for 20 minutes. After centrifugation, 150 µL of the supernatant was transferred to a new centrifuge tube, and 150 µL of 0.1% formic acid (Fisher cas: 207868) was added. The mixture was mixed well, and 5 µL of the sample was subjected to quantitative analysis by liquid chromatography-mass spectrometry (Q-TOF LC/MS).

Standard Curve Determination:
**1.** The test compound was serially diluted with DMSO to cover the compound concentrations expected in plasma samples to be tested, with a blank sample (containing only DMSO) included.
**2.** 2 µL of each diluted sample at various concentrations was added to 18 µL of normal ICR mouse plasma, mixed, and then added to 200 µL of acetonitrile (Merck cas: 75-05-8) containing 200 nM dexamethasone (Selleck cas: 50-02-2) as an internal standard. The mixture was centrifuged for 20 minutes (8000 r/min). 150 µL of the supernatant was transferred to a new centrifuge tube, and 150 µL of 0.1% formic acid (Fisher cas: 207868) was added. The mixture was mixed well, and 5 µL of the sample was subjected to quantitative analysis by liquid chromatography-mass spectrometry (Q-TOF LC/MS).
**3.** The final standard curve was plotted using the concentration of the test compound after dilution on the abscissa and the ratio of the signal of the compound to the internal standard (dexamethasone) on the ordinate. The standard curve was generated using linear regression with GraphPad Prism 8 software (R2 > 0.9900).

Data Analysis: calculation of pharmacokinetic parameters: the concentration of the test compound in plasma at different time points after intravenous injection or oral gavage in ICR mice was determined based on the above standard curve, and pharmacokinetic parameters (T_{1/2}, Cmax, AUC, *etc.)* were calculated using the Phoenix WinNonlin 8.1 non-compartmental analysis model.

**Test Results:** See Table 6.

**Table 6: Pharmacokinetic Data of Mice after Oral Gavage (PO, 10 mpk)**

| **Test Compound** | **AUC_{0-∞} (ng·hr/mL)** | **Cmax (ng/mL)** | **t_{1/2} (hr)** |
|---|---|---|---|
| **AZD5305*** | 356,592 | 37,283 | 8.25 |
| **1** | 972,211 | 39,635 | 17.9 |
| **11** | 1,497,448 | 55,884 | 18.9 |
| **14** | 2,716,871 | 127,992 | 27.8 |
| **24** | 1,100,248 | 26,344 | 28.69 |
| **52** | 1,548,502 | 50,212 | 25.91 |
| **54** | 987,363 | 48,164 | 17.9 |
| **79** | 2,421,942 | 56,956 | 30.5 |
| **88** | 1,649,634 | 38,163 | 26.32 |
| **90** | 1,362,843 | 49,892 | 20.24 |
| **93** | 795,715 | 31,876 | 18.37 |
| **95** | 2,048,283 | 37,508 | 37.95 |

| | | | |
|---|---|---|---|
| *AZD5305 (see Jeffrey W Johannes et al., J. Med. Chem., 2021, 64, 14498-14612 was used as a reference compound, and has the following structure 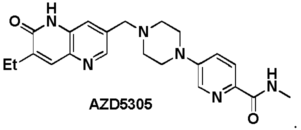 | | | |

**Experimental Conclusion:** The exposure and half-life of the example compounds of the present application in mice were significantly better than those of the reference compound AZD5305.

### Experimental Example 5: Evaluation of Pharmacokinetics in Rats

**Test Objective:** The pharmacokinetics of the example compounds of the present application were tested in rats. By measuring the drug concentration in rat plasma, the pharmacokinetic behavior of the compounds of the present invention was studied to evaluate their pharmacokinetic characteristics.

### Test Animals: 6-8 week old male SD rats

### Experimental Method:

Oral Gavage Drug Preparation: An appropriate amount of the test compound was first weighed and dissolved in DMSO (Macklin cas: 67-68-5) to prepare a 100 mg/mL solution. Then, 100 µL of this solution was added to 200 µL of Solutol (Sigma cas: 70142-34-6), followed by 1.7 mL of 20% Captisol (Selleck cas: 182410-00-0), resulting in a final drug concentration of 5 mg/mL.

Intravenous Injection Drug Preparation: An appropriate amount of the test compound was first weighed and dissolved in DMSO (Macklin cas: 67-68-5) to prepare a 60 mg/mL solution. Then, 50 µL of this solution was added to 100 µL of Solutol (Sigma cas: 70142-34-6), followed by 850 µL of 20% Captisol (Selleck cas: 182410-00-0), resulting in a final drug concentration of 3 mg/mL.

Procedures: The rats were fasted overnight with free access to water before the experiment. Food was restored two hours after dosing. Blood samples were collected at different time points after intravenous injection or oral gavage of the test compound in rats, and the compound concentration in plasma was measured. Approximately 50 µL of blood was collected from each animal using a capillary sampling tube via the orbital venous plexus, with heparin sodium as an anticoagulant. Blood samples were placed on ice, centrifuged at 3500 r/min for 10 minutes to separate the plasma, and 20 µL of the collected plasma was added to 200 µL of acetonitrile (Merck cas: 75-05-8) containing 200 nM dexamethasone (Selleck cas: 50-02-2) as an internal standard. The mixture was centrifuged at 8000 r/min for 20 minutes. After centrifugation, 150 µL of the supernatant was transferred to a new centrifuge tube, and 150 µL of 0.1% formic acid (Fisher cas: 207868) was added. The mixture was mixed well, and 5 µL of the sample was subjected to quantitative analysis by liquid chromatography-mass spectrometry (Q-TOF LC/MS).

Standard Curve Determination:
**1.** The test compound was serially diluted with DMSO to cover the compound concentrations expected in plasma samples to be tested, with a blank sample (containing only DMSO) included.
**2.** 2 µL of each diluted sample at various concentrations was added to 18 µL of normal SD rat plasma, mixed, and then added to 200 µL of acetonitrile (Merck cas: 75-05-8) containing 200 nM dexamethasone (Selleck cas: 50-02-2) as an internal standard. The mixture was centrifuged for 20 minutes (8000 r/min). 150 µL of the supernatant was transferred to a new centrifuge tube, and 150 µL of 0.1% formic acid (Fisher cas: 207868) was added. The mixture was mixed well, and 5 µL of the sample was subjected to quantitative analysis by liquid chromatography-mass spectrometry (Q-TOF LC/MS).
**3.** The final standard curve was plotted using the concentration of the test compound after dilution on the abscissa and the ratio of the signal of the compound to the internal standard (dexamethasone) on the ordinate. The standard curve was generated using linear regression with GraphPad Prism 8 software (R2 > 0.9900).

Data Analysis: calculation of pharmacokinetic parameters: the concentration of the test compound in plasma at different time points after intravenous injection or oral gavage in SD rats was determined based on the above standard curve, and pharmacokinetic parameters (T1/2, Cmax, AUC, *etc.)* were calculated using the Phoenix WinNonlin 8.1 non-compartmental analysis model.

**Test Results:** See Table 7.

**Table 7: Pharmacokinetic Data of Rats after Oral Gavase (PO, 1 mpk)**

| **Test Compound** | **AUC_{0-∞} (ng·hr/mL)** | **Cmax (ng/mL)** | **t_{1/2} (hr)** |
|---|---|---|---|
| **AZD5305*** | 5,600 | 1,398 | 4.09 |
| **1** | 11,347 | 1,740 | 4.33 |
| **2** | 19,331 | 2,000 | 5.07 |
| **14** | 19,205 | 1,774 | 6.35 |
| **52** | 18,758 | 1,451 | 5.67 |
| **54** | 16,752 | 1,693 | 7.2 |

The test results indicat that the example compounds of the present application exhibit favorable pharmacokinetic properties.

### Experimental Example 6: In Vivo Efficacy Assay

### 1. Mouse CDX Tumor Model

**Test Objective:** The efficacy of the compounds was evaluated in a subcutaneous xenograft model of human breast cancer (MDA-MB-436) cell line in mice.

### Test Compounds: Olaparib, Compound 1

**Experimental Animals:** Female NOD SCID mice, 4-5 weeks old, purchased from Nanjing Jicui.

Housing and Management: The experimental animals were housed in SPF-grade, temperature- and humidity-controlled clean rooms, using individually ventilated cages (IVC) with no more than 5 mice per cage. Temperature/humidity was controlled within the range of (23±3)°C/40-70%. Feed and water: SPF-grade mouse feed, sterilized by cobalt-60 irradiation. Drinking water was ultrafiltered and autoclaved. Animals had free access to sterile food and water. Animal identification: ear tags.

**Tumor Cell Line and Tumor Model:** MDA-MB-436 cells (Nanjing Cobioer Biotechnology Co., Ltd., Cat. No: CBP60385) were cultured *in vitro* in DMEM complete medium containing 10% fetal bovine serum, 1% penicillin-streptomycin, 1% insulin, and 16 µg/mL glutathione (37°C, 5% CO₂). When the cells met the requirements for the *in vivo* efficacy experiment, they were collected, counted, and prepared into a PBS cell suspension. Each mouse (5-6 weeks old NOD SCID) was inoculated subcutaneously on the right flank with 0.9 × 10⁷ cells. Tumor volume was measured and calculated three times a week after inoculation. When the tumor volume reached approximately 200-300 mm³, the mice were grouped for administration.

**Experimental Groups:** The experiment was divided into the following groups: vehicle control group; Olaparib 100 mg/kg group; Compound 1 0.1 mg/kg group; Compound 1 0.3 mg/kg group; and Compound 1 1.0 mg/kg group. All groups were administered orally by gavage once daily. Tumor volume and body weight were measured three times a week after administration, and the mice were observed for activity, drinking, and eating behavior. After 28 days of administration, the tumor volume was used to calculate the tumor growth inhibition rate (TGI%), and the tumor inhibition effect was statistically analyzed using software such as Excel, *etc.* TGI={1-(Tumor volume at end of administration in treatment group - Tumor volume at grouping in treatment group)/(Tumor volume at end of administration in control group - Tumor volume at grouping in control group)}x100%

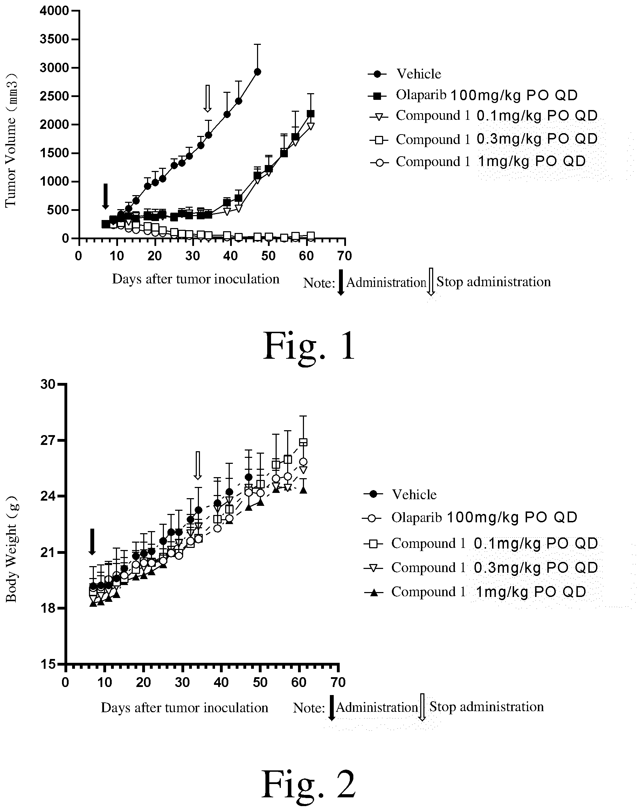

**Test Results:** As shown in Table 8, Compound **1** exhibited a dose-dependent inhibition of tumor growth. The tumor inhibition effect of Compound **1** at 0.1 mg/kg was comparable to that of Olaparib at 100 mg/kg. Compound **1** at doses of 0.3 mg/kg and 1.0 mg/kg completely inhibited tumor growth after 28 days of treatment. There was no significant difference in body weight between the treatment groups and the vehicle control group. Specific results are shown in Figures 1 and 2.

**Table 8: Tumor Growth Inhibition Rate in MDA-MB-436 Breast Cancer Mouse Model after 28 Days of administration**

| **Test Compound** | **Dose for Oral Administration** | **Tumor Growth Inhibition Rate (TGI)** |
|---|---|---|
| Olaparib | 100 mg/kg | 89% |
| **Compound 1** | 0.1 mg/kg | 89% |
| **Compound 1** | 0.3 mg/kg | 113% |
| **Compound 1** | 1.0 mg/kg | 115% |

### 2. Human Breast Cancer PDX Mouse Model

**Test Objective:** The *in vivo* efficacy of the test compound was evaluated in a subcutaneous xenograft tumor model of breast cancer BR-05-0044E in NOD SCID mice.

### Test Compound: Compound 1

**Experimental Animals:** Female NOD SCID mice, 6-8 weeks old, weighing 18-20 grams were provided by Beijing Charles River Laboratory Animal Technology Co., Ltd. The animals were acclimated in the experimental environment for 3-7 days before the experiment. The animals were housed in SPF-grade animal rooms in IVC cages (individually ventilated cages) with 4 mice per cage. All cages, bedding, and drinking water were sterilized before use. Cages, feed, and water were changed twice a week. The housing environment and lighting conditions were as follows: temperature: 20-26°C, humidity: 40-70%, light cycle: 12 hours light, 12 hours dark.

**PDX Model Establishment:** The human breast cancer BR-05-0044E model was initially derived from clinical samples surgically removed. After tumor tissue was expanded in mice, 20-30 mm³ BR-05-0044E tumor tissue blocks were subcutaneously implanted into the right flank of each mouse. Tumor growth was monitored, and when the average tumor volume reached approximately 150-200 mm³, the mice were randomly grouped for administration. Animals were weighed, and tumor volume was measured before administration. Random grouping was based on tumor volume (random block design).

**Experimental Groups:** The experiment was divided into the following groups: 1.Vehical control group, 2. Compound **1** 0.1 mg/kg, 3. Compound **1** 0.3 mg/kg, 4. Compound 1 1.0 mg/kg. All groups were administered orally by gavage once daily. Routine checks included observing tumor growth and the impact of drug treatment on the animals' daily behavior (such as activity, food and water intake, body weight changes (measured twice a week), appearance, or other abnormalities). The animal number of deaths and side effects in each group were recorded.

**Experimental Indicators:** The experimental indicators were to assess whether tumor growth was inhibited, delayed, or cured. Tumor diameter was measured twice a week with calipers. Tumor volume was calculated using the formula: V = 0.5*a* × *b*², where *a* and b represent the tumor's length and width, respectively.

The efficacy of the compound on inhibiting tumor was evaluated by tumor volume and tumor growth inhibition rate (TGI). TGI (%) was calculated as follows: TGI (%) = {1-(Average Tumor volume at end of administration in treatment group - Average Tumor volume at start of administration in treatment group)/(Average Tumor volume at end of administration in vehicle control group - Average Tumor volume at start of administration in vehicle control group)}x100%.

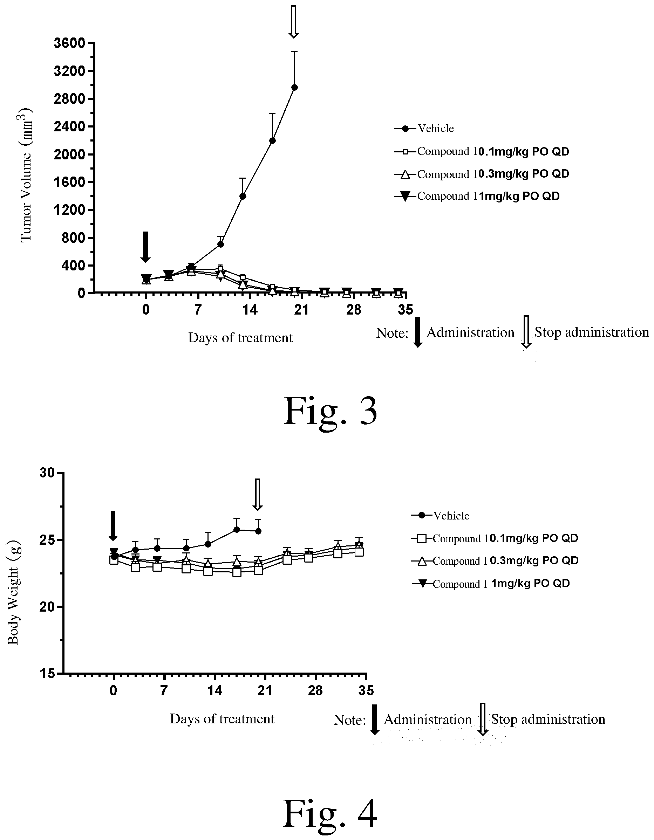

**Test Results:** In the BR-05-0044E FP5 breast cancer mouse PDX model, Compound **1** (0.1, 0.3, 1.0 mg/kg) was administered orally by gavage once daily for 21 days. All tested doses completely inhibited tumor growth, and tumors in the mice of the treatment group completely disappeared. After the end of administration, the mice were observed for additional 2 weeks, and no tumor growth was observed in the treatment group. There was no significant difference in body weight between the drug treatment groups and the vehicle control group. Specific results are shown in Table 9, Figure **3,** and Figure **4****.**

**Table 9: Tumor Growth Inhibition Rate in Breast Cancer PDX Model after 21 Days of administration**

| **Test Compound** | **Dose for Oral Administration** | **Tumor Growth Inhibition Rate (TGI)** |
|---|---|---|
| **Compound 1** | 0.1 mg/kg | 105% |
| **Compound 1** | 0.3 mg/kg | 106% |
| **Compound 1** | 1.0 mg/kg | 107% |

### Experimental Example 7: Effect of Compound on Rat Reticulocytes

**Test Objective:** The effect of the compound on peripheral blood reticulocytes was evaluated to determine its impact on bone marrow hematopoietic function.

### Test Compounds: Olaparib, Compound 1

**Experimental Animals:** Male Sprague Dawley rats were purchased from Beijing Charles River Laboratory Animal Technology Co., Ltd. Housing and Management: The experimental animals were housed in SPF-grade, temperature- and humidity-controlled clean rooms, using individually ventilated cages (IVC) with no more than 5 rats per cage. Temperature/humidity was controlled within the range of (23±3)°C/40-70%. Feed and water: SPF-grade rat feed, sterilized by cobalt-60 irradiation. Drinking water was ultrafiltered and autoclaved. Animals had free access to sterile food and water. Animal identification: ear tags.

**Experimental Method:** The experiment was divided into the following 5 groups: vehicle control group; Olaparib 100 mg/kg; Compound **1** 0.1 mg/kg; Compound **1** 0.3 mg/kg; and Compound **1** 1.0 mg/kg. All treatments were administered orally by gavage once daily for 4 days. Blood samples were collected before and after treatment to measure peripheral blood reticulocyte counts.

**Test Results:** Table **10** shows the changes in peripheral blood reticulocytes in rats after 4 days of administration. After 4 days of administration in rats, Olaparib at 100 mg/kg significantly reduced peripheral blood reticulocytes (p<0.01), indicating its inhibitory effect on bone marrow hematopoietic function. Compound **1** 0.1 mg/kg, Compound **1** 0.3 mg/kg, and Compound **1** 1.0 mg/kg had no significant effect on peripheral blood reticulocytes (p>0.05). Combined with the pharmacodynamic results, Compound **1** demonstrated a larger therapeutic window compared to Olaparib.

**Table 10: Effect of Compound on Peripheral Blood Reticulocvtes in Rats**

| **Test Compound** | **Dose for Oral Administration** | **Reticulocyte Inhibition Rate** |
|---|---|---|
| Olaparib | 100 mg/kg | 91.6% |
| **compound 1** | 0.1 mg/kg | 12.2% |
| **compound 1** | 0.3 mg/kg | 33.8% |
| **compound 1** | 1.0 mg/kg | 41.9% |

Various modifications of the invention in addition to those described herein will become apparent to those skilled in the art from the foregoing description. Such modifications are intended to fall within the scope of the appended claims. Each reference, including all patents, applications, journal articles, books and any other disclosure, referred to herein is hereby incorporated by reference in its entirety.

## Claims

1. A compound or a pharmaceutically acceptable salt, ester, stereoisomer, tautomer, polymorph, solvate, metabolite, isotopically labeled compound or prodrug thereof, wherein the compound has the structure of Formula (I): 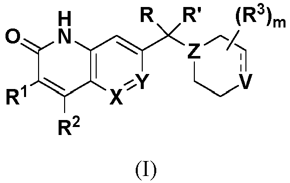 wherein:
- - - is a single bond or a double bond;
X is N or CR⁵;
Y is Nor CR⁵';
Z is CR⁶ or N;
when - - - is a single bond, V is CR⁷R^{A} or NR^{A}; when - - - is a double bond, V is CR^{A};
R^{A} is selected from the group consisting of H, halogen, -OH, -NH₂, -CN, -NO₂, C₁₋₆ alkyl, C₁₋₆ haloalkyl, C₃₋₆ cyclic hydrocarbyl, 3- to 10-membered heterocyclyl, C₆₋₁₀ aryl, 5- to 14-membered heteroaryl, C₆₋₁₂ aralkyl, -C(=O)R^{a}, -OC(=O)R^{a}, -C(=O)OR^{a}, -OR^{a}, -SR^{a}, -S(=O)R^{a}, -S(=O)₂R^{a}, -S(=O)₂NR^{a}R^{b}, -NR^{a}R^{b}, - C(=O)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -C₁₋₆ alkylene-CN, - C₁₋₆ alkylene-OR^{a}, -C₁₋₆ alkylene-NR^{a}R^{b}, -O-C₁₋₆ alkylene-NR^{a}R^{b} and 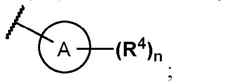
ring A is a C₃₋₆ hydrocarbon ring, 3- to 10-membered heterocycle, C₆₋₁₀ aromatic ring or 5- to 14-membered heteroaromatic ring;
R and R', at each occurrence, are each independently selected from the group consisting of H, halogen, -OH, -NH₂, -CN, -NO₂, C₁₋₆ alkyl, C₁₋₆ haloalkyl, C₃₋₆ cyclic hydrocarbyl, 3- to 10-membered heterocyclyl, C₆₋₁₀ aryl, 5- to 14-membered heteroaryl, C₆₋₁₂ aralkyl, -C(=O)R^{a}, -OC(=O)R^{a}, -C(=O)OR^{a}, -OR^{a}, -SR^{a}, - S(=O)R^{a}, -S(=O)₂R^{a}, -S(=O)₂NR^{a}R^{b}, -NR^{a}R^{b}, -C(=O)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -C₁₋₆ alkylene-CN, -C₁₋₆ alkylene-OR^{a}, -C₁₋₆ alkylene-NR^{a}R^{b} and -O-C₁₋₆ alkylene-NR^{a}R^{b}; preferably, R and R' are each independently selected from the group consisting of H, -CN and C₁₋₆ alkyl;
R¹ and R² are each independently selected from the group consisting of halogen, -OH, -NH₂, -CN, -NO₂, C₁₋₆ alkyl, C₁₋₆ haloalkyl, C₃₋₆ cyclic hydrocarbyl, 3- to 10-membered heterocyclyl, C₆₋₁₀ aryl, 5- to 14-membered heteroaryl, C₆₋₁₂ aralkyl, -C(=O)R^{a}, -OC(=O)R^{a}, -C(=O)OR^{a}, -OR^{a}, -SR^{a}, -S(=O)R^{a}, -S(=O)₂R^{a}, - S(=O)₂NR^{a}R^{b}, -NR^{a}R^{b}, -C(=O)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -C₁₋₆ alkylene-CN, -C₁₋₆ alkylene-OR^{a}, -C₁₋₆ alkylene-NR^{a}R^{b} and -O-C₁₋₆ alkylene-NR^{a}R^{b};
R³, at each occurrence, is each independently selected from the group consisting of halogen, -OH, =O, -NH₂, -CN, -NO₂, C₁₋₆ alkyl, C₁₋₆ haloalkyl, C₃₋₆ cyclic hydrocarbyl, 3- to 10-membered heterocyclyl, C₆₋₁₀ aryl, 5- to 14-membered heteroaryl, C₆₋₁₂ aralkyl, -C(=O)R^{a}, -OC(=O)R^{a}, -C(=O)OR^{a}, -OR^{a}, -SR^{a}, -S(=O)R^{a}, -S(=O)₂R^{a}, -S(=O)₂NR^{a}R^{b}, -NR^{a}R^{b}, -C(=O)NR^{a}R^{b}, -NR^{a}-C(=O)R^{b}, -NR^{a}-C(=O)OR^{b}, -NR^{a}-S(=O)₂-R^{b}, - NR^{a}-C(=O)-NR^{a}R^{b}, -C₁₋₆ alkylene-CN, -C₁₋₆ alkylene-OR^{a}, -C₁₋₆ alkylene-NR^{a}R^{b} and -O-C₁₋₆ alkylene-NR^{a}R^{b};
when m>1, two R³ groups optionally together form -C₁₋₆ alkylene- or -C₂₋₆ alkenylene-, the alkylene chain and alkenylene chain are optionally interrupted by one or more groups independently selected from the group consisting of O, C(=O), C(=O)O, NR, S, S=O and S(=O)₂;
or, R³ and R^{A} together with the groups to which they are attached optionally form a C₃₋₆ hydrocarbon ring, 3- to 10-membered heterocycle, C₆₋₁₀ aromatic ring or 5- to 14-membered heteroaromatic ring;
R4, at each occurrence, is each independently selected from the group consisting of halogen, -OH, -NH₂, -CN, -NO₂, C₁₋₆ alkyl, C₁₋₆ haloalkyl, C₃₋₆ cyclic hydrocarbyl, 3- to 10-membered heterocyclyl, C₆₋₁₀ aryl, 5-to 14-membered heteroaryl, C₆₋₁₂ aralkyl, -C(=O)R^{a}, -OC(=O)R^{a}, -C(=O)OR^{a}, -OR^{a}, -SR^{a}, -S(=O)R^{a}, - S(=O)₂R^{a}, -S(=O)₂NR^{a}R^{b}, -NR^{a}R^{b}, -C(=O)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -C₁₋₆ alkylene-CN, -C₁₋₆ alkylene-OR^{a}, -C₁₋₆ alkylene-NR^{a}R^{b} and -O-C₁₋₆ alkylene-NR^{a}R^{b}; when two R⁴ groups are ortho to each other on ring A, the two R⁴ groups together with the groups to which they are attached optionally form 3- to 10-membered heterocycle or 5- to 14-membered heteroaromatic ring;
or, R³ and R⁴ together with the groups to which they are attached optionally form a C₃₋₆ hydrocarbon ring, 3- to 10-membered heterocycle, C₆₋₁₀ aromatic ring or 5- to 14-membered heteroaromatic ring;
R⁵, R⁵', R⁶ and R⁷, at each occurrence, are each independently selected from the group consisting of H, halogen, -OH, -NH₂, -CN, -NO₂, C₁₋₆ alkyl, C₁₋₆ haloalkyl, C₃₋₆ cyclic hydrocarbyl, 3- to 10-membered heterocyclyl, C₆₋₁₀ aryl, 5- to 14-membered heteroaryl, C₆₋₁₂ aralkyl, -C(=O)R^{a}, -OC(=O)R^{a}, -C(=O)OR^{a}, - OR^{a}, -SR^{a}, -S(=O)R^{a}, -S(=O)₂R^{a}, -S(=O)₂NR^{a}R^{b}, -NR^{a}R^{b}, -C(=O)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -C₁₋₆ alkylene-CN, -C₁₋₆ alkylene-OR^{a}, -C₁₋₆ alkylene-NR^{a}R^{b} and -O-C₁₋₆ alkylene-NR^{a}R^{b};
R^{a} and R^{b}, at each occurrence, are each independently selected from the group consisting of H, C₁₋₆ alkyl, C₁₋₆ haloalkyl, C₂₋₆ alkenyl, C₃₋₁₀ cyclic hydrocarbyl, 3- to 10-membered heterocyclyl, C₆₋₁₀ aryl, 5- to 14-membered heteroaryl and C₆₋₁₂ aralkyl;
the above alkyl, alkylene, haloalkyl, alkenyl, alkenylene, hydrocarbon ring, cyclic hydrocarbyl, heterocycle, heterocyclyl, aryl, aromatic ring, heteroaryl, heteroaromatic ring and aralkyl, at each occurrence, are each optionally substituted with one or more substituents independently selected from the group consisting of: halogen, -OH, =O, -NH₂, -CN, -NO₂, C₁₋₆ alkyl, C₁₋₆ haloalkyl, C₃₋₆ cyclic hydrocarbyl, 3- to 10-membered heterocyclyl, C₆₋₁₀ aryl, 5- to 14-membered heteroaryl, C₆₋₁₂ aralkyl, -C(=O)R^{c}, -OC(=O)R^{c}, - C(=O)OR^{c}, -OR^{c}, -SR^{c}, -S(=O)R^{c}, -S(=O)₂R^{c}, -S(=O)₂NR^{c}R^{d}, -NR^{c}R^{d}, -C(=O)NR^{c}R^{d}, -NR^{c}-C(=O)R^{d}, -NR^{c}-C(=O)OR^{d}, -NR^{c}-S(=O)₂-R^{d}, -NR^{c}-C(=O)-NR^{c}R^{d}, -C₁₋₆ alkylene-OR^{c}, -C₁₋₆ alkylene-NR^{c}R^{d} and -O-C₁₋₆ alkylene-NR^{c}R^{d}, the alkyl, alkylene, haloalkyl, cyclic hydrocarbyl, heterocyclyl, aryl, heteroaryl and aralkyl are further optionally substituted with one or more substituents independently selected from the group consisting of: halogen, -OH, =O, -NH₂, -CN, -NO₂, C₁₋₆ alkyl, C₁₋₆ haloalkyl, C₃₋₆ cyclic hydrocarbyl, 3- to 10-membered heterocyclyl, C₆₋₁₀ aryl, 5- to 14-membered heteroaryl and C₆₋₁₂ aralkyl;
R^{c} and R^{d}, at each occurrence, are each independently selected from the group consisting of H, C₁₋₆ alkyl, C₁₋₆ haloalkyl, C₃₋₁₀ cyclic hydrocarbyl, 3- to 10-membered heterocyclyl, C₆₋₁₀ aryl, 5- to 14-membered heteroaryl and C₆₋₁₂ aralkyl; and
m and n are each independently an integer of 0, 1, 2, 3, or 4.

2. The compound according to claim 1, or a pharmaceutically acceptable salt, ester, stereoisomer, tautomer, polymorph, solvate, metabolite, isotopically labeled compound or prodrug thereof, wherein R^{A} is selected from the group consisting of C₁₋₆ haloalkyl (preferably 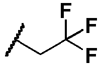 ), -C(=O)R^{a} (preferably 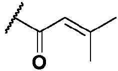 ) and

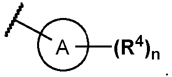

3. The compound according to claim 1 or 2, or a pharmaceutically acceptable salt, ester, stereoisomer, tautomer, polymorph, solvate, metabolite, isotopically labeled compound or prodrug thereof, wherein the compound has the structure of Formula (I)-1 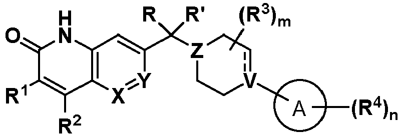 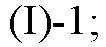 preferably, the compound has the structure of Formula (II), (III), (IV) or (V):
| | |
|---|---|
| 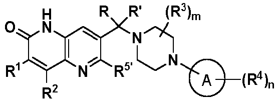 | 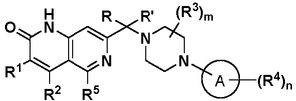 |
| (II) | (III) |
| 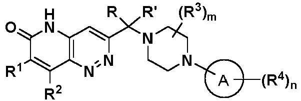 | 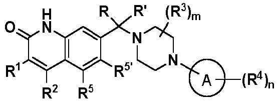 |
| (IV) | (V). |

4. The compound according to any one of claims 1 to 3, or a pharmaceutically acceptable salt, ester, stereoisomer, tautomer, polymorph, solvate, metabolite, isotopically labeled compound or prodrug thereof, wherein R¹ and R² are each independently halogen, -CN, C₁₋₆ alkyl or C₁₋₆ haloalkyl; for example, R¹ and R² are each independently C₁₋₆ alkyl or C₁₋₆ haloalkyl;
preferably, R¹ and R² are each independently F, -CN, methyl, difluoromethyl, trifluoromethyl, ethyl, n-propyl or isopropyl; for example, R¹ and R² are each independently methyl, trifluoromethyl, ethyl, n-propyl or isopropyl;
most preferably, R' is trifluoromethyl, and R² is methyl; or R¹ and R² are both methyl.

5. The compound according to any one of claims 1 to 4, or a pharmaceutically acceptable salt, ester, stereoisomer, tautomer, polymorph, solvate, metabolite, isotopically labeled compound or prodrug thereof, wherein R³, at each occurrence, is each independently halogen, -OH, =O, -NH₂, -CN, C₁₋₆ alkyl, C₁₋₆ haloalkyl, -C₁₋₆ alkylene-CN, -C₁₋₆ alkylene-OR^{a} or -C₁₋₆ alkylene-NR^{a}R^{b}; when m>1, two R³ groups optionally together form -C₁₋₄ alkylene-;
preferably, R³, at each occurrence, is each independently =O, -CN, C₁₋₆ alkyl, C₁₋₆ haloalkyl, -C₁₋₆ alkylene-CN, -C₁₋₆ alkylene-OR^{a} or -C₁₋₆ alkylene-NR^{a}R^{b}; when m>1, two R³ groups optionally together form -C₁₋₄ alkylene-;
more preferably, R³, at each occurrence, is each independently =O, -CN, -CH₃, -CF₃, -CH₂CN, -CH₂NH₂, -CH₂CH₂NH₂, -CH₂OH, -CH₂CH₂OH or -CH₂OCH₃; when m>1, two R³ groups optionally together form - CH₂CH₂-;
or, R³ and R^{A} together with the groups to which they are attached optionally form 5- to 6-membered heteroaromatic ring (preferably a triazole ring), which is optionally substituted with C₁₋₆ haloalkyl (preferably trifluoromethyl).

6. The compound according to any one of claims 1 to 5, or a pharmaceutically acceptable salt, ester, stereoisomer, tautomer, polymorph, solvate, metabolite, isotopically labeled compound or prodrug thereof, wherein R⁶ and R⁷, at each occurrence, are each independently selected from the group consisting of H, halogen, -OH, -CN, -C₁₋₆ alkylene-OR^{a} and -C₁₋₆ alkylene-NR^{a}R^{b};
R⁶ and R⁷, at each occurrence, are each independently selected from the group consisting of H, -F, -OH, -CN, -CH₂OH and -CH₂NH₂.

7. The compound according to any one of claims 1 to 6, or a pharmaceutically acceptable salt, ester, stereoisomer, tautomer, polymorph, solvate, metabolite, isotopically labeled compound or prodrug thereof, wherein 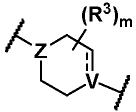 is selected from the group consisting of 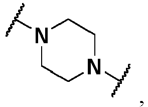 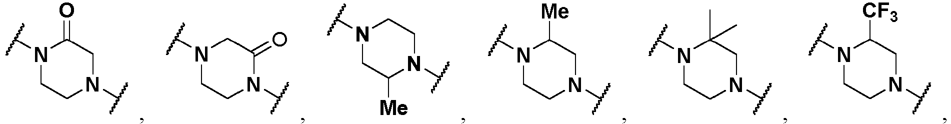 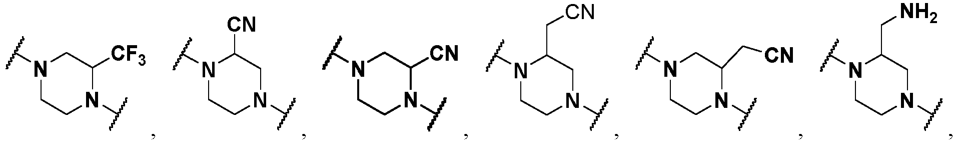 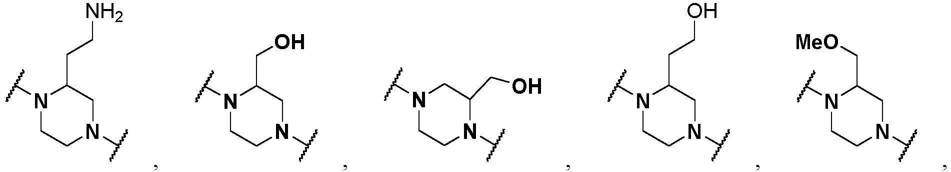 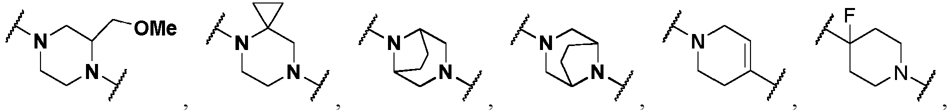 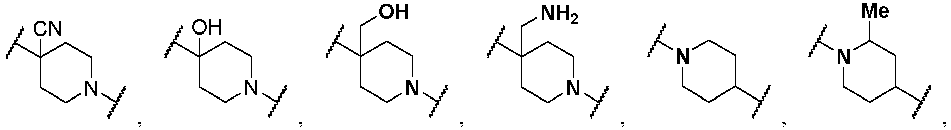 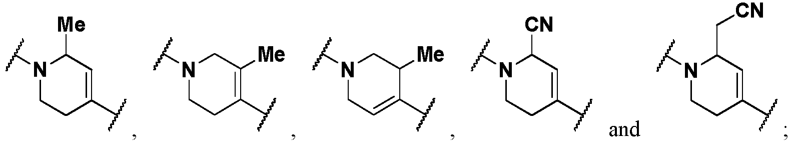 preferably, 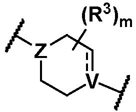 is selected from the group consisting of

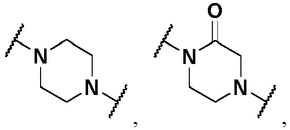

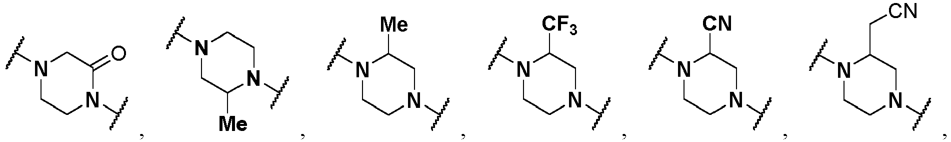

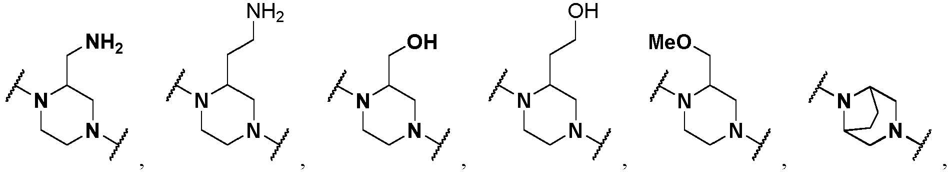

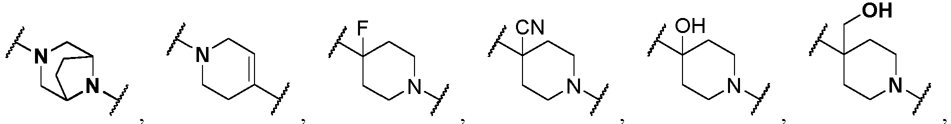

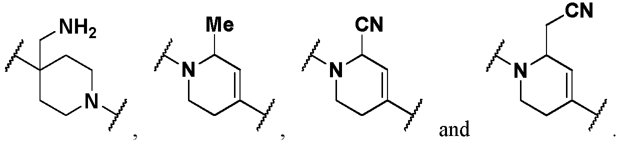

8. The compound according to any one of claims 1 to 7, or a pharmaceutically acceptable salt, ester, stereoisomer, tautomer, polymorph, solvate, metabolite, isotopically labeled compound or prodrug thereof, wherein R4, at each occurrence, is each independently halogen, -CN, C₁₋₆ alkyl, C₁₋₆ haloalkyl, C₃₋₆ cyclic hydrocarbyl, 5- to 14-membered heteroaryl, -S(=O)₂NR^{a}R^{b}, -C(=O)NR^{a}R^{b}, -NR^{a}-C(=O)R^{b}, -NR^{a}-C(=O)-NR^{a}R^{b} or -C₁₋₆ alkylene-OR^{a}; when two R⁴ groups are ortho to each other on ring A, the two R⁴ groups together with the groups to which they are attached optionally form 5- to 6-membered heterocycle or 5- to 6-membered heteroaromatic ring;
preferably, R4, at each occurrence, is each independently F, -CN, -CH₃, -CF₃, 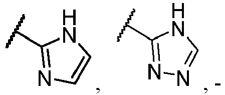 S(=O)₂NHCH₃, -C(=O)NH₂, -C(=O)NHCH₃, -C(=O)NHCD₃, -C(=O)NHCH₂CH₃, -C(=O)NHCH₂CF₃, - C(=O)NHCH₂CH₂OH, -C(=O)NH(cyclopropyl), -NHC(=O)CH₃, -NHC(=O)(cyclopropyl), - NHC(=O)NHCH₃, -CH₂OH, 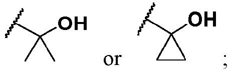 when two R⁴ groups are ortho to each other on ring A, the two R⁴ groups together with the groups to which they are attached optionally form 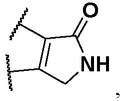 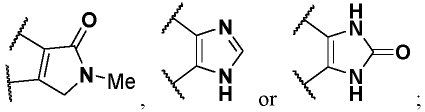
more preferably, R⁴, at each occurrence, is each independently F, -CN, -CH₃, -CF₃, 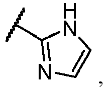 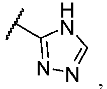 -S(=O)₂NHCH₃, -C(=O)NH₂, -C(=O)NHCH₃, -NHC(=O)CH₃, -NHC(=O)(cyclopropyl), - NHC(=O)NHCH₃, -CH₂OH, 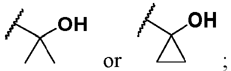 when two R⁴ groups are ortho to each other on ring A, the two R⁴ groups together with the groups to which they are attached optionally form 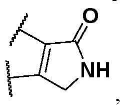 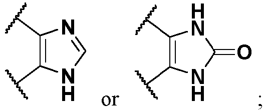
or, R³ and R⁴ together with the groups to which they are attached optionally form 5- to 6-membered heteroaromatic ring, preferably an imidazole ring.

9. The compound according to any one of claims 1 to 8, or a pharmaceutically acceptable salt, ester, stereoisomer, tautomer, polymorph, solvate, metabolite, isotopically labeled compound or prodrug thereof, wherein ring A is a C₄₋₆ hydrocarbon ring, 5- to 6-membered heterocycle, C₆ aromatic ring or 5- to 6-membered heteroaromatic ring, more preferably is a bicyclo[1.1.1]pentane ring, piperidine ring, benzene ring, imidazole ring, thiazole ring, pyridine ring, pyrazine ring, pyridazine ring, or pyrimidine ring, and most preferably is a bicyclo[1.1.1]pentane ring, piperidine ring, benzene ring, imidazole ring, thiazole ring, pyridine ring, pyridazine ring, or pyrimidine ring.

10. The compound according to any one of claims 1 to 9, or a pharmaceutically acceptable salt, ester, stereoisomer, tautomer, polymorph, solvate, metabolite, isotopically labeled compound or prodrug thereof, wherein 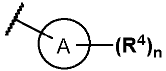 is 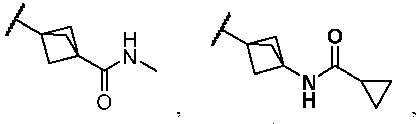 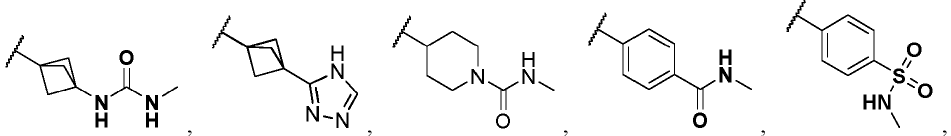 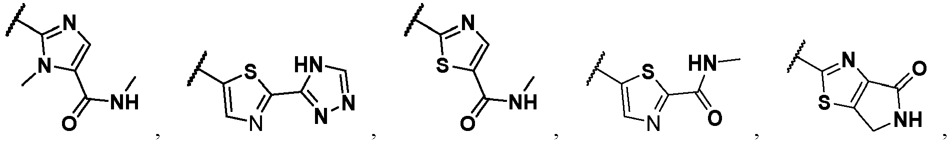 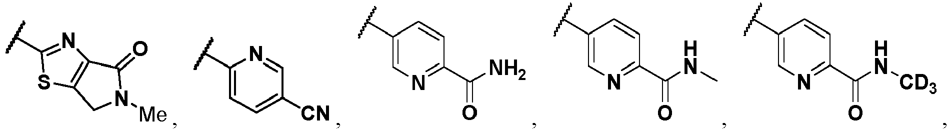 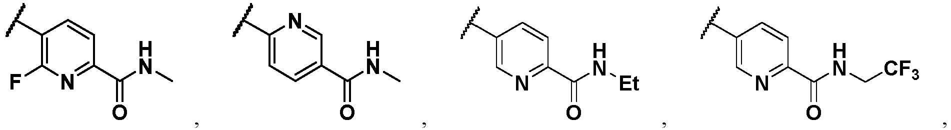 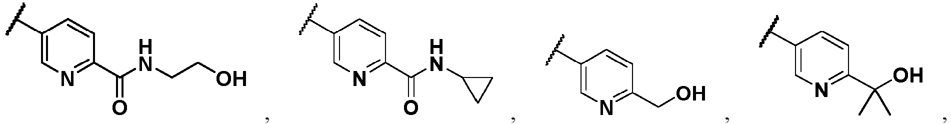 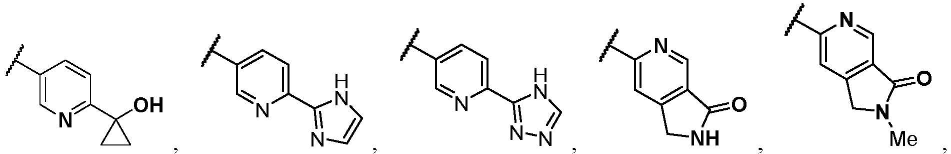 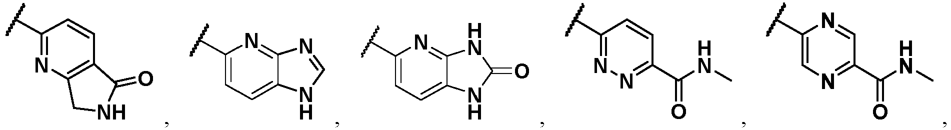 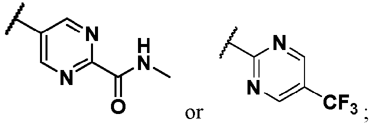 preferably, 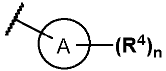 is

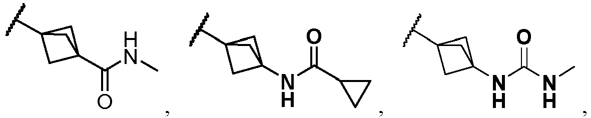

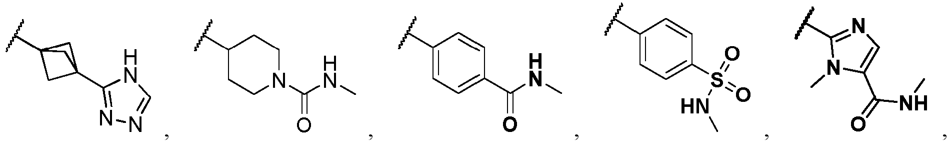

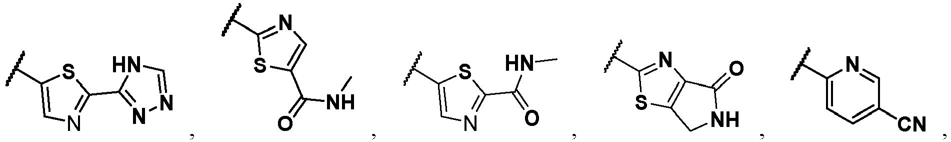

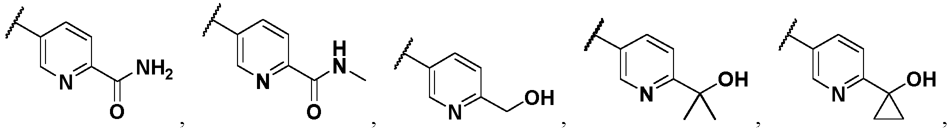

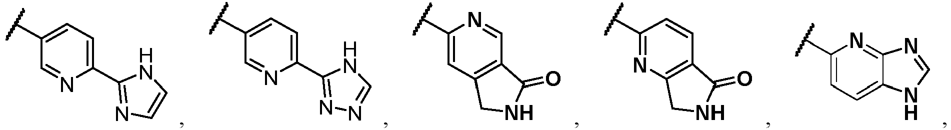

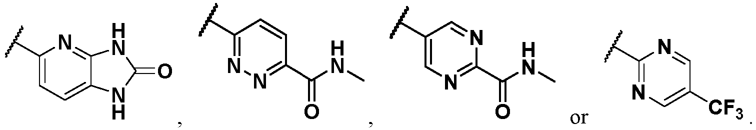

11. The compound according to any one of claims 1 to 10, or a pharmaceutically acceptable salt, ester, stereoisomer, tautomer, polymorph, solvate, metabolite, isotopically labeled compound or prodrug thereof, wherein the compound is selected from the group consisting of:

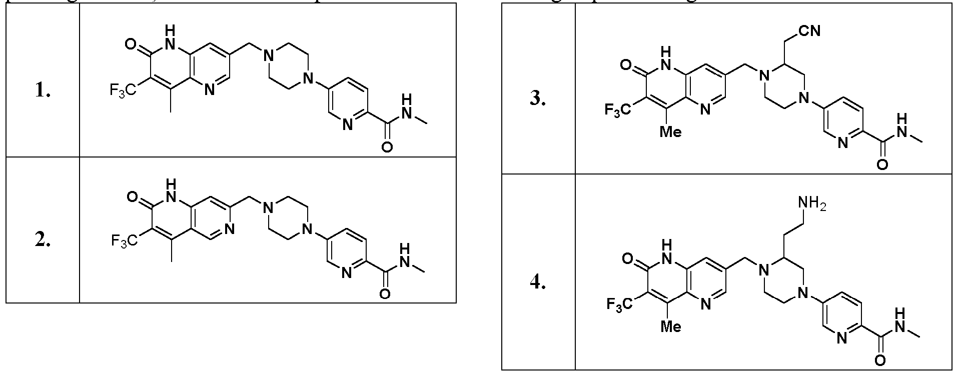

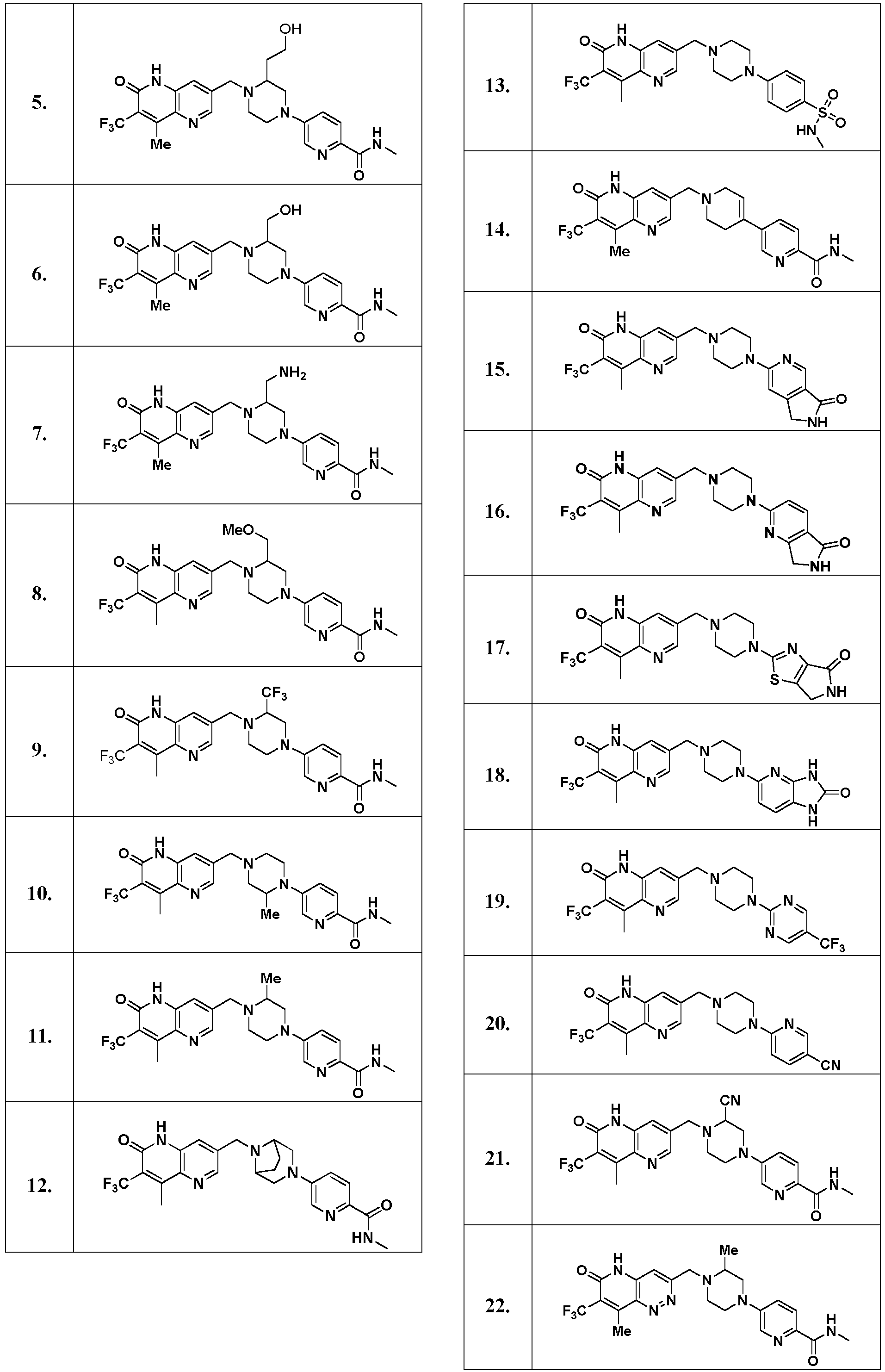

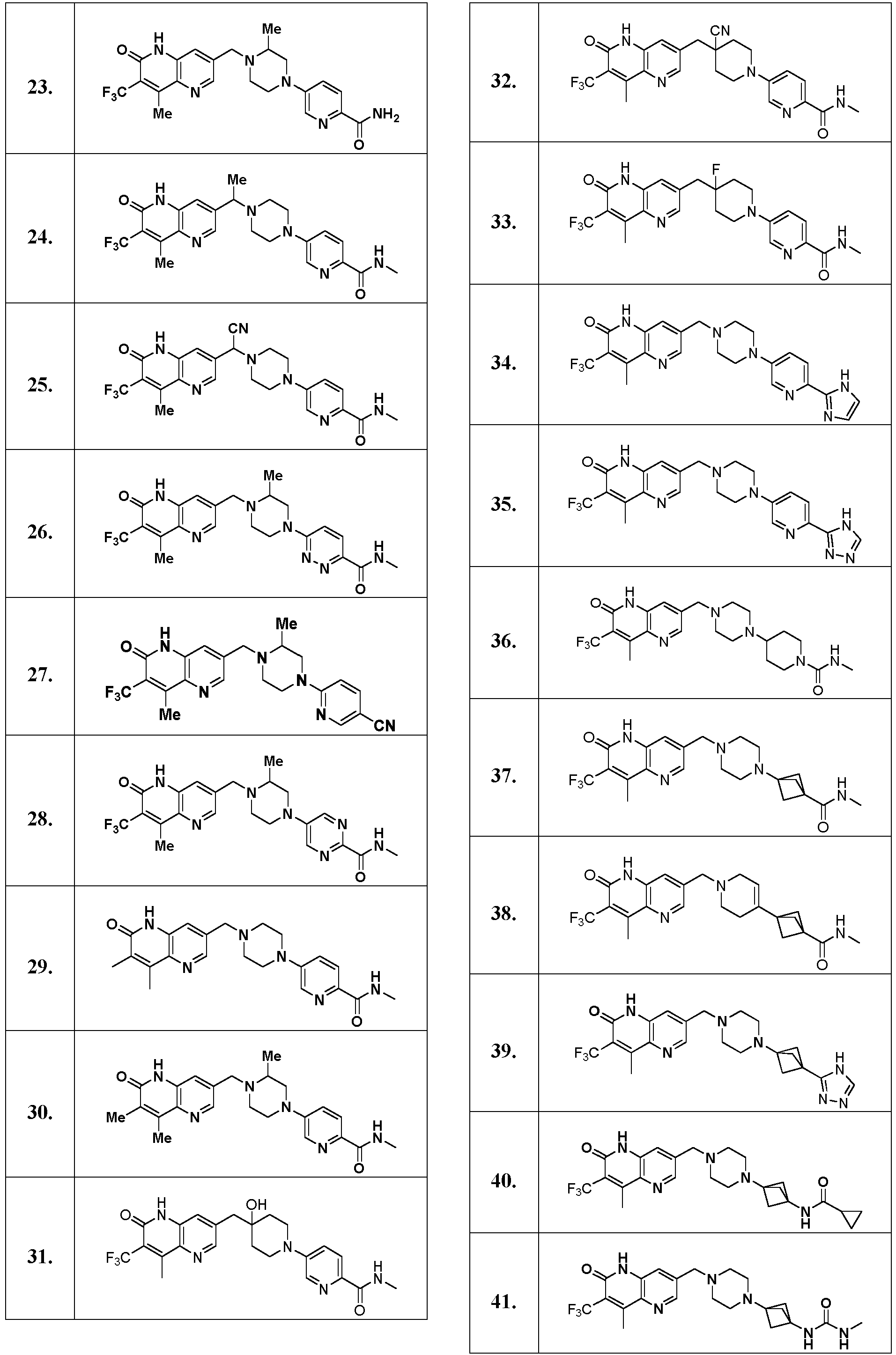

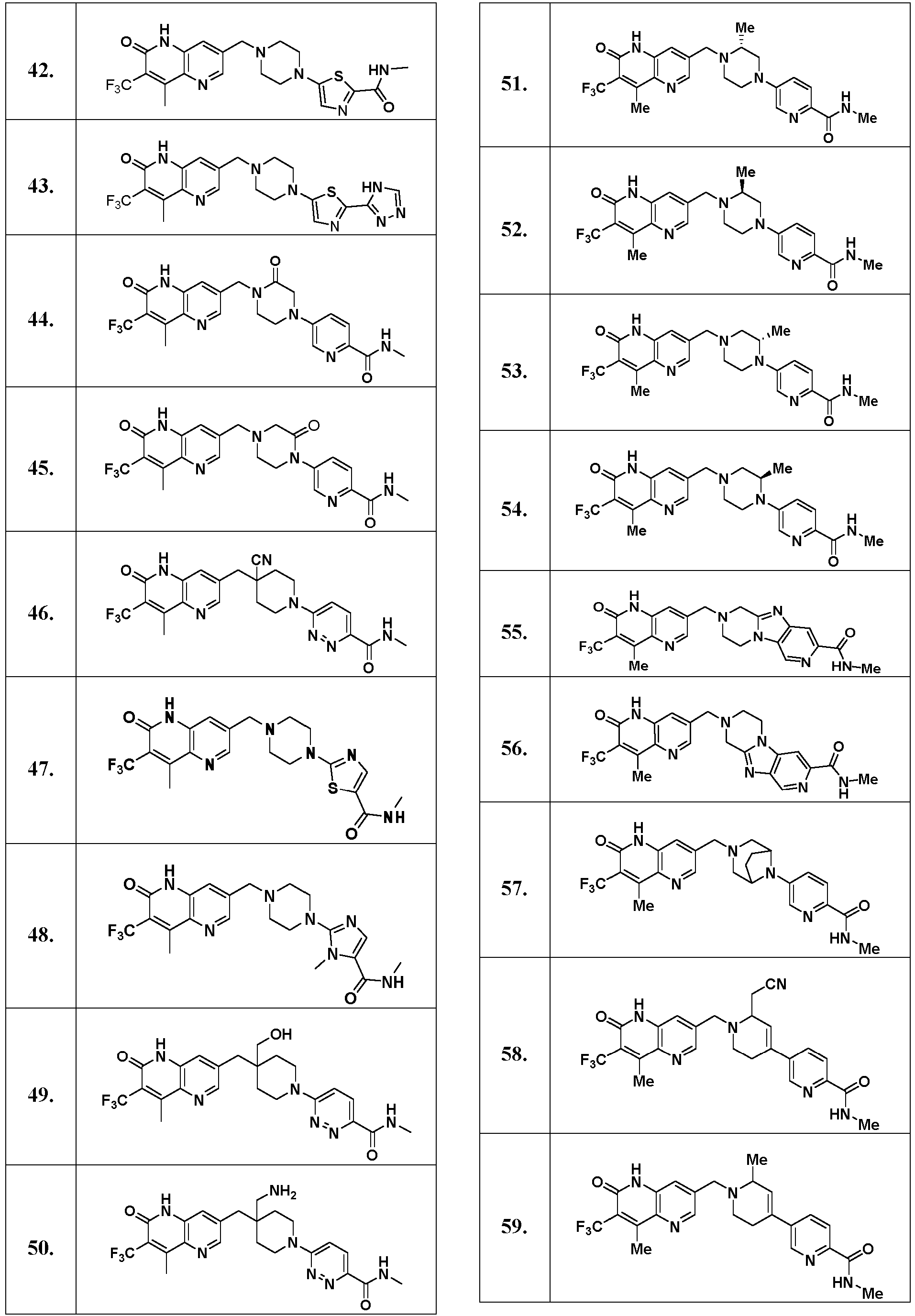

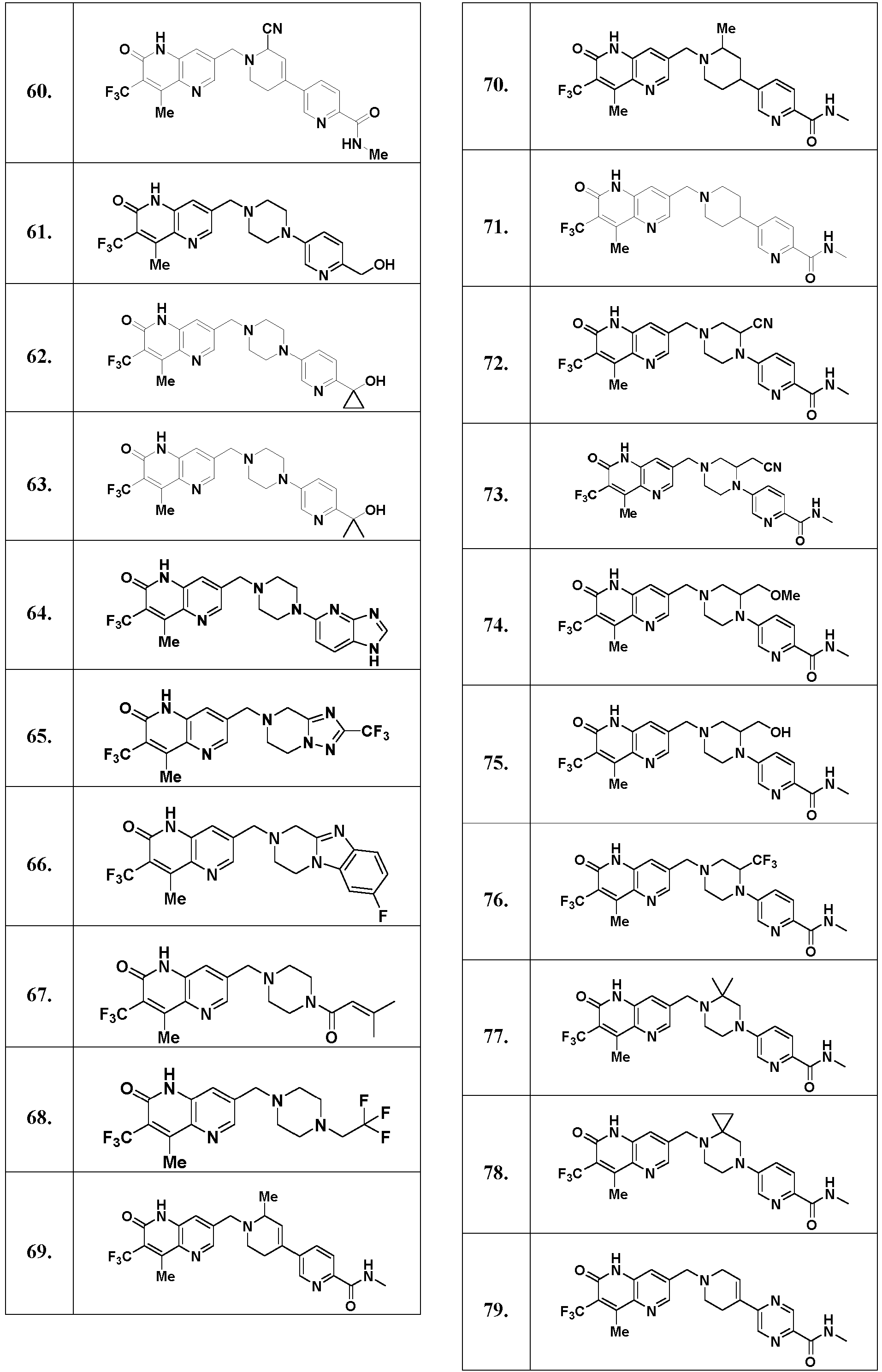

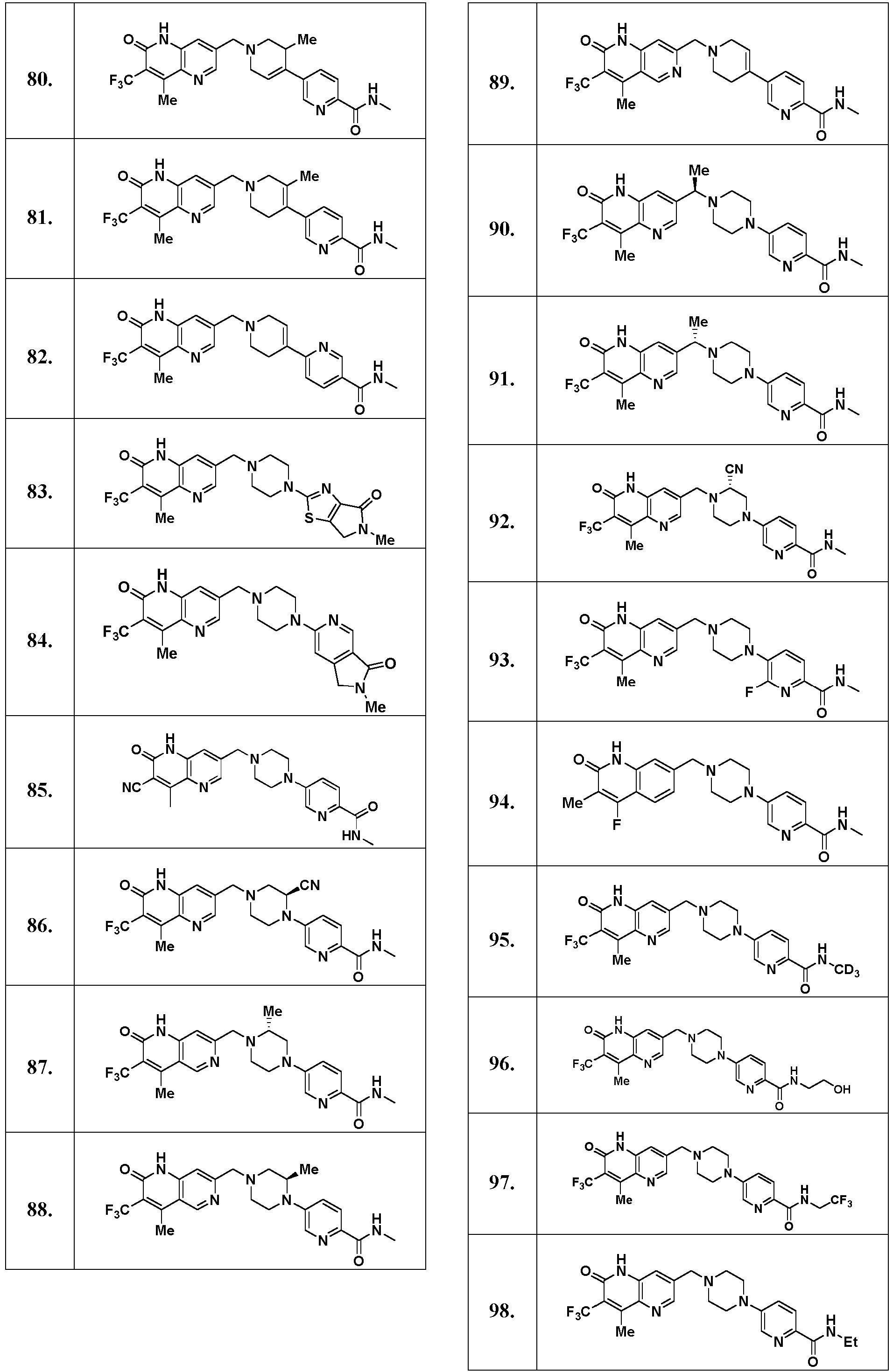

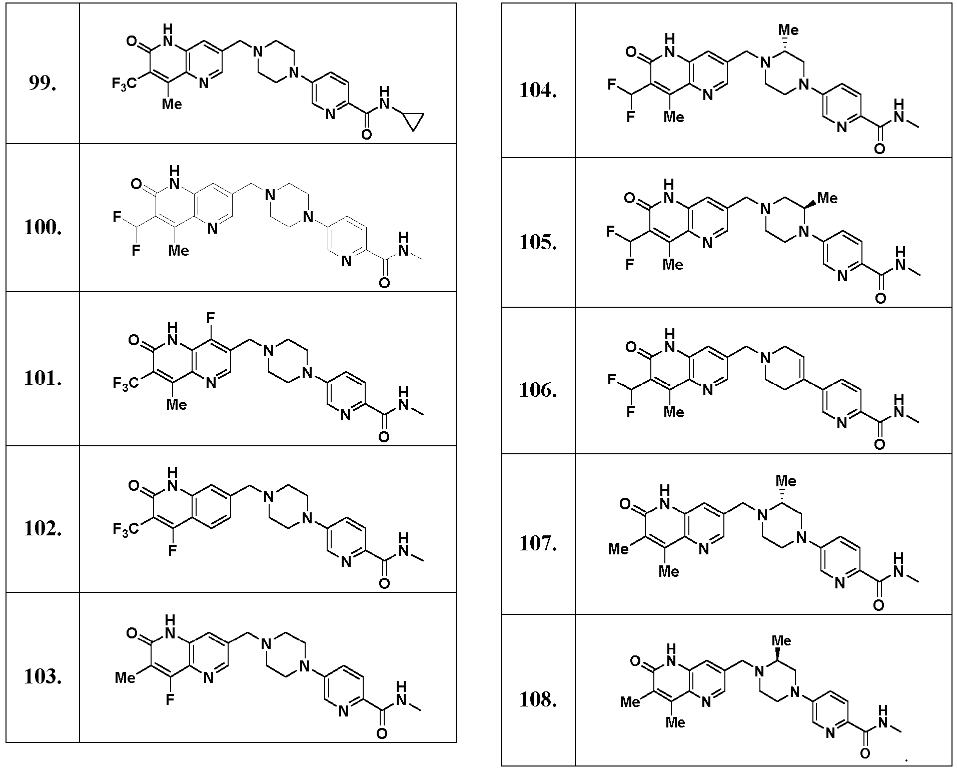

12. A pharmaceutical composition comprising a prophylactically or therapeutically effective amount of the compound according to any one of claims 1 to 11, or a pharmaceutically acceptable salt, ester, stereoisomer, tautomer, polymorph, solvate, metabolite, isotopically labeled compound or prodrug thereof, and one or more pharmaceutically acceptable carriers, and the pharmaceutical composition is preferably in the form of a solid, liquid or transdermal formulation.

13. Use of the compound according to any one of claims 1 to 11, or a pharmaceutically acceptable salt, ester, stereoisomer, tautomer, polymorph, solvate, metabolite, isotopically labeled compound or prodrug thereof or the pharmaceutical composition according to claim 12 in the manufacture of a medicament for use as a PARP inhibitor (preferably as a PARP1 selective inhibitor).

14. The use according to claim 13, wherein the medicament is for the treatment of cancer, preferably, the cancer is selected from the group consisting of ovarian cancer, breast cancer, prostate cancer, kidney cancer, liver cancer, pancreatic cancer, gastric cancer, lung cancer, head and neck cancer, thyroid cancer, malignant glioma, leukemia, lymphoma, and multiple myeloma.
